# EUROPEAN PATENT APPLICATION

(11) **EP 2 545 919 A1**
(43) Date of publication of application: **16.01.2013**
(21) Application number: 12166667.1
(22) Date of filing: 22.12.2006
(51) Int. Cl.: A61K 31/497, A61K 31/4709, A61P 25/14, A61P 25/16, A61P 25/28

(54) **Treatment of synucleinopathies**

(30) Priority: 23.12.2005 US 753809 P
(62) Divisional of application: 06848984.8
(71) Applicant: Link Medicine Corporation, Cambridge MA 02139 (US)
(72) Inventor: Justman, Craig J., Cambridge, MA Massachusetts 02139 (US); Lansbury, Peter T., Jr, Brookline, MA Massachusetts 02445 (US); Rosenberg, Adam, J., Concord, MA 01742 (US); Williams, Peter J., Dover, MA Massachusetts 02030 (US)
(74) Representative: Rands, Peter David

(57) **Abstract**

Methods and compositions useful in the treatment or prevention of synucleinopathies, such as Parkinson's Disease, Diffuse Lewy Body Disease, and Multiple System Artophy, or other neurodegenerative diseases are provided. The treatment including administering to a subject a farnesyl transferase inhibitor compound.

SEQUENCE LISTING

<110>  Link Medicine Corporation

<120>  Treatment Of Synucleinopathies

<130>  39823-502D01EP

<140>  12166667.1
<141>  2012-05-03

<150>  60/753809
<151>  2005-12-23

<150>  06848984.8
<151>  2008-07-09

<150>  PCT/US2006/048852
<151>  2006-12-22

<160>  8

<170>  PatentIn version 3.5

<210>  1
<211>  30
<212>  DNA
<213>  Artificial Sequence

<220>
<223>  chemically synthesized oligo

<400>  1
ctaaagctta tgcagctcaa gccgatggag                                        30


<210>  2
<211>  33
<212>  DNA
<213>  Artificial Sequence

<220>
<223>  chemically synthesized oligo

<400>  2
ctaagactcg agttaggctg ccttgctgag agc                                    33


<210>  3
<211>  58
<212>  DNA
<213>  Artificial Sequence

<220>
<223>  chemically synthesized oligo

<400>  3
ctaaagctta tggactacaa ggatgacgac gacaaagatg cagctcaagc cgatggag         58


<210>  4
<211>  30
<212>  DNA
<213>  Artificial Sequence

<220>
<223>  chemically synthesied oligo

<400>  4
atcctcgagt taggctgcct tgacgagagc                                        30


<210>  5
<211>  58
<212>  DNA
<213>  Artificial Sequence

<220>
<223>  chemically synthesized oligo

<400>  5
ctaaagctta tggactacaa ggatgacgac gacaaagatg gagggtcaac gctggctg         58


<210>  6
<211>  34
<212>  DNA
<213>  Artificial Sequence

<220>
<223>  chemically synthesized oligo

<400>  6
atcctcgagc tatgctgcag aaagagcaat cgca                                   34


<210>  7
<211>  44
<212>  DNA
<213>  Artificial Sequence

<220>
<223>  chemically synthesized oligo

<400>  7
atcctcgagc tatgctgcct tagaaagagc aatcgcatta aatc                        44


<210>  8
<211>  6
<212>  PRT
<213>  Homo sapiens

<400>  8

Lys Thr Lys Glu Gly Val
1               5

## Description

### Related Applications

The present application claims priority under 35 U.S.C. § 119(e) to U.S. provisional patent application, USSN 60/753,809, filed December 23, 2005, which is incorporated herein by reference.

### Field of the Invention

The present invention relates to the treatment of neurodegenerative diseases, particularly synucleinopathies, such as Parkinson's disease (PD), diffuse Lewy body disease (DLBD), and multiple system atrophy (MSA).

### Background of the Invention

Synucleinopathies are a diverse group of neurodegenerative disorders that share a common pathologic lesion containing abnormal aggregates of insoluble α-synuclein protein in selectively vulnerable populations of neurons and glia. Certain evidence links the formation of filamentous aggregates to the onset and progression of clinical symptoms and the degeneration of affected brain regions in neurodegenerative disorders including Parkinson's disease (PD), diffuse Lewy body disease (DLBD), multiple system atrophy (MSA), and disorders of brain iron concentration including pantothenate kinase-associated neurodegeneration (*e.g*., PANK1). The current treatment options for these diseases include symptomatic medications such as carbidopa-levodopa, anticholinergics, and monoamine oxidase inhibitors, with widely variable benefit. Even for the best responders, *i.e.,* patients with idiopathic Parkinson's Disease, an initial good response to levodopa is typically overshadowed by drug-induced complications such as motor fluctuations and debilitating dyskinesia, following the first five to seven years of therapy. For the rest of the disorders, the current medications offer marginal symptomatic benefit. Given the severe debilitating nature of these disorders and their prevalence, there is a clear need in the art for novel approaches towards treating and managing these diseases.

### Summary of the Invention

The present invention relates to therapeutic approaches to the treatment of synucleinopathies, such as Parkinson's disease (PD), diffuse Lewy body disease (DLBD), multiple system atrophy (MSA), and pantothenate kinase-associated neurodegeneration (PANK), by treatment with farnesyl transferase inhibitors. Other neurodegenerative diseases where abnormal synuclein metabolism or accumulation has been implicated such as amyotrophic lateral sclerosis (ALS), Huntington's Disease (HD), and Alzheimer's Disease (AD) may also be treated with farnesyl transferase inhibitors.

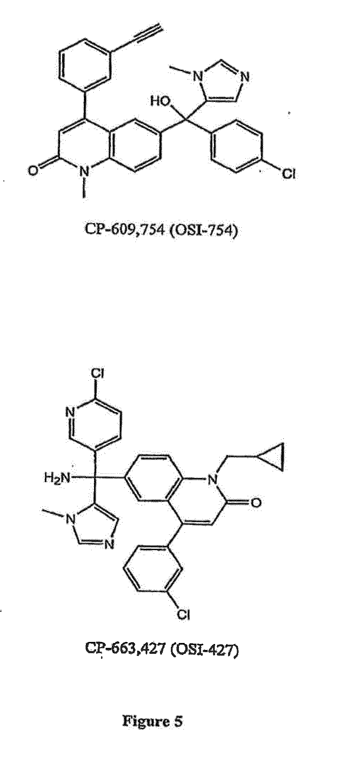

In one aspect, the invention provides methods for treating a subject with a synucleinopathy or other neurodegenerative diseases by administering a therapeutically effective amount of a farnesyl transferase inhibitor or composition thereof. In certain embodiments, the farnesyl transferase inhibitor is a small molecule. In some embodiments, the farnesyl transferase inhibitor is of one of the formulae disclosed herein, or a derviative, analog, stereoisomer, isomer, solvate, or salt thereof. In one embodiment, the composition includes one of the farnesyl transferase inhibitors shown in *Figure 5*.

In another aspect, the invention provides methods for treating a subject with a synucleinopathy or other neurodegenerative disease by administering both a farnesyl transferase inhibitor or composition thereof and a second therapeutic agent or composition thereof. The two compounds and/or compositions can be administered as a combination composition comprising both compounds. Alternatively, the two compounds can be administered separately (*e.g*., as two different compositions) either simultaneously or sequentially as described herein. In some embodiments, a farnesyl transferase inhibitor composition includes one or more farnesyl transferase inhibitors disclosed herein, or a derviative, analog, stereoisomer, isomer, solvate, or salt thereof. In one embodiment, a farnesyl transferase inhibitor composition includes the farnesyl transferase inhibitors shown in *Figure 5*, or a derviative, analog, stereoisomer, isomer, solvate, or salt thereof. In some embodiments, the second therapeutic agent may be, but is not limited to, dopamine agonists (*e.g*., pramipexole), monoamine oxidase inhibitors (*e.g*., rasagiline), glutamate antagonists (*e.g*., memantine), anticholinergic agents (*e.g*., trihexyphenidyl), or acetylcholinesterase inhibitors (*e.g.*, rivastigmine).

According to the invention, FTI-277, a farnesyl transferase inhibitor, lowers synuclein level in COS-7 cells and inhibits synuclein toxicity in neuronally differentiated SH-SY5Y cells. These neuroblastoma cells can be differentiated to dopaminergic cells and are useful for assessing the pathogenesis of Parkinson's disease (PD). FTI-277 has also been shown to inhibit α-synuclein toxicity in primary dopaminergic cultures.

It should be appreciated that aspects and embodiments of the invention described herein in connection with one farnesyl transferase inhibitor may also be practiced using two or more farnesyl transferase inhibitors (*e.g*., between 2 and 50, between 2 and 25, between 2 and 10, between 2 and 5, 2, 3, 4, 5, 6, 7, 8, or 9). Similarly, aspects and embodiments of the invention described herein in connection with one other agent also may be practiced using two or more other agents (*e.g*., between 2 and 50, between 2 and 25, between 2 and 10, between 2 and 5, 2, 3, 4, 5, 6, 7, 8, or 9).

### Brief Description of the Drawing

*Figure 1* shows that UCH-L1 membrane association is regulated by its farnesylation.

*Figure 2* shows that C220S mutation abolished the inhibitory effect of UCH-L1 WT on α-synuclein degradation.

*Figure 3* shows that farnesyl transferase inhibitor can rescue the α-synuclein toxicity in infected SH-SY5Y cells overexpressing α-synuclein.

*Figure 4* shows that FTI-277 rescued α-synuclein toxicity in SH-SY5Y cells by reducing the amount of α-synuclein accumulation.

*Figure 5* shows the formula of compound OSI-754 (CP-609,754) and OSI-427 (CP-663,427).

*Figure 6* is a graph showing the number of cells positive for α-synuclein immunoreactivity in the cortex (top panel) and hippocampus (bottom panel) of 11 month old α-synuclein transgenic mice after 30 days of treatment with Zarnestra and control.

*Figure 7* shows the (A) frontal cortex of α-synuclein transgenic mice treated with vehicle (left panel) or Zarnestra (right panel); and (B) hippocampus of α-synuclein transgenic mice treated with vehicle (left panel) or Zarnestra (right panel). Immunofluorescence analysis of brain sections performed with a primary antibody to full-length human α-synuclein, then a secondary Cy2-conjugated antibody. Magnification: 100 fold.

*Figure 8* shows ubiquitin immunohistochemistry in the cortex and parts of the neuronal layer in the hippocampus of α-synuclein transgenic mice treated with vehicle (left panel) or Zarnestra (right panel). Magnification: 200 fold.

*Figure 9* shows Campbell Switzer staining of the Lewy body-like inclusions in the hippocampus of α-synuclein transgenic mice treated with vehicle (left panel) or Zarnestra (right panel). Magnification: 400 fold.

*Figure 10* shows the quantification of α-synuclein by ELISA in the cytoplasmic fraction from of cortex of non-transgenic (ntg) or α-synuclein transgenic (syn tg) mice treated for 30 days.

*Figure 11* shows the quantification of α-synuclein by ELISA in the membrane fraction of the cortex of non-transgenic (ntg) or α-synuclein transgenic (syn tg) mice treated for 30 days.

*Figure 12* shows the quantification of farnesylated UCH-L1 in the membrane fraction from the cortex of non-transgenic (ntg) or α-synuclein transgenic (syn tg) mice treated for 30 days.

*Figure 13* shows the quantification of α-synuclein by ELISA in the cytoplasmic fraction from the cortex of α-synuclein transgenic mice treated for 30 days.

*Figure 14* shows the quantification of α-synuclein by ELISA in the membrane fraction from the cortex of α-synuclein transgenic mice treated for 30 days.

*Figure 15* shows the quantification of UCH-L1 in the membrane fraction from the cortex of α-synuclein transgenic mice treated for 30 days.

*Figure 16* demonstrates the number of cells positive for α-synuclein immunoreactivity in the cortex (top panel) and hippocampus (bottom panel) of 7 month old α-synuclein transgenic mice after 90 days of treatment.

*Figure 17* shows the hippocampus of α-synuclein transgenic mice treated with 7 month old α-synuclein transgenic mice after 90 days of treatment with vehicle or OSI-754. Immunofluorescence analysis of brain sections performed with a primary antibody to full-length human α-synuclein, then a secondary Cy2-conjugated antibody. Magnification: 20 fold.

### Detailed Description of the Invention

The invention provides a system for treating synucleinopathic subjects or patients with other neurodegenerative diseases. In certain embodiments, the invention includes methods of treating a subject with a prototypic synucleinopathy, such as Parkinson's Disease (PD), diffuse Lewy body disease (DLBD), multiple system atrophy (MSA), and pantothenate kinase-associated neurodegeneration (PANK). In certain other embodiments, the invention includes methods of treating a subject with a neurodegenerative disease such as amyotrophic lateral sclerosis (ALS), Huntington's Disease (HD), or Alzheimer's Disease (AD). Without wishing to be bound by any particular theory or mechanism of action, the methods of the invention are useful in accelerating the degradation of α-synuclein, the accumulation of which is pathogenic in synucleinopathies. In other embodiments, the treatment methods inhibit the accumulation of α-synuclein. In yet other embodiments, the methods are useful in preventing the aggregation of α-synuclein. In still other embodiments, the methods are useful in decreasing levels of both soluble and insoluble α-synuclein. The invention provides methods for treating a subject with a synucleinopathy or other neurodegenerative disease, including the step of administering to the subject a therapeutically effective amount of a farnesyl transferase inhibitor or composition thereof. In certain embodiments, the subject is a mammal. In preferred embodiments, the subject is a human. The human may be male or female, and the human may be at any stage of development.

In one embodiment, the invention is a method for treating a subject comprising administering to the subject a farnesyl transferase inhibitor of the formula **(0):** 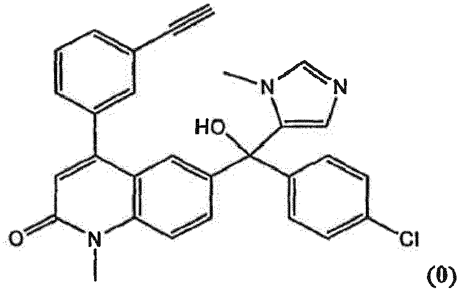 or a pharmaceutically acceptable derviative, analog, stereoisomer, isomer, solvate, or salt thereof, at a therapeutically effective dose and frequency. In certain embodiments, the tartrate salt of the compound is administered.

In another embodiment, the invention is a method for treating a subject comprising administering to the subject a farnesyl transferase inhibitor of the formula **(I):** 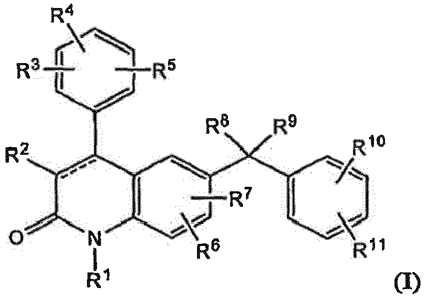 wherein
the dashed line indicates that the bond between C-3 and C-4 of the quinolin-2-one ring is a single or double bond;
R¹ is selected from H, C₁-C₁₀ alkyl, -(CR¹³R¹⁴)_{q}C(O)R¹², -(CR¹³R¹⁴)_{q}C(O)OR¹⁵, -(CR¹³R¹⁴)_{q}OR¹², -(CR¹³R¹⁴)_{q}SO₂R¹⁵, -(CR¹³R¹⁴)ₜ(C₃-C₁₀ cycloalkyl), -(CR¹³R¹⁴)ₜ(C₆-C₁₀ aryl), and -(CR¹³R¹⁴)ₜ(4-10 membered heterocyclic), wherein t is an integer from 0 to 5 and q is an integer from 1 to 5, said cycloalkyl, aryl and heterocyclic R¹ groups are optionally fused to a C₆-C₁₀ aryl group, a C₅-C₈ saturated cyclic group, or a 4-10 membered heterocyclic group; and the foregoing R¹ groups, except H but including any optional fused rings referred to above, are optionally substituted by one to four R⁶ groups;
R² is halo, cyano, -C(O)OR¹⁵, or a group selected from the substituents provided in the definition of R¹²;
each R³, R⁴, R⁵, R⁶, and R⁷ is independently selected from H, C₁-C₁₀ alkyl, C₂-C₁₀ alkenyl, halo, cyano, nitro, mercapto, trifluoromethyl, trifluoromethoxy, azido, -OR¹², - C(O)R¹², -C(O)OR¹², -NR¹³C(O)OR¹⁵, -OC(O)R¹², -NR¹³SO₂R¹⁵, -SO₂NR¹²R¹³, - NR¹³C(O)R¹², -C(O)NR¹²R¹³, -NR¹²R¹³, -CH=NOR¹², -S(O)ⱼR¹² wherein j is an integer from 0 to 2, -(CR¹³R¹⁴)ₜ(C₆-C₁₀ aryl), -(CR¹³R¹⁴)ₜ(4-10 membered heterocyclic), -(CR¹³R¹⁴)ₜ(C₃-C₁₀ cycloalkyl), and -(CR¹³R¹⁴)ₜC≡CR¹⁶, and wherein in the foregoing R³, R⁴, R⁵, R⁶, and R⁷ groups t is an integer from 0 to 5; the cycloalkyl, aryl and heterocyclic moieties of the foregoing groups are optionally fused to a C₆-C₁₀ aryl group, a C₅-C₈ saturated cyclic group, or a 4-10 membered heterocyclic group; and said alkyl, alkenyl, cycloalkyl, aryl and heterocyclic groups are optionally substituted by 1 to 3 substituents independently selected from halo, cyano, nitro, trifluoromethyl, trifluoromethoxy, azido, -NR¹³SO₂R¹⁵, - SO₂NR¹³, -C(O)R¹², -C(O)OR¹², -OC(O)R¹², -NR¹³C(O)OR¹⁵, -NR¹³C(O)R¹²,-C(O)NR¹²R¹³, -NR¹²R¹³, -OR¹², C₁-C₁₀ alkyl, C₂-C₁₀ alkenyl, C₂₋C₁₀ alkynyl, -(CR¹³R¹⁴)*ₜ*(C₆-C₁₀ aryl), and -(CR¹³R¹⁴)*ₜ*(4-10 membered heterocyclic), wherein t is an integer from 0 to 5;
R⁸ is H, -OR¹², -NR¹²R¹³, -NR¹²C(O)R¹³, cyano, -C(O)OR¹³, -SR¹², -(CR¹³R¹⁴)ₜ(4-10 membered heterocyclic), wherein t is an integer from 0 to 5, or C₁-C₆ alkyl, wherein said heterocyclic and alkyl moieties are optionally substituted by 1 to 3 R⁶ substituents;
R⁹ is -(CR¹³R¹⁴)ₜ(imidazolyl) wherein t is an integer from 0 to 5 and said imidazolyl moiety is optionally substituted by one or two R⁶ substituents;
each R¹⁰ and R¹¹ is independently selected from the substituents provided in the definition of R⁶;
each R¹² is independently selected from H, C₁-C₁₀ alkyl, -(CR¹³R¹⁴)*ₜ*(C₃-C₁₀ cycloalkyl), -(CR¹³R¹⁴)*ₜ*(C₆-C₁₀ aryl), and -(CR¹³R¹⁴)*ₜ*(4-10 membered heterocyclic), wherein t is an integer from 0 to 5; said cycloalkyl, aryl and heterocyclic R¹² groups are optionally fused to a C₆-C₁₀ aryl group, a C₅-C₈ saturated cyclic group, or a 4-10 membered heterocyclic group; and the foregoing R¹² substituents, except H, are optionally substituted by 1 to 3 substituents independently selected from halo, cyano, nitro, trifluoromethyl, trifluoromethoxy, azido, -C(O)R¹³, -C(O)OR¹³, -OC(O)R¹³, -NR¹³C(O)R¹⁴, -C(O)NR¹³R¹⁴, - NR¹³R¹⁴, hydroxy, C₁-C₆ alkyl, and C₁-C₆ alkoxy;
each R¹³ and R¹⁴ is independently H or C₁-C₆ alkyl, and where R¹³ and R¹⁴ are as -(CR¹³R¹⁴)_{q} or (CR¹³R¹⁴)ₜ each is independently defined for each iteration of q or t in excess of 1;
R¹⁵ is selected from the substituents provided in the definition of R¹² except R¹⁵ is not H;
R¹⁶ is selected from the list of substituents provided in the definition of R¹² and -SiR¹⁷R¹⁸R¹⁹;
R¹⁷, R¹⁸, and R¹⁹ are each independently selected from the substituents provided in the definition of R¹² except R¹⁷, R¹⁸, and R¹⁹ are not H; and
provided that at least one of R³, R⁴, and R⁵ is -(CR¹³R¹⁴)ₜC≡CR¹⁶ wherein t is an integer from 0 to 5 and R¹³, R¹⁴, and R¹⁶ are as defined above;
or a derviative, analog, stereoisomer, isomer, solvate, or salt thereof, at a therapeutically effective dose and frequency. In certain embodiments, a racemate is used in the invention. In other embodiments, an enantiomerically pure compound is used. In other embodiments, an enantiomerically enriched mixture is used (*e.g.*, 70%, 75%, 80%, 90%, 95%, 98%, 99% of one enantiomer).

For certain compounds of formula **I,** the stereochemistry is defined as follows:

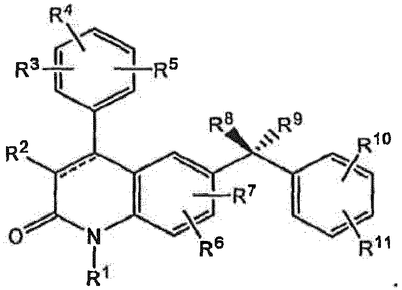

For other compounds of formula **I,** the stereochemistry is defined as follows:

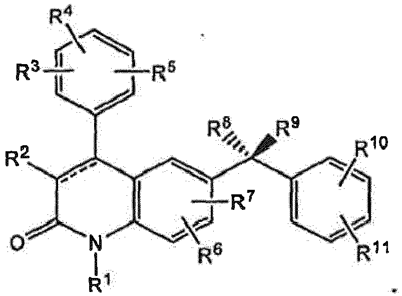

In certain classes of compounds of formula **I,** the dashed line represents one bond of a double bond between C-3 and C-4 of the quinolin-2-one ring.

In other classes of compounds of formula **I**, R¹ is H or C₁-C₆ alkyl. In certain compounds useful in the invention, R¹ is H, methyl, ethyl, *iso*-propyl, or *n*-propyl. In certain particular compounds, R¹ is methyl.

In other classes of compounds of formula **I**, R² is H, halo, or C₁-C₆ alkyl. In certain compounds, R² is H.

In yet other classes of compounds of formula **I**, one of R³, R⁴, and R⁵ is - (CR¹³R¹⁴)ₜC≡CR¹⁶, wherein t is an integer from 0 to 5, inclusive, and R¹³, R¹⁴, and R¹⁶ are as defined above; and the other two of R³, R⁴, and R⁵ are H. In other compounds, one of R³, R⁴, and R⁵ is -C≡CH. In yet other compounds, one of R³, R⁴, and R⁵ is -C≡CH; and the other two of R³, R⁴, and R⁵ are H.

In other classes of the compounds of formula **I**, R⁶ is H.

In other classes of the compounds of formula **I**, R⁷ is H.

In yet other classes of the compounds of formula I, R⁸ is H, -OR¹², or -NR¹²R¹³, wherein R¹² and R¹³ are as defined above. R⁸ is hydroxy or amino. In other compounds, R⁸ is hydroxy. In yet other componds, R⁸ is amino.

In certain classes of the compounds of formula **I**, R⁹ is an imidazolyl moiety, optionally substituted with one or two R⁶ substituents, wherein R⁶ is defined as above. In certain compounds, R⁹ is an imidazolyl moiety substituted with one R⁶ substituents, wherein R⁶ is defined as above. In certain compounds, R⁹ is an imidazolyl moiety substituted with one R⁶ substituents, wherein R⁶ is C₁-C₆ alkyl, preferably methyl. In certain compounds, R⁹ 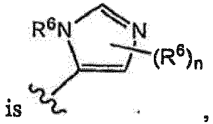 wherein R⁶ is as defined above and t is an integer between 0 and 2, inclusive. In other compounds, R⁹ is 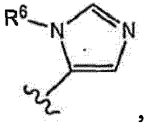 wherein R⁶ is as defined above. In other compounds, R⁹ is

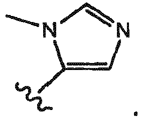

In certain classes of the compounds of formula **I**, R¹⁰ is H, C₁-C₁₀ alkyl, halo, cyano, nitro, or amino. In certain compounds, R¹⁰ is halo, preferably chloro or fluoro. In certain particular compounds, R¹⁰ is chloro. In certain compounds, at least one of R¹⁰ and R¹¹ is H.

In certain classes of the compounds of formula **I**, R¹¹ is H, C₁-C₁₀ alkyl, halo, cyano, nitro, or amino. In certain compounds, R¹¹ is halo, preferably chloro or fluoro. In certain particular compounds, R¹¹ is chloro.

Certain compounds of formula **I** include those wherein R¹ is H, C₁-C₆ alkyl, or cyclopropylmethyl; R² is H; R³ is -C≡CR¹⁶; and R⁸ is -NR¹²R¹³, -OR¹², or a heterocyclic group selected from triazolyl, imidazolyl, pyrazolyl, and piperidinyl, wherein said heterocyclic group is optionally substituted by an R⁶ group. Other compounds of formula **I** include those wherein R⁹ is imidazolyl optionally substituted by C₁-C₆ alkyl; R⁸ is hydroxy, amino, or triazolyl; and R⁴, R⁵, R¹⁰ and R¹¹ are each independently selected from H and halo.

Other compounds of formula **I** include those wherein R¹ is -(CR¹³R¹⁴)ₜ(C₃-C₁₀ cycloalkyl), wherein t is an integer from 0 to 3; R² is H; R³ is -C≡CR¹⁶; and R⁸ is -NR¹²R¹³,-OR¹², or a heterocyclic group selected from triazolyl, imidazolyl, pyrazolyl, and piperidinyl, wherein said heterocyclic group is optionally substituted by an R⁶ group. Yet other compounds of formula **I** include those wherein R⁹ is imidazolyl, optionally substituted by C₁-C₆ alkyl; R⁸ is hydroxy, amino, or triazolyl; R⁴, R⁵, R¹⁰ and R¹¹ are each independently selected from H and halo; and R¹ is cyclopropylmethyl.

Other compounds of formula **I** include those wherein R³ is ethynyl and the other substituents are as defined above. Other compounds of formula **I** include those wherein R³ is -C≡CR¹⁶. For certain compounds, R¹⁶ is H. For other compounds, R¹⁶ is -SiR¹⁷R¹⁸R¹⁹. For other compounds, R¹⁶ is C₁-C₆ alkyl.

Compounds useful in the present invention include compounds of the formula (II): 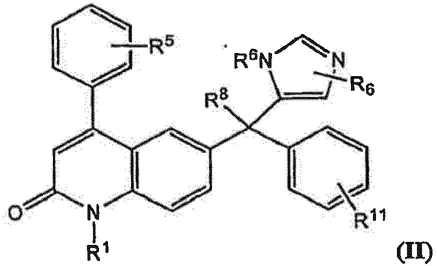 wherein R¹, R⁵, R⁶, R⁸, and R¹¹ are defined as above.

Compounds useful in the present invention also include compounds of the formula **(III):** 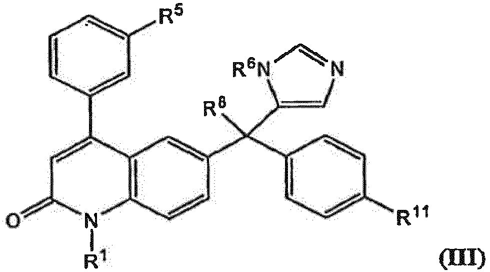 wherein R¹, R⁵, R⁶, R⁸, and R¹¹ are defined as above.

Compounds useful in the present invention include compounds of the formula **(IV):** 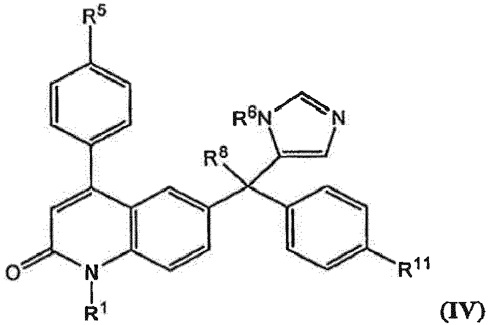 wherein R¹, R⁵, R⁶, R⁸, and R¹¹ are defined as above.

Compounds useful in the present invention include compounds of the formula **(V):** 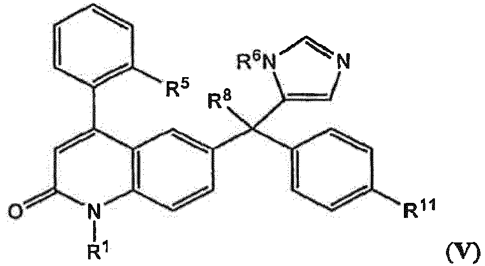 wherein R¹, R⁵, R⁶, R⁸, and R¹¹ are defined as above.

In other classes of compounds of formula **II-V,** R¹ is H or C₁-C₆ alkyl. In certain compounds useful in the invention, R' is H, methyl, ethyl, *iso*-propyl, or *n-*propyl. In certain particular compounds, R¹ is methyl.

In yet other classes of compounds of formula **II-V,** R⁵ is -(CR¹³R¹⁴)ₜC≡CR¹⁶, wherein t is an integer from 0 to 5, inclusive, and R¹³, R¹⁴, and R¹⁶ are as defined above; and the other two R³ and R⁴ are H. In yet other compounds, R⁵ is -C≡CR¹⁶. For certain compounds, R⁵ is C₂-C₆ alkynyl. In other compounds, R⁵ is -C≡CH.

In other classes of the compounds of formula **II-V,** R⁶ is H. In other classes of the compounds of formula **II-V,** R⁶ is C₁-C₆ alkyl. In certain compounds, R⁶ is methyl.

In yet other classes of the compounds of formula **II-V,** R⁸ is H, -OR¹², or - NR¹²R¹³, wherein R¹² and R¹³ are as defined above. R⁸ is hydroxy or amino. In other compounds, R⁸ is hydroxy. In yet other componds, R⁸ is amino.

In certain classes of the compounds of formula **II-V,** R¹¹ is H, C₁-C₁₀ alkyl, halo, cyano, nitro, or amino. In certain compounds, R¹¹ is halo, preferably chloro or fluoro. In certain particular compounds, R¹¹ is chloro.

Compounds useful in the present invention include compounds of the formula **(VI):** 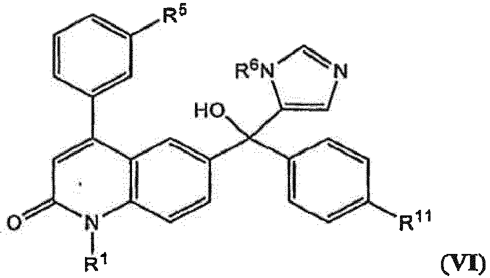 wherein R¹, R⁵, R⁶, and R¹¹ are defined as above.

In other classes of compounds of formula **VI,** R¹ is H or C₁-C₆ alkyl. In certain compounds useful in the invention, R¹ is H, methyl, ethyl, *iso*-propyl, or *n*-propyl. In certain particular compounds, R¹ is methyl.

In yet other classes of compounds of formula **VI,** R⁵ is -(CR¹³R¹⁴)ₜC≡CR¹⁶, wherein t is an integer from 0 to 5, inclusive, and R¹³, R¹⁴, and R¹⁶ are as defined above; and the other two of R³, R⁴, and R⁵ are H. For certain compounds, R⁵ is C₂-C₆ alkynyl. In other compounds, R⁵ is -C≡CH.

In certain classes of the compounds of formula **VI,** R¹¹ is H, C₁-C₁₀ alkyl, halo, cyano, nitro, or amino. In certain compounds, R¹¹ is halo, preferably chloro or fluoro. In certain particular compounds, R¹¹ is chloro.

Exemplary compounds useful in the present invention include the following:
6-[(4-Chloro-phenyl)-hydroxy-(3-methyl-3H-imidazol-4-yl)-methyl]-4-(3-ethynyl-phenyl)-1-methyl-1 H-quinolin-2-one (*R* enantiomer);
6-[(4-Chloro-phenyl)-hydroxy-(3-methyl-3H-imidazol-4-yl)-mothyl]-4-(3-ethynyl-phenyl)-1-methyl-1H-quinolin-2-one (*S* enantiomer);
6-[Amino-(4-chloro-phenyl)-(3-methyl-3H-imidazol-4-yl)-methyl]-4-(3-ethynyl-phenyl)- 1-methyl-1 H-quinolin-2-one (*R* enantiomer);
6-[Amino-(4-chloro-phenyl)-(3-methyl-3H-imidazol-4-yl)-methyl]-4-(3-ethynyl-phenyl)- 1-methyl-1 H-quinolin-2-one (*S* enantiomer);
6-[(4-Chloro-phenyl)-hydroxy-(3-methyl-3H-imidazol-4-yl)-methyl]-4-(3-ethynyl-4-fluoro-phenyl)-1-methyl-1H-quinolin-2-one;
6-[(4-chloro-phenyl)-hydroxy-(3-methyl-3H-imidazol-4-yl)-methyl]-4-[3-(3-hydroxy-3-methyl-but-1-ynyl)-phenyl]-1-methyl-1H-quinolon-2-one (*S* enantiomer); and
6-[(4-chloro-phenyl)-hydroxy-(3-methyl-3H-imidazol-4-yl)-methyl]-4-[3-(3-hydroxy-3-methyl-but-1-ynyl)-phenyl]-1-methyl-1H-quinolon-2-one (*R* enantiomer).

In another embodiment, the invention is a method for treating a subject comprising administering to the subject a farnesyl transferase inhibitor of the formula **(VII):** 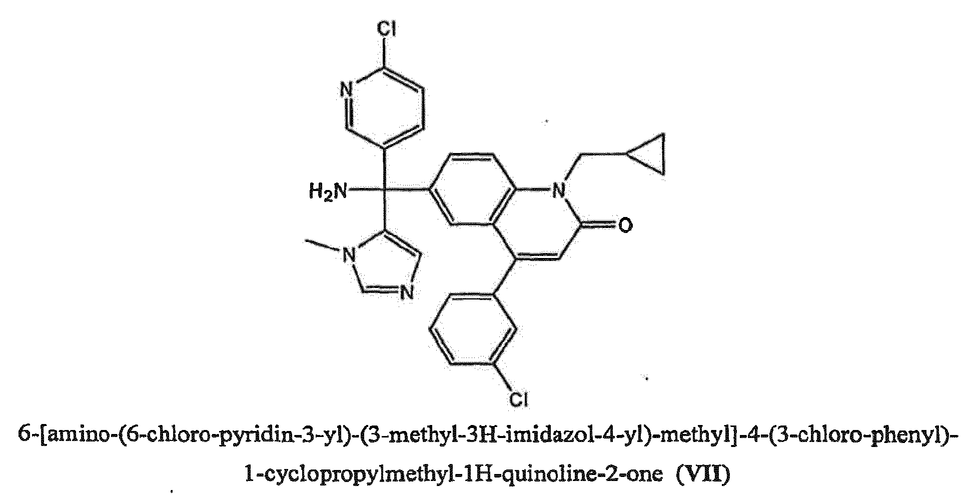 or a pharmaceutically acceptable derviative, analog, stereoisomer, isomer, solvate, or salt thereof, at a therapeutically effective dose and frequency. In certain embodiments, the tartrate salt of the compound is administered. In certain particular embodiments, the compound of formula **VII** useful in the invention is (+)-6-[amino-(6-chloro-pyridin-3-yl)-(3-methyl-3H-imidazol-4-yl)-mithyl]-4-(3-chloro-phenyl)-1-cyclopropylmethyl-1H-quinoline-2-one. In certain particular embodiments, the compound of formula **VII** useful in the invention is (-)-6-[amino-(6-chloro-pyridin-3-yl)-(3-methyl-3H-imidazol-4-yl)-methyl]-4-(3-chloro-phenyl)-1-cyclopropylmethyl-1H-quinoline-2-one.

In another embodiment, the invention is a method for treating a subject comprising administering to the synucleinopathic subject a farnesyl transferase inhibitor of the formula **(VIII):** 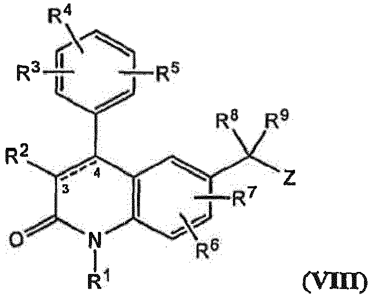 wherein
the dashed line indicates an optional second bond connecting C-3 and C-4 of the quinolin-2-one ring;
R¹ selected from H, C₁-C₁₀ alkyl, -(CR¹³R¹⁴)*_{q}*C(O)R¹², -(CR¹³R¹⁴)*_{q}*C(O)OR¹⁵,-(CR¹³R¹⁴)*_{q}*C(O)R¹², -(CR¹³R¹⁴)*_{q}*SO₂R¹⁵, -(CR¹³R¹⁴)*ₜ*(C₃-C₁₀ cycloalkyl), - (CR¹³R¹⁴)*ₜ*(C₆-C₁₀ aryl), and -(CR¹³R¹⁴)*ₜ*(4-10 membered heterocyclic), wherein said cycloalkyl, aryl and heterocyclic R¹ groups are optionally fused to a C₆-C₁₀ aryl group, a Cs-C₈ saturated cyclic group, or a 4-10 membered heterocyclic group; and the foregoing R¹ groups, except H but including any optional fused rings referred to above, are optionally substituted by 1 to 4 R⁶ groups;
R² is halo, cyano, -C(O)OR¹⁵, or a group selected from the substituents provided in the definition of R¹²;
each R³, R⁴, R⁵, R⁶, and R⁷ is independently selected from H, C₁-C₁₀ alkyl, C₂-C₁₀ alkenyl, C₂-C₁₀ alkynyl, halo, cyano, nitro, trifluoromethyl, trifluoromethoxy, azido, -OR¹², -C(O)R¹², -C(O)OR¹², -NR¹³C(O)OR¹⁵, -OC(O)R¹², -NR¹³SO₂R¹⁵, -SO₂NR¹²R¹³, -NR¹³C(O)R¹², -C(O)NR¹²R¹³, -NR¹²R¹³, -CH=NOR¹², -S(O)*ⱼ*R¹² wherein j is an integer from 0 to 2, -(CR¹³R¹⁴)*ₜ*(C₆-C₁₀ aryl), -(CR¹³R¹⁴)*ₜ*(4-10 membered heterocyclic), - (CR¹³R¹⁴)*ₜ*(C₃-C₁₀ cycloalkyl), and -(CR¹³R¹⁴)*ₜ*C≡CR¹⁶; and wherein the cycloalkyl, aryl, and heterocyclic moieties of the foregoing groups are optionally fused to a C₆-C₁₀ aryl group, a C₅-C₈ saturated cyclic group, or a 4-10 membered heterocyclic group; and said alkyl, alkenyl, cycloalkyl, aryl and heterocyclic groups are optionally substituted by 1 to 3 substituents independently selected from halo, cyano, nitro, trifluoromethyl, trifluoromethoxy, azido, -NR¹³SO₂R¹⁵, -SO₂NR¹²R¹³, -C(O)R¹², -C(O)OR¹², ₋OC(O) R¹², -NR¹³C(O)OR¹⁵, -NR¹³C(O)R¹², -C(O)NR¹²R¹³, -NR¹²R¹³, -OR¹², C₁-C₁₀ alkyl, C₂-C₁₀ alkenyl, C₂-C₁₀ alkynyl, -(CR¹³R¹⁴)*ₜ*(C₆-C₁₀ aryl), and -(CR¹³R¹⁴)*ₜ*(4-10 membered heterocyclic);
Z is an aromatic 4-10 membered heterocyclic group, substituted by 1 to 4 R⁶ substituents;
R⁸ is H, -OR¹², -OC(O)R¹², -NR¹²R¹³, -N=CR¹²R¹³, -NR¹²C(O)R¹³, cyano, - C(O)OR¹³, -SR¹², or -(CR¹³R¹⁴)*ₜ*(4-10 membered heterocyclic), wherein said heterocyclic R⁸ groups are substituted by 1 to 4 R⁶ groups;
R⁹ is -(CR¹³R¹⁴)*ₜ*(imidazolyl) or -(CR¹³R¹⁴)*ₜ*(pyridinyl) wherein said imidazolyl or pyridinyl moiety is substituted by 1 or 2 R⁶ substituents;
each R¹² is independently selected from H, C₁-C₁₀ alkyl, -(CR¹³R¹⁴)ₜ(C₃ C₁₀ cycloalkyl), -(CR¹³R¹⁴)ₜ(C₆ C₁₀ aryl), and -(CR¹³R¹⁴)*ₜ*(4-10 membered heterocyclic); said cycloalkyl, aryl and heterocyclic R¹² groups are optionally fused to a C₆-C₁₀ aryl group, a C₅-C₈ saturated cyclic group, or a 4-10 membered heterocyclic group; and the foregoing R¹² substituents, except H but including any optional fused rings, are optionally substituted by 1 to 3 substituents independently selected from halo, cyano, nitro, trifluoromethyl, trifluoromethoxy, azido, -C(O)R¹³, -C(O)OR¹³,-OC(O)R¹³, -NR¹³C(O)R¹⁴,-C(O)NR¹³R¹⁴,-NR¹³R¹⁴, hydroxy, C₁-C₆ alkyl, and C₁C₆ alkoxy;
each t is independently an integer from 0 to 5 and each q is independently an integer from 1 to 5;
each R¹³ and R¹⁴ is independently H or C₁-C₆ alkyl, and where R¹³ and R¹⁴ are as-(CR¹³R¹⁴)*_{q}* or -(CR¹³R¹⁴)*ₜ* each is independently defined for each iteration of q or t in excess of 1;
R¹⁵ is selected from the substituents provided in the definition of R¹² except R¹⁵ is not H;
R¹⁶ is selected from the list of substituents provided in the definition of R¹² and - SiR¹⁷R¹⁸R¹⁹; and
R¹⁷, R¹⁸ and R¹⁹ are each independently selected from the substituents provided in the definition of R¹² except at least one of R¹⁷, R¹⁸ and R¹⁹ is not H; or a pharmaceutically acceptable derviative, analog, stereoisomer, isomer, solvate, or salt thereof, at a therapeutically effective dose and frequency. In certain embodiments, a racemate is used in the invention. In other embodiments, an enantiomerically pure compound is used. In other embodiments, an enantiomerically enriched mixture is used (*e.g*., 70%, 75%, 80%, 90%, 95%, 98%, 99% of one enantiomer).

For certain compounds of formula **VIII,** the stereochemistry is defined as follows:

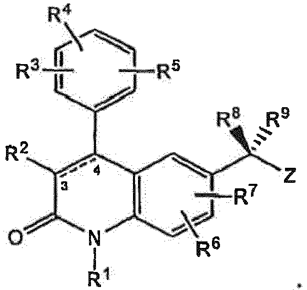

For other compounds of formula **VIII,** the stereochemistry is defined as follows:

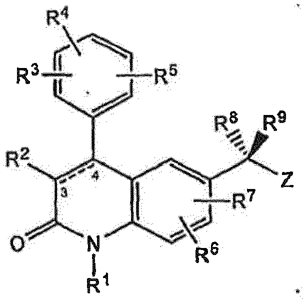

In certain embodiments, compounds of formula **VIII** are those wherein Z is a 5 or 6 membered aromatic heterocyclic group substituted with from 1 to 4 R⁶ substituents. In certain particular embodiments, compounds of formula **VIII** are those wherein Z is a pyridine or thiophene group substituted with from 1 to 4 R⁶ substituents. In certain embodiments, Z is a pyridine group substituted with 1 to 4 R⁶ substituents. In certain particular embodiments, Z is a pyridine group substituted with one R⁶ substituent. In certain embodiments, Z is 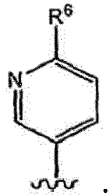 In certain particular embodiments, Z is a pyridine group substituted with one R⁶ substituent, wherein the R⁶ substituent is halo (*e.g.,* chloro). In certain particular embodiments, Z is 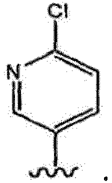 In other embodiments, compounds of formula **VIII** are those wherein Z is a 5 or 6 membered aromatic heterocyclic group fused to a benzene group, substituted with from 1 to 4 R⁶ substituents. Preferably, Z comprises from 1 to 3 heteroatoms selected from 0, S and N.

In certain embodiments, compounds of formula **VIII** are those wherein R¹ is H, C₁-C₆ alkyl, or cyclopropylmethyl. In certain embodiments, R¹ is cyclopropylmethyl.

In certain embodiments, compounds of formula **VIII** are those wherein R⁸ is - NR¹²R¹³, -OR¹², or -(CR¹³R¹⁴)*ₜ*(4-10 membered heterocyclic) substituted with from 1 to 4 R⁶ groups, wherein said 4-10 membered heterocyclic is selected from triazolyl, imidazolyl, pyrazolyl, and piperidinyl. In certain embodiments, said heterocyclic is substituted with one R⁶ group. In certain embodiments, R⁸ is hydroxy, amino, or triazolyl. In certain embodiments, R⁸ is hydroxy. In certain other embodiments, R⁸ is amino.

In certain embodiments, compounds of formula **VIII** are those wherein R⁸ is H, -OR¹², -OC(O)R¹², -NR¹²R¹³, -NR¹²C(O)R¹³, cyano, -C(O)OR¹³, -SR¹², or - (CR¹³R¹⁴)*ₜ*(4-10 membered heterocyclic), wherein said heterocyclic R⁸ groups are substituted by 1 to 4 R⁶ groups.

In certain embodiments, compounds of formula **VIII** are those wherein R³, R⁴, R⁵, and R⁶ are independently selected from H, halo, and C₁-C₆ alkoxy. In certain embodiments, one of R³, R⁴, and R⁵ is halo (*e.g*., chloro), and the others are hydrogen.

In certain embodiments, compounds of formula **VIII** are those wherein R⁶ and R⁷ are both hydrogen.

In certain embodiments, compound of formula **VIII** are those wherein R⁹ is an imidazolyl moiety, optionally substituted with one or two R⁶ substituents, wherein R⁶ is defined as above. In certain compounds, R⁹ is an imidazolyl moiety substituted with one R⁶ substituents, wherein R⁶ is defined as above. In certain compounds, R⁹ is an imidazolyl moiety substituted with one R⁶ substituents, wherein R⁶ is C₁-C₆ alkyl, preferably methyl. In certain compounds, R⁹ is 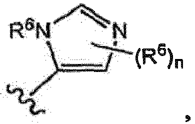 wherein R⁶ is as defined above and t is an integer between 0 and 2, inclusive. In other compounds, R⁹ is 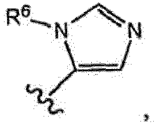 wherein R⁶ is as defined above. In other compounds, R⁹ is

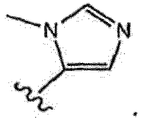

Compounds useful in the present invention include compounds of the formula: 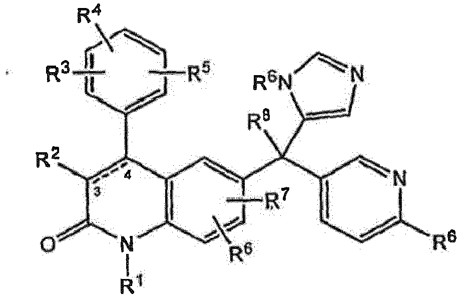 wherein R¹, R², R³, R⁴, R⁵, R⁶, R⁷, and R⁸ are defined as above.

Compounds useful in the present invention include compounds of the formula: 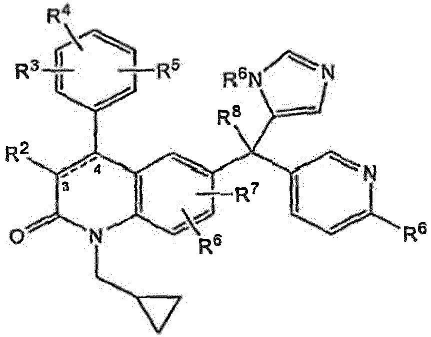 wherein R², R³, R⁴, R⁵, R⁶, R⁷, and R⁸ are defined as above.

Compounds useful in the present invention include compounds of the formula: 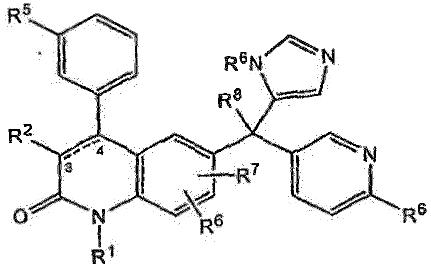 wherein R¹, R², R⁵, R⁶, R⁷, and R⁸ are defined as above.

Compounds useful in the present invention include compounds of the formula: 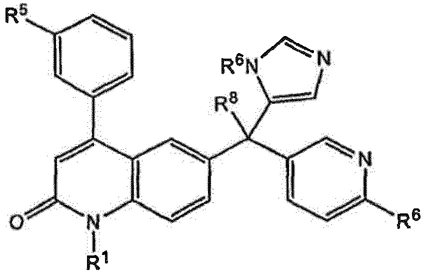 wherein R¹, R⁵, R⁶, and R⁸ are defined as above.

Compounds useful in the present invention include compounds of the formula: 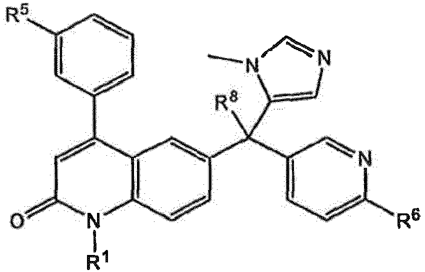 wherein R¹, R⁵, R⁶, and R⁸ are defined as above.

Exemplary compounds of the invention include:
6-[amino-(6-chloro-pyridin-3-yl)-(3-methyl-3H-imidazol-4-yl)-methyl]-4-(3-chlorophenyl)-1-methyl-1H-quinolin-2-one (R enantiomer);
6-[amino -(6-chloro-pyridin-3-yl)-(3-methyl-3H-imidazol-4-yl)-methyl]-4-(3-chlorophenyl)-1-methyl-1H-quinolin-2-one (S enantiomer);
4-(3-chloro-phenyl)-6-[(6-chloro-pyridin-3-yl)-hydroxy-(3-methyl-3H-imidazol-4-yl)-methyl]-1-cyclopropylmethyl-1H-quinolin-2-one;
6-[amino -(6-chloro-pyridin-3-yl)-(3-methyl-3H-imidazol-4-yl)-methyl]-4-(3-chlorophenyl)-1-cyclopropylmethyl-1H-quinolin-2-one;
4-(3-chloro-phenyl )-6-[(5-chloro-pyridin-2-yl)-bydroxy-(3-methyl-3H-imidazol-4-yl)-methyl]-1-methyl-1H-quinolin-2-one;
6-[amino-(5 -chloro-pyridin-2-yl)-(3-methyl-3H-imid azol- 4-yl)-methyl]-4-(3-chlorophenyl)-1-methyl-1H- quinolin-2-one;
6-[amino-(5-chloro-pyridin-2-yl)-(3-methyl-3H-imidazol- 4-yl)-methyl]-4-(3-chlorophenyl)-1-cyclopropyhnethyl-1H-quinolin-2-one;
6-[amino-(6-chloro-pyridin-3-yl)-(3-methyl-3H-imidazol- 4-yl)-methyl]-4-(3,5-dichloro-phenyl)-1-methyl-1H- quinolin-2-one;
6-[amino-(5-chloro-thiophen-2-yl)-(3-methyl-3H-imidazol- 4-yl)-methyl]-4-(3-chloro-phenyl)-1-methyl-1H- quinolin-2-one;
6-[(5-chloro-thiophen-2-yl)-hydroxy-(3-methyl-3H- imidazol-4-yl)-methyl]-4-(3-ethoxy-phenyl)-1-methyl- 1H-quinolin-2-one;
amino-(5-chloro-thiophen-2-yl)-(3-methyl-3H-imidazol-4- yl)-meth yl]-4-(3-ethoxyphenyl)-1-meth yl-1H-quinolin- 2-one;
6-[(6-chloro-pyridin-3-yl)-hydroxy-(3-methyl-3H- imidazol-4-yl)-methyl]-4-(3-ethoxy-phenyl)-1-methyl-1H-quinolin-2-one;
6-[amino-(6-chloro-pyridin-3-yl)-(3-methyl-3H-imidazol- 4-yl)-methyl]-4-(3-etho henyl)-1-methyl-1H-quinolin- 2-one;
6[benzo[b]thiophen-2-yl-hydroxy-(3-methyl-3H-imidazol- 4-yl)-methyl]-4-(3-chlorophenyl)-1-methyl-1H- quinolin-2-one;
6-[amino-(6-chloro-pyridin-3-yl)-(3-methyl-3H-imidazol- 4-yl)-methyl]-4-(3-chlorophenyl)-1 H-quinolin-2-one;
(-)-6-[amino-(6-chloro-pyridin-3-yl)-(3-methyl-3H- imidazol-4-yl)-methyl]-4-(3-chloro-phenyl)-1- cyclopropylmethyl-1H-quinolin-2-one;
6-[amino-(6-me thyl-pyridin-3-yl)-(3-methyl-3H-imidazol- 4-yl)-methyl]-4-(3-chlorophenyl)-1-methyl-1H- quinolin-2-one;
6-[ amin o-(p yridin-3-yl)-(3-methyl-3 H-imid a zol-4-yl)- methyl]-4-(3-chlorophenyl)-1-cyclopropylmethyl-1H-quinolin-2-one;
(+)-4-(3-chloro-phenyl)-6-[(6-chloro-pyridin-3-yl)- hydroxy-(3-methyl-3H-imidazol-4-yl)-methyl]-1- cyclopropylmethyl-1H-quinolin-2-one; and
pharmaceutically acceptable derivatives, analogs, stereoisomers, isomers, solvates, or salts of the foregoing compounds.

In another embodiment, the invention is a method for treating a subject comprising administering to the synucleinopathic subject a farnesyl transferase inhibitor of the formula **(IX):** 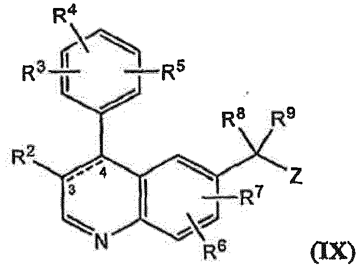 wherein
the dashed line indicates an optional second bond connecting C-3 and C-4 of the quinoline ring;
R² is halo, cyano, -C(O)OR¹⁵, or a group selected from the substituents provided in the definition of R¹²;
each R³, R⁴, R⁵, R⁶, and R⁷ is independently selected from H, C₁-C₁₀ alkyl, C₂-C₁₀ alkenyl, C₂-C₁₀alkynyl, halo, cyano, nitro, trifluoromethyl, trifluoromethoxy, azido, -OR¹², -C(O)R¹², -C(O)OR¹², -NR¹³C(O)OR¹⁵ -OC(O)R¹², -NR¹³SO₂R¹⁵ -SO₂NR¹²R¹³,-NR¹³C(O)R¹², -C(O)NR¹²R¹³, -NR¹²R¹³-CH=NOR¹² -S(O)R¹² wherein j is an integer from 0 to 2, -(CR¹³R¹⁴)*ₜ*(C₆-C₁₀ aryl), -(CR¹³R¹⁴)*ₜ*(4-10 membered heterocyclic),-(CR¹³R¹⁴), -(C₃-C₁₀ cycloalkyl), and- (CR¹³R¹⁴)*ₜ*C≡CR¹⁶; and wherein the cycloalkyl, aryl, and heterocyclic moieties of the foregoing groups are optionally fused to a C₆-C₁₀ aryl group, a C₅-C₈ saturated cyclic group, or a 4-10 membered heterocyclic group; and said alkyl, alkenyl, cycloalkyl, aryl and heterocyclic groups are optionally substituted by 1 to 3 substituents independently selected from halo, cyano, nitro, trifluoromethyl, trifluoromethoxy, azido, -NR¹³SO₂R¹⁵, -SO₂NR¹³, -C(O)R¹², -C(O)OR¹², -OC(O)R¹², -NR¹³C(O)OR¹⁵, -NR¹³C(O)R¹², -C(O)NR¹²R¹³, -NR¹²R¹³, -OR¹², C₁-C₁₀ alkyl, C₂-C₁₀ alkenyl, C₂-C₁₀ alkynyl, -(CR¹³R¹⁴)*ₜ*(C₆-C₁₀ aryl), and -(CR¹³R¹⁴)*ₜ*(4-10 membered heterocyclic);
Z is an aromatic 4-10 membered heterocyclic group, substituted by 1 to 4 R⁶ substituents;
R⁸ is H, -OR¹², -OC(O)R¹², -NR¹²R¹³, -NR¹²C(O)R¹³, cyano, -C(O)OR¹³, -SR¹², or ₋(CR¹³R¹⁴)*ₜ*(4-10 membered heterocyclic), wherein said heterocyclic R⁸ groups are substituted by 1 to 4 R⁶ groups;
R⁹ is-(CR¹³R¹⁴)*ₜ*(imidazolyl) or-(CR¹³R¹⁴)*ₜ*(pyridinyl), wherein said imidazolyl or pyridinyl moiety is substituted by 1 or 2 R⁶ substituents;
each R¹² is independently selected from H, C₁-C₁₀ alkyl, -(CR¹³R¹⁴)*ₜ*(C₃-C₁₀ cycloalkyl), -(CR¹³R¹⁴)*ₜ*(C₆-C₁₀ aryl), and -(CR¹³R¹⁴)*ₜ*(4-10 membered heterocyclic); said cycloalkyl, aryl, and heterocyclic R¹² groups are optionally fused to a C₆-C₁₀ aryl group, a C₅-C₈ saturated cyclic group, or a 4-10 membered heterocyclic group; and the foregoing R₁₂ substituents, except H but including any optional fused rings, are optionally substituted by 1 to 3 substituents independently selected from halo, cyano, nitro, trifluoromethyl, trifluoromethoxy, azido, -C(O)R¹³, -C(O)OR¹³, -OC(O)R¹³, -NR¹³C(O)R¹⁴, -C(O)NR¹³R¹⁴, -NR¹³R¹⁴, hydroxy, C₁-C₆ alkyl, and C₁-C₆ alkoxy;
each t is independently an integer from 0 to 5;
each R¹³ and R¹⁴ is independently H or C₁-C₆ alkyl, and where R¹³ and R¹⁴ are as - (CR¹³R¹⁴), each is independently defined for each iteration of t in excess of 1;
R¹⁵ is selected from the substituents provided in the definition of R¹² except R¹⁵ is not H;
R¹⁶ is selected from the list of substituents provided in the definition of R¹² and - SiR¹⁷R¹⁸R¹⁹; and,
R¹⁷, R³⁸ and R¹⁹ are each independently selected from the substituents provided in the definition of R¹² except at least one of R¹⁷, R¹⁸ and R¹⁹ is not H;
or a pharmaceutically acceptable derviative, analog, stereoisomer, isomer, solvate, or salt thereof, at a therapeutically effective dose and frequency. In certain embodiments, a racemate is used in the invention. In other embodiments, an enantiomerically pure compound is used. In other embodiments, an enantiomerically enriched mixture is used (*e.g.,* 70%, 75%, 80%, 90%, 95%, 98%, 99% of one enantiomer).

For certain compounds of formula **IX,** the stereochemistry is defined as follows:

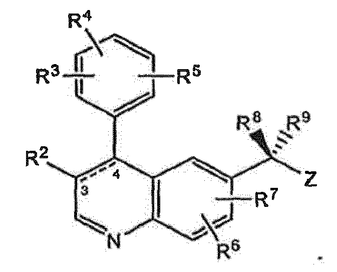

For other compounds of formula **IX,** the stereochemistry is defined as follows:

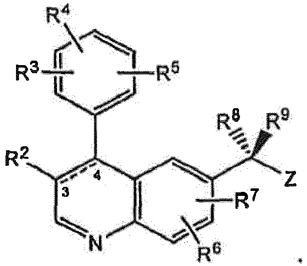

In certain embodiments, compounds of formula **IX** are those wherein Z is a 5 or 6 membered aromatic heterocyclic group substituted with from 1 to 4 R⁶ substituents. In certain particular embodiments, compounds of formula **IX** are those wherein Z is a pyridine or thiophene group substituted with from 1 to 4 R⁶ substituents. In certain embodiments, Z is a pyridine group substituted with 1 to 4 R⁶ substituents. In certain particular embodiments, Z is a pyridine group substituted with one R⁶ substituent. In certain embodiments, Z is 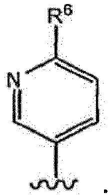 In certain particular embodiments, Z is a pyridine group substituted with one R⁶ substituent, wherein the R⁶ substituent is halo (*e.g.,* chloro). In certain particular embodiments, Z is 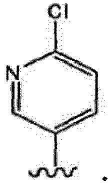 In other embodiments, compounds of formula **IX** are those wherein. Z is a 5 or 6 membered aromatic heterocyclic group fused to a benzene group, substituted with from 1 to 4 R⁶ substituents. Preferably, Z comprises from 1 to 3 heteroatoms selected from 0, S and N.

In certain embodiments, compounds of formula **IX** are those wherein R⁸ is-NR¹²R¹³, -OR¹², or -(CR¹³R¹⁴)*ₜ*(4-10 membered heterocyclic) substituted with from 1 to 4 R⁶ groups, wherein said 4-10 membered heterocyclic is selected from triazolyl, imidazolyl, pyrazolyl, and piperidinyl. In certain embodiments, said heterocyclic is substituted with one R⁶ group. In certain embodiments, R⁸ is hydroxy, amino, or triazolyl. In certain embodiments, R⁸ is hydroxy. In certain other embodiments, R⁸ is amino.

In certain embodiments, compounds of formula **IX** are those wherein R⁸ is H,-OR¹², -OC(O)R¹², -NR¹²R¹³, -NR¹²C(O)R¹³, cyano, -C(O)OR¹³, -SR¹², or-(CR¹³R¹⁴)*ₜ*(4-10 membered heterocyclic), wherein said heterocyclic R⁸ groups are substituted by 1 to 4 R⁶ groups.

In certain embodiments, compounds of formula **IX** are those wherein R³, R⁴, R⁵, and R⁶ are independently selected from H, halo, and C₁-C₆ alkoxy. In certain embodiments, one of R³, R⁴, and R⁵ is halo (*e.g*., chloro), and the others are hydrogen.

In certain embodiments, compounds of formula **IX** are those wherein R⁶ and R⁷ are both hydrogen.

In certain embodiments, compound of formula **IX** are those wherein R⁹ is an imidazolyl moiety, optionally substituted with one or two R⁶ substituents, wherein R⁶ is defined as above. In certain compounds, R⁹ is an imidazolyl moiety substituted with one R⁶ substituents, wherein R⁶ is defined as above. In certain compounds, R⁹ is an imidazolyl moiety substituted with one R⁶ substituents, wherein R⁶ is C₁-C₆ alkyl, preferably methyl. In certain compounds, R⁹ is 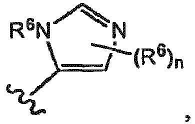 wherein R⁶ is as defined above and t is an integer between 0 and 2, inclusive. In other compounds, R⁹ is 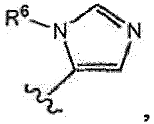 wherein R⁶ is as defined above. In other compounds, R⁹ is

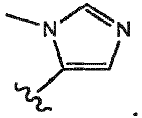

Compounds useful in the present invention include compounds of the formula: 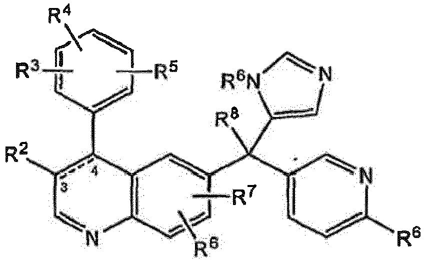 wherein R², R³, R⁴, R⁵, R⁶, R⁷, and R⁸ are defined as above.

Compounds useful in the present invention include compounds of the formula: 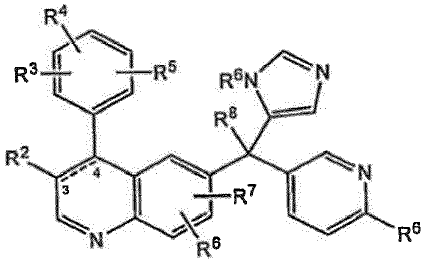 wherein R², R³, R⁴, R⁵, R⁶, R⁷, and R⁸ are defined as above.

Compounds useful in the present invention include compounds of the formula: 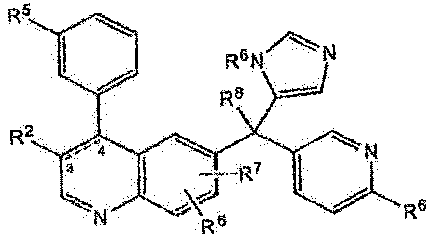 wherein R², R⁵, R⁶, R⁷, and R⁸ are defined as above.

Compounds useful in the present invention include compounds of the formula: 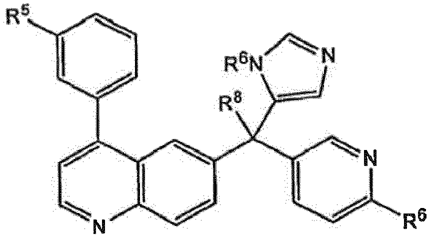 wherein R⁵, R⁶, and R⁸ are defined as above.

Compounds useful in the present invention include compounds of the formula: 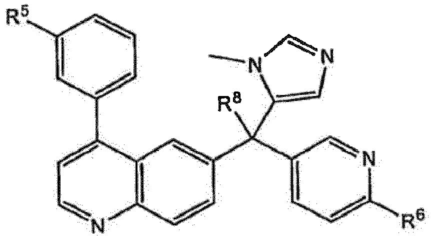 wherein R⁵, R⁶, and R⁸ are defined as above.

In another embodiment, the invention is a method for treating a subject comprising administering to the synucleinopathic subject a farnesyl transferase inhibitor of the formula **(X):** 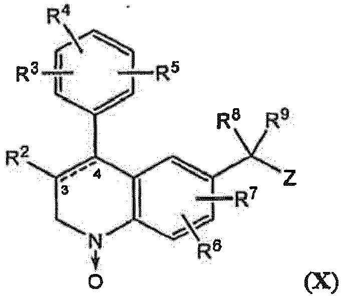 wherein
the dashed line indicates an optional second bond connecting C-3 and C-4 of the quinoline ring;
R² is halo, cyano, -C(O)OR¹⁵, or a group selected from the substituents provided in the definition of R¹²;
each R³, R⁴, R⁵, R⁶, and R⁷ is independently selected from H, C₁-C₁₀ alkyl, C₂-C₁₀ alkenyl, C₂-C₁₀ alkynyl, halo, cyano, nitro, trifluoromethyl, trifluoromethoxy, azido, -OR¹², -C(O)R¹², -C(O)OR¹², -NR¹³C(O)OR¹⁵, -OC(O)R¹², -NR¹³SO₂R¹⁵, -SO₂NR¹²R³, -NR¹³C(O)R¹², -C(O)NR¹²R¹³, -NR¹²R¹³, -CH=NOR¹², -S(O)*ⱼ*R¹² wherein j is an integer from 0 to 2, -(CR¹³R¹⁴)*ₜ*(C₆-C₁₀ aryl), -(CR¹³R¹⁴)*ₜ*(4-10 membered heterocyclic), -(CR¹³R¹⁴)*ₜ*(C₃-C₁₀ cycloalkyl), and -(CR¹³R¹⁴)*ₜ*C≡CR¹⁶; and wherein the cycloalkyl, aryl and heterocyclic moieties of the foregoing groups are optionally fused to a C₆-C₁₀ aryl group, a C₁-C₈ saturated cyclic group, or a 4-10 membered heterocyclic group; and said alkyl, alkenyl, cycloalkyl, aryl and heterocyclic groups are optionally substituted by 1 to 3 substituents independently selected from halo, cyano, nitro, trifluoromethyl, trifluoromethoxy, azido,-NR¹³SO₂R¹⁵, -SO₂NR¹²R¹³, -C(O)R¹², -C(O)OR¹²,-OC(O)R¹², -NR¹³C(O)OR¹⁵, -NR¹³C(O)R¹², -C(O)NR¹²R¹³, -NR¹²R¹³, -OR¹², C₁-C₁₀ alkyl, C₂-C₁₀ alkenyl, C₂-C₁₀ alkynyl, -(CR¹³R¹⁴)*ₜ*(C₆-C₁₀ aryl), and -(CR¹³R¹⁴)*ₜ*(4-10 membered heterocyclic);
Z is an aromatic 4-10 membered heterocyclic group, substituted by 1 to 4 R⁶ substituents;
R⁸ is H, -OR¹², -OC(O)R¹², -NR¹²R¹³,-NR¹²C(O)R¹³, cyano, -C(O)OR¹³,-SR¹², or -(CR¹³R¹⁴)*ₜ*(4-10 membered heterocyclic), wherein said heterocyclic R⁸ groups are substituted by 1 to 4 R⁶ groups;
R⁹ is -(CR¹³R¹⁴)*ₜ*(imidazolyl) or -(CR¹³R¹⁴)*ₜ*(pyrldinyl) wherein said imidazolyl or pyridinyl moiety is substituted by 1 or 2 R⁶ substituents;
each R¹² is independently selected from H, C₁-C₁₀ alkyl, -(CR¹³R¹⁴)*ₜ*(C₃-C₁₀ cycloalkyl), -(CR¹³R¹⁴)*ₜ*(C₆-C₁₀ aryl), and -(CR¹³R¹⁴)*ₜ*(4-10 membered heterocyclic); said cycloalkyl, aryl, and heterocyclic R¹² groups are optionally fused to a C₆-C₁₀ aryl group, a C₅-Cg saturated cyclic group, or a 4-10 membered heterocyclic group; and the foregoing R¹² substituents, except H but including any optional fused rings, are optionally substituted by 1 to 3 substituents independently selected from halo, cyano, nitro, trifluoromethyl, trifluoromethoxy, azido, -C(O)R¹³, -C(O)OR¹³, -OC(O)R¹³, -NR¹³C(O)R¹⁴, - C(O)NR¹³R¹⁴, -NR¹³R¹⁴, hydroxy, C₁-C₆ alkyl, and C₁-C₆ alkoxy;
each t is independently an integer from 0 to 5;
each R¹³ and R¹⁴ is independently H or C₁-C₆ alkyl, and where R¹³ and R¹⁴ are as - (CR¹³R¹⁴)*ₜ* each is independently defined for each iteration of t in excess of 1; R¹⁵ is selected from the substituents provided in the definition of R¹² except R¹⁵ is not H;
R¹⁶ is selected from the list of substituents provided in the definition of R¹² and-SiR¹⁷R¹⁸R¹⁹; and,
R¹⁷, R¹⁸ and R¹⁹ are each independently selected from the substituents provided in the definition of R¹² except at least one of R¹⁷, R¹⁸ and R¹⁹ is not H;
or a pharmaceutically acceptable derviative, analog, stereoisomer, isomer, solvate, or salt thereof, at a therapeutically effective dose and frequency. In certain embodiments, a racemate is used in the invention. In other embodiments, an enantiomerically pure compound is used. In other embodiments, an enantiomerically enriched mixture is used (*e.g.,* 70%, 75%, 80%, 90%, 95%, 98%, 99% of one enantiomer).

For certain compounds of formula **X,** the stereochemistry is defined as follows:

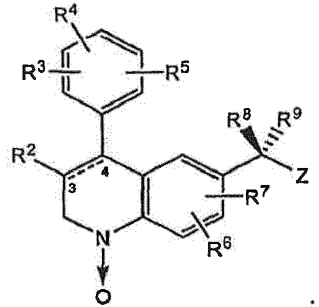

For other compounds of formula **X**, the stereochemistry is defined as follows:

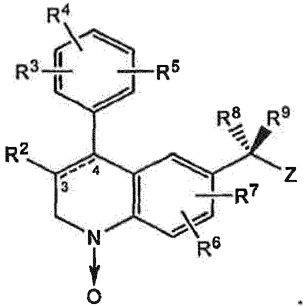

In certain embodiments, compounds of formula **X** are those wherein Z is a 5 or 6 membered aromatic heterocyclic group substituted with from 1 to 4 R⁶ substituents. In certain particular embodiments, compounds of formula **X** are those wherein Z is a pyridine or thiophene group substituted with from 1 to 4 R⁶ substituents. In certain embodiments, Z is a pyridine group substituted with 1 to 4 R⁶ substituents. In certain particular embodiments, Z is a pyridine group substituted with one R⁶ substituent. In certain embodiments, Z is 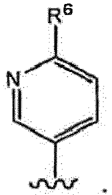 In certain particular embodiments, Z is a pyridine group substituted with one R⁶ substituent, wherein the R⁶ substituent is halo (*e.g*., chloro). In certain particular embodiments, Z is 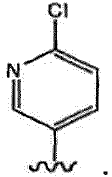 In other embodiments, compounds of formula **X** are those wherein Z is a 5 or 6 membered aromatic heterocyclic group fused to a benzene group, substituted with from 1 to 4 R⁶ substituents. Preferably, Z comprises from 1 to 3 heteroatoms selected from 0, S and N.

In certain embodiments, compounds of formula **X** are those wherein R⁸ is - NR¹²R¹³, -OR¹², or -(CR¹³R¹⁴)*ₜ*(4-10 membered heterocyclic) substituted with from 1 to 4 R⁶ groups, wherein said 4-10 membered heterocyclic is selected from triazolyl, imidazolyl, pyrazolyl, and piperidinyl. In certain embodiments, said heterocyclic is substituted with one R⁶ group. In certain embodiments, R⁸ is hydroxy, amino, or triazolyl. In certain embodiments, R⁸ is hydroxy. In certain other embodiments, R⁸ is amino.

In certain embodiments, compounds of formula **X** are those wherein R⁸ is H, - OR¹², -OC(O)R¹², -NR¹²R¹³, -NR¹²C(O)R¹³, cyano, -C(O)OR¹³, -SR¹², or-(CR¹³R¹⁴)*ₜ*(4-10 membered heterocyclic), wherein said heterocyclic R⁸ groups are substituted by 1 to 4 R⁶ groups.

In certain embodiments, compounds of formula **X** are those wherein R³, R⁴, R⁵, and R⁶ are independently selected from H, halo, and C₁-C₆ alkoxy. In certain embodiments, one of R³, R⁴, and R⁵ is halo (*e.g.,* chloro), and the others are hydrogen.

In certain embodiments, compounds of formula **X** are those wherein R⁶ and R⁷ are both hydrogen.

In certain embodiments, compound of formula **X** are those wherein R⁹ is an imidazolyl moiety, optionally substituted with one or two R⁶ substituents, wherein R⁶ is defined as above. In certain compounds, R⁹ is an imidazolyl moiety substituted with one R⁶ substituents, wherein R⁶ is defined as above. In certain compounds, R⁹ is an imidazolyl moiety substituted with one R⁶ substituents, wherein R⁶ is C₁-C₆ alkyl, preferably methyl. In certain compounds, R⁹ is 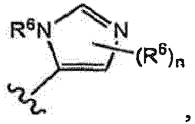 wherein R⁶ is as defined above and t is an integer between 0 and 2, inclusive. In other compounds, R⁹ is 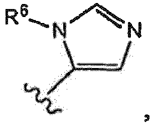 wherein R⁶ is as defined above. In other compounds, R⁹ is

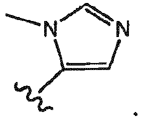

Compounds useful in the present invention include compounds of the formula: 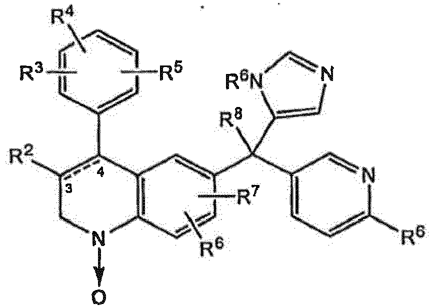 wherein R², R³, R⁴, R⁵, R⁶, R⁷, and R⁸ are defined as above.

Compounds useful in the present invention include compounds of the formula: 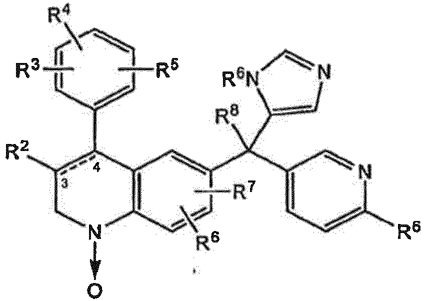 wherein R², R³, R⁴, R⁵, R⁶, R⁷, and R⁸ are defined as above.

Compounds useful in the present invention include compounds of the formula: 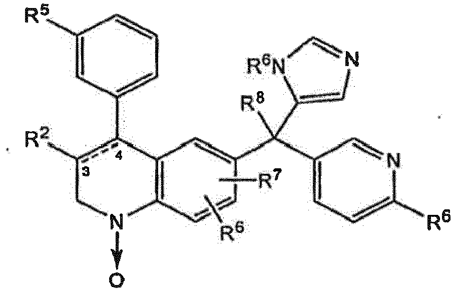 wherein R², R⁵, R⁶, R⁷, and R⁸ are defined as above.

Compounds useful in the present invention include compounds of the formula: 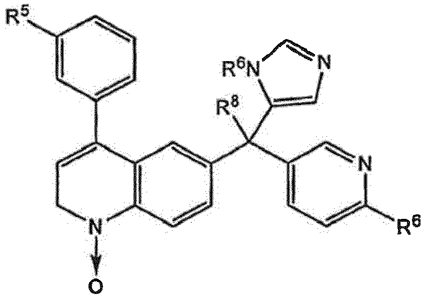 wherein R⁵, R⁶, and R⁸ are defined as above.

Compounds useful in the present invention include compounds of the formula: 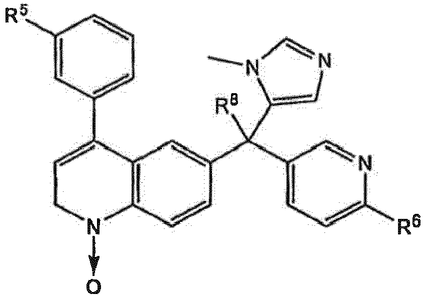 wherein R⁵, R⁶, and R⁸ are defined as above.

In another embodiment, the invention is a method for treating a subject comprising administering to the synucleinopathic subject a farnesyl transferase inhibitor of the formula **(XI):** 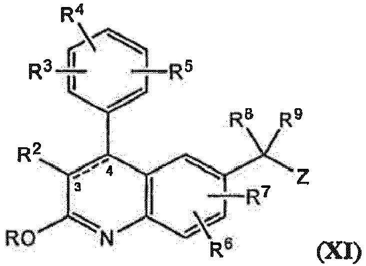 wherein
the dashed line indicates an optional second bond connecting C-3 and C-4 of the quinoline ring;
R is C₁-C₆ alkyl;
R² is halo, cyano, -C(O)OR¹⁵, or a group selected from the substituents provided in the definition of R¹²;
each R³, R⁴, R⁵, R⁶, and R⁷ is independently selected from H, C₁-C₁₀ alkyl, C₂-C₁₀ alkenyl, C₂-C₁₀alkynyl, halo, cyano, nitro, trifluoromethyl, trifluoromethoxy, azido, -OR¹², -C(O)R¹², -C(O)OR¹², -NR¹³C(O)OR¹⁵, -OC(O)R¹², -NR¹³SO₂R¹⁵, -SO₂NR¹²R¹³, -NR¹³C(O)R¹², -C(O)NR¹²R¹³, -NR¹²R¹³, -CH=NOR¹², -S(O)*ⱼ*R¹² wherein j is an integer from 0 to 2, -(CR¹³R¹⁴)*ₜ*(C₆-C₁₀ aryl), -(CR¹³R¹⁴)*ₜ*(4-10 membered heterocyclic), -(CR¹³R¹⁴)*ₜ*(C₃-C₁₀ cycloalkyl), and-(CR¹³R¹⁴)*ₜ*C≡CR¹⁶; and wherein the cycloalkyl, aryl, and heterocyclic moieties of the foregoing groups are optionally fused to a C₆-C₁₀ aryl group, a C₅-C₈ saturated cyclic group, or a 4-10 membered heterocyclic group; and said alkyl, alkenyl, cycloalkyl, aryl, and heterocyclic groups are optionally substituted by 1 to 3 substituents independently selected from halo, cyano, nitro, trifluoromethyl, trifluoromethox azido, -NR¹³SO₂R¹⁵, -SO2NR¹²R¹³, -C(O)R¹², -C(O)OR¹², -OC(O)R¹², - NR¹³C(O)OR¹⁵, -NR¹³C(O)R¹², -C(O)NR¹²R¹³, -NR¹²R¹³, -OR¹², C₁-C₁₀ alkyl, C₂-C₁₀ alkenyl, C₂-C₁₀ alkynyl, -(CR¹³R¹⁴)*ₜ*(C₆-C₁₀ aryl), and -(C¹³R¹⁴)*ₜ*(4-10 membered heterocyclic);
Z is an aromatic 4-10 membered heterocyclic group, substituted by 1 to 4 R⁶ substituents;
R⁸ is H, -OR¹², -OC(O)R¹², -NR¹²R¹³, -R¹²C(O) R¹³, cyano, -(O)OR¹³, -R¹², or -(CR¹²R¹⁴)*ₜ*(4-10 membered heterocyclic), wherein said heterocyclic R⁸ groups are subsituted by 1 to 4 R⁶ groups;
R⁹ is -(CR¹³R¹⁴)*ₜ*(imidazolyl) or -(CR¹³R¹⁴)*ₜ*(pyridinyl), wherein said imidazolyl or pyridinyl moiety is substituted by 1 or 2 R⁶ substituents;
each R¹² is independently selected from H, C1-C¹⁰ alkyl, -(CR¹³R¹⁴)*ₜ*(C₃-C₁₀ cycloalkyl), -(CR¹³R¹⁴)*ₜ*(C₆-C₁₀ aryl), and -(CR¹³R¹⁴)*ₜ*(4-10 membered heterocyclic); said cycloalkyl, aryl, and heterocyclic R¹² groups are optionally fused to a C₆-C₁₀ aryl group, a C₅-C₈ saturated cyclic group, or a 4-10 membered heterocyclic group; and the foregoing R¹² substituents, except H but including any optional fused rings, are optionally substituted by 1 to 3 substituents independently selected from halo, cyano, nitro, trifluoromethyl, trifluoromethoxy, azido, -C(O)R¹³, -C(O)OR¹³, -OC(O)R¹³, -NR¹³C(O)R¹⁴, - C(O)NR¹³R¹⁴, -NR¹³R¹⁴, hydroxy, C₁-C₆ alkyl, and C₁-C₆ alkoxy;
each t is independently an integer from 0 to 5;
each R¹³ and R¹⁴ is independently H or C₁-C₆ alkyl, and where R¹³ and R¹⁴ are as - (CR¹³R¹⁴)*ₜ* each is independently defined for each iteration of t in excess of 1;
R¹⁵ is selected from the substituents provided in the definition of R¹² except R¹⁵ is not H;
R¹⁶ is selected from the list of substituents provided in the definition of R¹² and - SiR¹⁷R¹⁸R¹⁹; and,
R¹⁷, R¹⁸ and R¹⁹ are each independently selected from the substituents provided in the definition of R¹² except at least one of R¹⁷, R¹⁸ and R¹⁹ is not H;
or a pharmaceutically acceptable derviative, analog, stereoisomer, isomer, solvate, or salt thereof, at a therapeutically effective dose and frequency. In certain embodiments, a racemate is used in the invention. In other embodiments, an enantiomerically pure compound is used. In other embodiments, an enantiomerically enriched mixture is used (*e.g*., 70%, 75%, 80%, 90%, 95%, 98%, 99% of one enantiomer).

For certain compounds of formula **XI**, the stereochemistry is defined as follows:

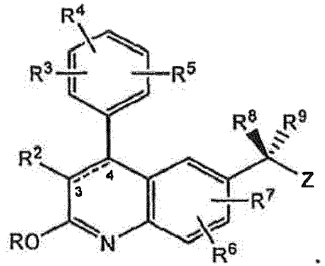

For other compounds of formula **XI**, the stereochemistry is defined as follows:

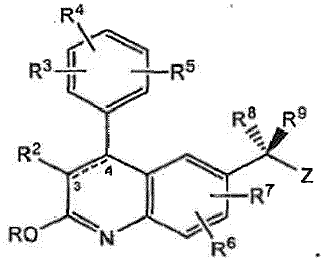

In certain embodiments, compounds of formula **IX** are those wherein Z is a 5 or 6 membered aromatic heterocyclic group substituted with from 1 to 4 R⁶ substituents. In certain particular embodiments, compounds of formula **IX** are those wherein Z is a pyridine or thiophene group substituted with from 1 to 4 R⁶ substituents. In certain embodiments, Z is a pyridine group substituted with 1 to 4 R⁶ substituents. In certain particular embodiments, Z is a pyridine group substituted with one R⁶ substituent. In certain embodiments, Z is 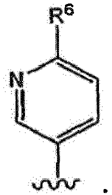 In certain particular embodiments, Z is a pyridine group substituted with one R⁶ substituent, wherein the R⁶ substituent is halo (*e.g*., chloro). In certain particular embodiments, Z is 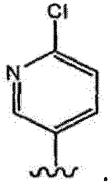 In other embodiments, compounds of formula **IX** are those wherein Z is a 5 or 6 membered aromatic heterocyclic group fused to a benzene group, substituted with from 1 to 4 R⁶ substituents. Preferably, Z comprises from 1 to 3 heteroatoms selected from 0, S and N.

In certain embodiments, compounds of formula **IX** are those wherein R⁸ is - NR¹²R¹³, -OR¹², or -(CR¹³R¹⁴)*ₜ*( 4-10 membered heterocyclic) substituted with from 1 to 4 R⁶ groups, wherein said 4-10 membered heterocyclic is selected from triazolyl, imidazolyl, pyrazolyl, and piperidinyl. In certain embodiments, said heterocyclic is substituted with one R⁶ group. In certain embodiments, R⁸ is hydroxy, amino, or triazolyl. In certain embodiments, R⁸ is hydroxy. In certain other embodiments, R⁸ is amino.

In certain embodiments, compounds of formula **IX** are those wherein R⁸ is H, - OR^{1Z}, -OC(O)R¹², -NR¹²R¹³, -NR¹²C(O)R¹³, cyano, -C(O)OR¹³, -SR¹², or - (CR¹³R¹⁴)*ₜ*(4-10 membered heterocyclic), wherein said heterocyclic R⁸ groups are substituted by 1 to 4 R⁶ groups.

In certain embodiments, compounds of formula **IX** are those wherein R³, R⁴, R⁵, and R⁶ are independently selected from H, halo, and C₁-C₆ alkoxy. In certain embodiments, one of R³, R⁴, and R⁵ is halo (*e.g.,* chloro), and the others are hydrogen.

In certain embodiments, compounds of formula **IX** are those wherein R⁶ and R⁷ are both hydrogen.

In certain embodiments, compound of formula **IX** are those wherein R⁹ is an imidazolyl moiety, optionally substituted with one or two R⁶ substituents, wherein R⁶ is defined as above. In certain compounds, R⁹ is an imidazolyl moiety substituted with one R⁶ substituents, wherein R⁶ is defined as above. In certain compounds, R⁹ is an imidazolyl moiety substituted with one R⁶ substituents, wherein R⁶ is C₁-C₆ alkyl, preferably methyl. In certain compounds, R⁹ is 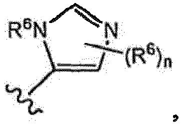 wherein R⁶ is as defined above and t is an integer between 0 and 2, inclusive. In other compounds, R⁹ is 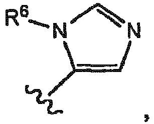 wherein R⁶ is as defined above. In other compounds, R⁹ is

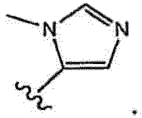

Compounds useful in the present invention include compounds of the formula: 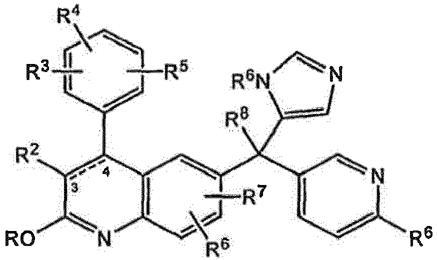 wherein R², R³, R⁴, R⁵, R⁶, R⁷, and R⁸ are defined as above.

Compounds useful in the present invention include compounds of the formula: 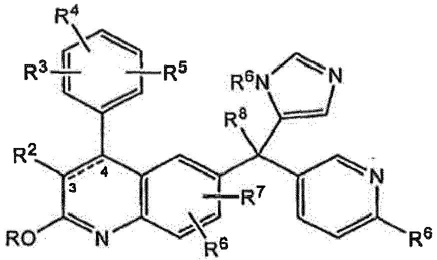 wherein R², R³, R⁴, R⁵, R⁶, R⁷, and R⁸ are defined as above.

Compounds useful in the present invention include compounds of the formula: 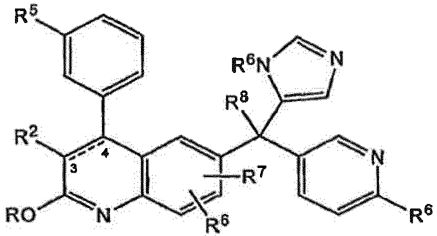 wherein R², R⁵, R⁶, R⁷, and R⁸ are defined as above.

Compounds useful in the present invention include compounds of the formula: 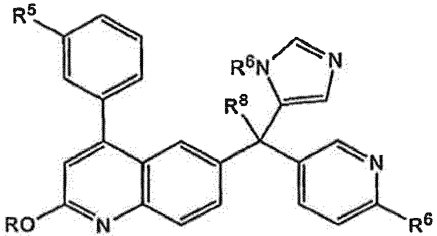 wherein R⁵, R⁶, and R⁸ are defined as above.

Compounds useful in the present invention include compounds of the formula: 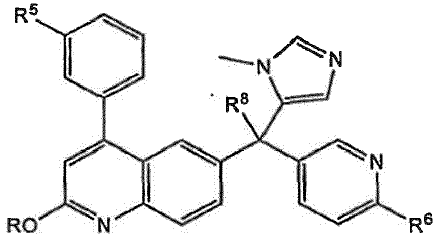 wherein R⁵, R⁶, and R⁸ are defined as above.

As used herein, the term "synucleinopathic subject" or "subject with a synucleinopathy" refers to a subject that is diagnosed with, affected by, or at risk of developing a synucleinopathy (*e.g*., predisposed, for example genetically predisposed, to developing a synucleinopathy) and/or any neurodegenerative disorder characterized by pathological synuclein aggregations. Several neurodegenerative disorders including Parkinson's disease, diffuse Lewy body disease (DLBD), and multiple system atrophy (MSA) are collectively grouped as synucleinopathies.

Synucleins are small proteins (123 to 143 amino acids) characterized by repetitive imperfect repeats KTKEGV (SEQ ID NO: XX) distributed throughout most of the amino terminal half of the polypeptide in the acidic carboxy-terminal region. There are three human synuclein proteins termed α, β, and γ, and they are encoded by separate genes mapped to chromosomes 4221.3-q22, 5q23, and 10q23.2-q23.3, respectively. The most recently cloned synuclein protein synoretin, has a close homology to γ-synuclein and is predominantly expressed within the retina. α-synuclein, also referred to as non-amyloid component of senile plaques precursor protein (NACP), SYN1 or synelfin, is a heat-stable, "natively unfolded" protein of poorly defined function. It is predominantly expressed in the central nervous system (CNS) neurons where it is localized to presynaptic terminals. Electron microscopy studies have localized α-synuclein in close proximity to synaptic vesicles at axonal termini, suggesting a role for α-synuclein in neurotransmission or synaptic organization, and biochemical analysis has revealed that a small fraction of α-synuclein may be associated with vesicular membranes but most α-synuclein is cytosolic.

Genetic and histopathological evidence supports the idea that α-synuclein is the major component of several proteinaceous inclusions characteristic of specific neurodegenerative diseases. Pathological synuclein aggregations are restricted to the α-synuclein isoforms, as β and γ synucleins have not been detected in these inclusions. The presence of α-synuclein positive aggregates is disease specific. Lewy bodies, neuronal fibrous cytoplasmic inclusions that are histopathological hallmarks of Parkinson's disease (PD) and diffuse Lewy body disease (DLBD) are strongly labeled with antibodies to α-synuclein. Dystrophic ubiquitin-positive neurites associated with PD pathology, termed Lewy neurites (LN) and CA2/CA3 ubiquitin neurites are also α-synuclein positive. Furthermore, pale bodies, putative precursors of LBs, thread-like structures in the perikarya of slightly swollen neurons and glial silver positive inclusions in the midbrains of patients with LB diseases are also immunoreactive for α-synuclein. α-synuclein is likely the major component of glial cell inclusions (GCIs) and neuronal cytoplasmic inclusions in MSA and brain iron accumulation type 1 (PANK1). α-synuclein immunoreactivity is present in some dystrophic neurites in senile plaques in Alzheimer's Disease (AD) and in the cord and cortex in amyotrophic lateral sclerosis (ALS). α-synuclein immunoreactivity is prominent in transgenic and toxin-induced mouse models of PD, AD, ALS, and HD.

Further evidence supports the notion that α-synuclein is the actual building block of the fibrillary components of LBs, LNs, and GCIs. Immunoelectron microscopic studies have demonstrated that these fibrils are intensely labeled with α-synuclein antibodies *in situ.* Sarcosyl-insoluble α-synuclein filaments with straight and twisted morphologies can also be observed in extracts of DLBD and MSA brains. Moreover, α-synuclein can assemble *in vitro* into elongated homopolymers with similar widths as sarcosyl-insoluble fibrils or filaments visualized *in situ.* Polymerization is associated with a concomitant change in secondary structure from random coil to anti-parallel β-sheet structure consistent with the Thioflavine-S reactivity of these filaments. Furthermore, the PD-association with α-synuclein mutation, A53T, may accelerate this process, as recombinant A53T α-synuclein has a greater propensity to polymerize than wild-type α-synuclein. This mutation also affects the ultrastructure of the polymers; the filaments are slightly wider and are more twisted in appearance, as if assembled from two protofilaments. The A30P mutation may also modestly increase the propensity of α-synuclein to polymerize, but the pathological effects of this mutation also may be related to its reduced binding to vesicles. Interestingly, carboxyl-terminally truncated α-synuclein may be more prone to form filaments than the full-length protein.

According to the invention, the proteosomal degradation of α-synuclein is a mediated by parkin and neuronal ubiquitin C-terminal hydrolase (UCH-L1). Parkin is an E3 ligase that ubiquitinylates α-synuclein and thereby tags it for degradation. UCH-L1 acts in normal neuronal tissues to cleave the ubiquitinylated proteins that are products of the proteosomal degradation of the polyubiquitinylated proteins.

The invention provides methods for treating synucleinopathic disorders using inhibitors of farnesyl transferase. It has been now discovered that UCH-L1 is farnesylated *in vivo.* UCH-L1 is associated with the membrane and this membrane association is mediated by farnesylation. Farnesylated UCH-L1 also stabilizes the accumulation of α-synuclein. The invention relates to the prevention or inhibition of UCH-L1 farnesylation which would result in UCH-L1 membrane disassociation and acceleration of the degradation of α-synuclein. Since α-synuclein accumulation is pathogenic in PD, DLBD, and MSA, an increased degradation of α-synuclein and/or inhibition of α-synuclein accumulation ameliorates the toxicity associated with a pathogenic accumulation of α-synuclein.

The modification of a protein by a farnesyl group can have an important effect on function for a number of proteins. Farnesylated proteins typically undergo further C-terminal modification events that include a proteolytic removal of three C-terminal ammo acids and carboxymethylation of C-terminal cystines. These C-terminal modifications facilitate protein-membrane association as well as protein-protein interactions. Farnesylation is catalyzed by a protein farnesyltransferase (FTase), a heterodimeric enzyme that recognizes the CAAX motif present at the C-terminus of the substrate protein. FTase transfers a farnesyl group from farnesyl pyrophosphate and forms a thioether linkage between the farnesyl and the cystine residues in the CAAX motif. A number of inhibitors of FTase have been developed and are known in the art. However, the invention provides novel methods for using certain farnesyl transferase inhibitors to treat subjects having symptoms associated with α-synuclein accumulation.

In methods of the invention, the term "synucleionopathy" refers to neurological disorders that are characterized by a pathological accumulation of α-synuclein. This group of disorders includes PD, DLBD, and MSA.

Parkinson's disease (PD) is a neurological disorder characterized by bradykinesia, rigidity, tremor, and postural instability. The pathologic hallmark of PD is loss of neurons in the substantia nigra pars compacta (SNpc) and the appearance of Lewy bodies in remaining neurons. It appears that more than about 50% of the cells in the SNpc need to be lost before motor symptoms appear. Associated symptoms often include small handwriting (micrographia), seborrhea, orthostatic hypotension, urinary difficulties, constipation and other gastrointestinal dysfunction, sleep disorders, depression and other neuropsychiatric phenomena, dementia, and smelling disturbances (occurs early). Patients with Parkinsonism have greater mortality, about two times compared to general population without PD. This is attributed to greater frailty or reduced mobility.

The term "synucleinopathic subject" encompasses a subject that is affected by, or is at risk of developing PD. These subjects can be readily identified by persons of ordinary skill in the art by symptomatic diagnosis and neurologic examination and/or in some instances in conjunction with genetic screening, brain scans, SPEC, PET imaging, etc.

Diagnosis of PD is mainly clinical and is based on the clinical findings listed above. Parkinsonism, refers to any combination of two of bradykinesia, rigidity, and/or tremor. PD is the most common cause of parkinsonism. Other causes of parkinsonism are side effects of drugs, mainly the major tranquilizers, such as Haldol, strokes involving the basal ganglia, and other neurodegenerative disorders, such as Diffuse Lewy Body Disease (DLBD), progressive supranuclear palsy (PSP), frontotemporal dementia (FTD), MSA, and Huntington's disease. The pathological hallmark of PD is the Lewy body, an intracytoplasmatic inclusion body typically seen in affected neurons of the substantia nigra and to a variable extent, in the cortex. Recently, α-synuclein has been identified as the main component of Lewy bodies in sporadic Parkinsonism.

Although parkinsonism can be clearly traced to viruses, stroke, or toxins in a few individuals, for the most part, the cause of Parkinson's disease in any particular case is unknown. Environmental influences which may contribute to PD may include drinking well water, farming and industrial exposure to heavy metals (*e.g*., iron, zinc, copper, mercury, magnesium and manganese), alkylated phosphates, and orthonal chlorines. Paraquat (a herbicide) has also been associated with increased prevalence of Parkinsonism including PD. Cigarette smoking is associated with a decreased incidence of PD. The current consensus is that PD may either be caused by an uncommon toxin combined with high genetic susceptibility or a common toxin combined with relatively low genetic susceptibility.

A small percentage of subjects that are at risk of developing PD can be identified for example by genetic analysis. There is good evidence for certain genetic factors being associated with PD. Large pedigrees of autosomal dominantly inherited PDs have been reported. For example, a mutation in α-synuclein is responsible for one pedigree and triplication of the SNCA gene (the gene coding for α-synuclein) is associated with PD in others.

Methods of the invention can be used in combination with one or more other medications, including medications that are currently used to treat synucleinopathies or symptoms arising as side-effects of the disease or of the aforementioned medications.

For example, methods of the invention can be used in combination with medications for treating PD. Levodopa mainly in the form of combination products containing carbodopa and levodopa (Sinemet and Sinemet CR) is the mainstay of treatment and is the most effective agent for the treatment of PD. Levodopa is a dopamine precursor, a substance that is converted into dopamine by an enzyme in the brain. Carbodopa is a peripheral decarboxylase inhibitor which prevents side effects and lower the overall dosage requirement. The starting dose of Sinemet is a 25/100 or 50/200 tablet prior to each meal. Dyskinesias may result from overdose and also are commonly seen after prolonged (*e.g*., years) use. Direct acting dopamine agonists may have less ofthis side effect. About 15% of patients do not respond to levodopa. Stalevo (carbodopa, levodopa, and entacapone) is a new combination formulation for patients who experience signs and symptoms of "wearing-off." The formulation combines carbodopa and levodopa (the most widely used agents to treat PD) with entacapone, a catechol-O-methyltransferase inhibitor. While carbodopa reduces the side effects of levodopa, entacapone extends the time levodopa is active in the brain, up to about 10% longer.

Amantidine (SYMMETREL^{®}) is a mild agent thought to work by multiple mechansims including blocking the re-uptake of dopamine into presynaptic neurons. It also activates the release of dopamine from storage sites and has a glutamate receptor blocking activity. It is used as early monotherapy, and the dosing is 200 to 300 mg daily. Amantadine may be particularly helpful in patients with predominant tremor. Side effects include ankle swelling and red blotches. It may also be useful in later stage disease to decrease the intensity of drug-induced dyskinesia.

Anticholinergics (trihexyphenidyl, benztropine mesylate, procyclidine, artane, cogentin) do not act directly on the dopaminergic system. Direct-acting dopamine agonists include bromocriptidine (Parlodel), pergolide (Permax), ropinirol (Requip), and pramipexole (Mirapex). These agents cost substantially more than levodopa (Sinemet), and additional benefits are controversial. Depending on which dopamine receptor is being stimulated, D1 and D2 agonist can exert anti-Parkinson effects by stimulating the D1 and D2 receptors, such as Ergolide. Mirapex and Requip are the newer agents. Both are somewhat selected for dopamine receptors with highest affinity for the D2 receptor and also activity at the D3 receptor. Direct dopamine agonists, in general, are more likely to produce adverse neuropsychiatric side effects such as confusion than levodopa. Unlike levodopa, direct dopamine agonists do not undergo conversion to dopamine and thus do not produce potentially toxic free radical as they are metabolized. It is also possible that the early use of direct dopamine agonist decreases the propensity to develop the late complications associated with direct stimulation of the dopamine receptor by dopamine itself, such as the "on-off" effect and dyskinesia.

Monoaminoxidase-B inhibitors (MAO) such as selegiline (Diprenyl, or Eldepryl), taken in a low dose, may reduce the progression of Parkinsonism. These compounds can be used as an adjunctive medication. A study has documented that selegiline delays the need for levodopa by roughly three months, although interpretation of this data is confounded by the mild symptomatic benefit of the drug. Nonetheless, theorectical and *in vitro* support for a neuroprotective effect for some members of the selectiv MAOB class of inhibitors remains (*e.g*., rasagiline).

Catechol-O-methyltransferase inhibitors (COMT) can also be used in combination treatments of the invention. Catechol-O-methyltransferase is an enzyme that degrades levodopa, and inhibitors can be used to reduce the rate of degradation. Entacapone is a peripherally acting COMT inhibitor, which can be used in certain methods and compositions of the invention. Tasmar or Tolcapone, approved by the FDA in 1997, can also be used in certain methods and compositions of the invention. Psychiatric adverse effects that are induced or exacerbated by PD medication include psychosis, confusion, agitation, hallucinations, and delusions. These can be treated by decreasing dopamine medication, reducing or discontinuing anticholinergics, amantadine or selegiline or by using low doses of atypical antipsychotics such as clozapine or quetiapine.

Methods of the invention can also be used in combination with surgical therapies for the treatment of PD. Surgical treatment is presently recommended for those who have failed medical management of PD. Unilateral thallamotomy can be used to reduce tremor. It is occasionally considered for patients with unilateral tremor not responding to medication. Bilateral procedures are not advised. Unilateral deep brain stimulation of the thalamus for tremor may also be a benefit for tremor. Unilateral pallidotomy is an effective technique for reducing contralateral drug-induced dyskinesias. Gamma knife surgery-thalamotomy or pallidotomy-can be performed as a radiological alternative to conventional surgery. The currently preferred neurosurgical intervention is, however, bilateral subthalamic nucleus stimulation. Neuronsplantation strategies remain experimental. In addition to surgery and medication, physical therapy in Parkinsonism maintains muscle tone, flexibility, and improves posture and gait.

According to the invention, the term "synucleinopathic subject" also encompasses a subject that is affected by, or is at risk of developing DLBD. These subjects can be readily identified by persons of ordinary skill in the art by symptomatic diagnosis or by genetic screening, brain scans, SPECT, PET imaging etc.

DLBD is the second most common cause of neurodegenerative dementia in older people, it effects 7% of the general population older than 65 years and 30% of those aged over 80 years. It is part of a range of clinical presentations that share a neurotic pathology based on normal aggregation of the synaptic protein α-synuclein. DLBD has many of the clinical and pathological characteristics of the dementia that occurs during the course of Parkinson's disease. A "one year rule" can been used to separate DLBD from PD. According to this rule, onset of dementia within 12 months of Parkinsonism qualifies as DLBD, whereas more than 12 months of Parkinsonism before onset of dementia qualifies as PD. The central features of DLBD include progressive cognitive decline of sufficient magnitude to interfere with normal social and occupational function. Prominent or persistent memory impairment does not necessarily occur in the early stages, but it is evident with progression in most cases. Deficits on tests of attention and of frontal cortical skills and visual spatial ability can be especially prominent.

Core diagnostic features, two of which are essential for diagnosis of probable and one for possible DLBD are fluctuating cognition with pronounced variations in attention and alertness, recurrent visual hallucinations that are typically well-formed and detailed, and spontaneous features of Parkinsonism. In addition, there can be some supportive features, such as repeated falls, syncope, transient loss of consciousness, neuroleptic sensitivity, systematized delusions, hallucinations and other modalities, REM sleep behavior disorder, and depression. Patients with DLBD do better than those with Alzheimer's Disease in tests of verbal memory, but worse on visual performance tests. This profile can be maintained across the range of severity of the disease, but can be harder to recognize in the later stages owing to global difficulties. DLBD typically presents with recurring episodes of confusion on a background of progressive deterioration. Patients with DLBD show a combination of cortical and subcortical neuropsychological impairments with substantial attention deficits and prominent frontal subcortical and visual spatial dysfunction. These help differentiate this disorder from Alzheimer's disease.

Rapid eye movement (REM), sleep behavior disorder is a parasomnia manifested by vivid and frightening dreams associated with simple or complex motor behavior during REM sleep. This disorder is frequently associated with the synucleinopathies, DLBD, PD, and MSA, but it rarely occurs in amyloidopathies and taupathies. The neuropsychological pattern of impairment in REM sleep behavior disorder/dementia is similar to that reported in DLBD and qualitatively different from that reported in Alzheimer's Disease. Neuropathological studies of REM sleep behavior disorder associated with neurodegenerative disorder have shown Lewy body disease or multiple system atrophy. REM sleep wakefulness disassociations (REM sleep behavior disorder, daytime hypersomnolence, hallucinations, cataplexy) characteristic of narcolepsy can explain several features of DLBD, as well as PD. Sleep disorders could contribute to the fluctuations typical of DLBD, and their treatment can improve fluctuations and quality of life. Subjects at risk of developing DLBD can be identified. Repeated falls, syncope, transient loss of consciousness, and depression are common in older people with cognitive impairment and can serve as (a red flag) to a possible diagnosis of DLBD. By contrast, narcoleptic sensitivity in REM sleep behavior disorder can be highly predictive of DLBD. Their detection depends on the clinicians having a high index of suspicion and asking appropriate screening questions.

Clinical diagnosis of synucleinopathic subjects that are affected by or at risk of developing LBD can be supported by neuroimaging investigations. Changes associated with DLBD include preservation of hippocampal, and medialtemporal lobe volume on MRI and occipital hypoperfusion on SPECT. Other features, such as generalized atrophy, white matter changes, and rates of progression of whole brain atrophy are not helpful in differential diagnosis. Dopamine transporter loss in the caudate and putamen, a marker of nigrostriatal degeneration, can be detected by dopamenergic SPECT and can prove helpful in clinical differential diagnosis. A sensitivity of 83% and specificity of 100% has been reported for an abnormal scan with an autopsy diagnosis of DLBD.

Consensus criteria for diagnosing DLBD include ubiquitin immunohistochemistry for Lewy body identification and staging into three categories; brain stem predominant, limbic, or neocortical, depending on the numbers and distribution of Lewy bodies. The recently-developed α-synuclein immunohistochemistry can visualize more Lewy bodies and is also better at indicating previously under recognized neurotic pathology, termed Lewy neurites. Use of antibodies to α-synuclein moves the diagnostic rating for many DLBD cases from brain stem and limbic groups into the neocortical group.

In most patients with DLBD, there are no genetic mutations in the α-synuclein or other Parkinson's disease-associated genes. Pathological up-regulation of normal, wild-type α-synuclein due to increased mRNA expression is a possible mechanism, or Lewy bodies may form because α-synuclein becomes insoluble or more able to aggregate. Another possibility is that α-synuclein is abnormally processed, for example, by a dysfunctional proteasome system and that toxic "proto fibrils" are therefore produced. Sequestering of these toxic fibrils into Lewy bodies could reflect an effort by the neurons to combat biological stress inside the cell, rather than their simply being neurodegenerative debris.

Target symptoms for the accurate diagnosis of DLBD can include extrapyramidal motor features, cognitive impairment, neuropsychiatric features (including hallucinations, depression, sleep disorder, and associated behavioral disturbances), or autonomic dysfunction.

Methods of the invention can be used in combination with one or more other medications for treating DLBD. For example, the lowest acceptable doses of levodopa can be used to treat DLBD. D2-receptor antagonists, particularly traditional neuroleptic agents, can provoke severe sensitivity reactions in DLBD subjects with an increase in mortality of two to three times. Cholinsterase inhibitors dicussed above are also used in the treatment of DLBD.

According to the invention, the term "synucleinopathic subject" also encompasses a subject that is affected by, or is at risk of developing multiple system atrophy (MSA). These subjects can be readily identified by persons of ordinary skill in the art by symptomatic diagnosis and neurological examination sometimes in conjunction with genetic screening, brain scans, SPECT, PET imaging, etc.

MSA is a neurodegenerative disease marked by a combination of symptoms; affecting movement, cognition, autonomic and other body functions, hence the label "multiple system atrophy". The cause of MSA is unknown. Symptoms of MSA vary in distribution of onset and severity from person to person. Because of this, the nomenclature initially included three distinct terms: Shy-Drager syndrome, striatonigral degeneration (SD), and olivopontocerebellar atrophy (OPCA).

In Shy-Drager syndrome, the most prominent symptoms are those involving the autonomic system; blood pressure, urinary function, and other functions not involving conscious control. Striatonigral degeneration causes Parkinsonism symptoms, such as slowed movements and rigidity, while OPCA principally affects balance, coordination and speech. The symptoms for MSA can also include orthostatic hypertension, male impotence, urinary difficulties, constipation, speech and swallowing difficulties, and blurred vision.

The initial diagnosis of MSA is usually made by carefully interviewing the patient and performing a physical examination. Several types of brain imaging, including computer tomography, scans, magnetic resonance imaging (MRI), and positron emission tomography (PET), can be used as corroborative studies. An incomplete and relatively poor response to dopamine replacement therapy, such as Sinemet, may be a clue that the presentation of bradykinesia and rigidity (parkinsonism) is not due to PD. A characteristic involvement of multiple brain systems with prominent autonomic dysfunction is a defining feature of MSA and one that at autopsy confirms the diagnosis. Patients with MSA can have the presence of glial cytoplasmic inclusions in certain types of brain cells, as well. Prototypic Lewy bodies are not present in MSA. However, α-synuclein staining by immunohistochemistry is prominent. In comparison to Parkinson's, in addition to the poor response to Sinemet, there are a few other observations that are strongly suggested for MSA, such as postural instability, low blood pressure on standing (orthostatic hypotension) and high blood pressure when lying down, urinary difficulties, impotence, constipation, speech and swallowing difficulties out of proportion to slowness and rigidity.

Methods of the invention can be used in combination with one or more alternative medications for treating MSA. Typically, the drugs that can be used to treat various symptoms of MSA become less effective as the disease progresses. Levodopa and dopamine agonists used to treat PD are sometimes effective for the slowness and rigidity of MSA. Orthostatic hypertension can be improved with cortisone, midodrine, or other drugs that raise blood pressure. Male impotence may be treated with penile implants or drugs. Incontinence may be treated with medication or catheterization. Constipation may improve with increased dietary fiber or laxatives.

In addition to providing methods for treating synucleinopathies, the invention also provides methods for treating other neurodegenerative diseases. Other neurodegenerative diseases that may be treated include Alzheimer's disease (AD), Huntington's disease (HD), and amyotrophic lateral sclerosis (ALS). Methods of the invention can be used in combination with one or more other medications for treating a neurodegnerative disease.

According to the invention, the term "treatment" includes prophylaxis and therapy, and includes managing a subject's symptoms and halting the progression of the disease. Treatment includes preventing, slowing, stopping, or reversing (*e.g.,* curing) the development of a synucleinopathy or other neurodegenerative disease, and/or the onset of certain symptoms associated with a synucleinopathy or other neurodegenerative disease in a subject with, or at risk of developing, a synucleinopathy, a related disorder, or other neurodegnerative disease. For the treatment of a synucleinopathy, the therapy typically includes preventing, slowing, stopping or reversing (*e.g.,* curing) the accumulation of α-synuclein in a subject with a synucleinopathy. Therapy also includes decreasing the amount of accumulated α-synuclein in a subject with a synucleinopathy.

The phrase "therapeutically-effective amount" as used herein means that amount of a compound, material, or composition comprising a compound of the present invention which is effective for producing some desired therapeutic effect in a subject at a reasonable benefit/risk ratio applicable to any medical treatment. Accordingly, a therapeutically effective amount prevents, minimizes, or reverses disease progression associated with a synucleinopathy or other neurodegenerative disease. Disease progression can be monitored by clinical observations, laboratory, and neuroimaging investigations apparent to a person skilled in the art. A therapeutically effective amount can be an amount that is effective in a single dose or an amount that is effective as part of a multi-dose therapy, for example an amount that is administered in two or more doses or an amount that is administered chronically.

The "pharmaceutically acceptable acid or base addition salts" mentioned herein are meant to comprise the therapeutically active non-toxic acid and non-toxic base addition salt forms that the compounds are able to form. The compounds that have basic properties can be converted into their pharmaceutically acceptable acid addition salts by treating the base form with an appropriate acid. Appropriate acids include, for example, inorganic acids such as hydrohalic acids, *e.g.,* hydrochloric or hydrobromic acid; sulfuric; nitric; phosphoric and the like acids; or organic acids such as, for example, acetic, propanoic, hydroxyacetic, lactic, pyruvic, oxalic, malonic, succinic (*i.e.,* butanedioic acid), maleic, fumaric, malic, tartaric, citric, methanesulfonic, ethanesulfonic, benzenesulfonic, p-toluenesulfonic, cyclamic, salicylic, p-aminosalicylic, pamoic and the like acids. In certain embodiments, the salt is a tartrate salt. The tartrate salt may be either L-tartric acid or D-tartric acid. Both tartric acids are available from Aldrich Chemical Company, Inc. (Milwaukee, Wisconsin). The salts may be anhydrous or hydrous forms.

The compounds that have acidic properties can be converted into their pharmaceutically acceptable base addition salts by treating the acid form with a suitable organic or inorganic base. Appropriate base salt forms include, for example, the ammonium salts, the alkali and earth alkaline metal salts, e.g. the lithium, sodium, potassium, magnesium, calcium salts and the like, salts with organic bases, e.g. the benzathine, N-methyl-D-glucamine, hydrabamine salts, and salts with amino acids such as, for example, arginine, lysine and the like.

The terms acid or base addition salt also comprise the hydrates and the solvent addition forms which the compounds are able to form. Examples of such forms are e.g. hydrates, alcoholates and the like.

The term stereochemically isomeric forms of compounds, as used herein, include all possible compounds made up of the same atoms bonded by the same sequence of bonds but having different three-dimensional structures which are not interchangeable, which the compounds may possess. Unless otherwise mentioned or indicated, the chemical designation of a compound encompasses the mixture of all possible stereochemically isomeric forms that the compound can take. The mixture can contain all diastereomers and/or enantiomers of the basic molecular structure of the compound. All stereochemically isomeric forms of the compounds both in pure form or in admixture with each other are intended to be embraced within the scope of the present invention.

Some of the compounds may also exist in their tautomeric forms. Such forms although not explicitly indicated in the above formula are intended to be included within the scope of the present invention.

The methods and structures described herein relating to compounds and compositions of the invention also apply to the pharmaceutically acceptable acid or base addition salts and all stereoisomeric forms of these compounds and compositions.

In the compounds and compositions of the invention, the term "alkyl" refers to the radical of saturated aliphatic groups, including straight-chain alkyl groups, branched-chain alkyl groups, cycloalkyl (alicyclic) groups, alkyl substituted cycloalkyl groups, and cycloalkyl substituted alkyl groups. In preferred embodiments, a straight chain or branched chain alkyl has 12 or fewer carbon atoms in its backbone (*e.g*., C₁-C₁₂ for straight chain, C₃-C₁₂ for branched chain), and more preferably 6 or fewer, and even more preferably 4 or fewer. Likewise, preferred cycloalkyls have from 3-10 carbon atoms in their ring structure, and more preferably have 5, 6, or 7 carbons in the ring structure.

Unless the number of carbons is otherwise specified, "lower alkyl" as used herein means an alkyl group, as defined above, but having from one to ten carbons, more preferably from one to six carbon atoms in its backbone structure, and even more preferably from one to four carbon atoms in its backbone structure. Likewise, "lower alkenyl" and "lower alkynyl" have similar chain lengths. Preferred alkyl groups are lower alkyls. In preferred embodiments, a substituent designated herein as alkyl is a lower alkyl.

As used herein, the term "halogen" designates -F, -Cl, -Br or -I; the term "sulfhydryl" means -SH; and the term "hydroxyl" means -OH.

The term "methyl" refers to the monovalent radical -CH₃, and the term "methoxyl" refers to the monovalent radical -CH₂OH.

The term "aralkyl" or "arylalkyl", as used herein, refers to an alkyl group substituted with an aryl group (*e.g*., an aromatic or heteroaromatic group).

The terms "alkenyl" and "alkynyl" refer to unsaturated aliphatic groups analogous in length and possible substitution to the alkyls described above, but that contain at least one double or triple bond respectively.

The term "aryl" as used herein includes 5-, 6- and 7-membered single-ring aromatic groups that may include from zero to four heteroatoms, for example, benzene, pyrrole, furan, thiophene, imidazole, oxazole, thiazole, triazole, pyrazole, pyridine, pyrazine, pyridazine and pyrimidine, and the like. Those aryl groups having heteroatoms in the ring structure may also be referred to as "aryl heterocycles" or "heteroaromatics." The aromatic ring can be substituted at one or more ring positions with such substituents as described above, for example, halogen, azide, alkyl, aralkyl, alkenyl, alkynyl, cycloalkyl, hydroxyl, alkoxyl, amino, nitro, sulfhydryl, imino, amido, phosphonate, phosphinate, carbonyl, carboxyl, silyl, ether, alkylthio, sulfonyl, sulfonamido, ketone, aldehyde, ester, heterocyclyl, aromatic or heteroaromatic moieties, -CF₃, -CN, or the like. The term "aryl" also includes polycyclic ring systems having two or more cyclic rings in which two or more carbons are common to two adjoining rings (the rings are "fused rings") wherein at least one of the rings is aromatic, *e.g.,* the other cyclic rings can be cycloalkyls, cycloalkenyls, cycloalkynyls, aryls and/or heterocyclyls.

The terms "ortho", "meta", and "para" apply to 1,2-, 1,3- and 1,4-disubstituted benzenes, respectively. For example, the names 1,2-dimethylbenzene and ortho-dimethylbenzene are synonymous.

The terms "heterocyclyl" or "heterocyclic group" or "heteroaryl" refer to 3- to 10-membered ring structures, more preferably 3- to 7-membered rings, whose ring structures include one to four heteroatoms. Heterocycles can also be polycycles. Heterocyclyl groups include, for example, thiophene, benzothiophene, thianthrene, furan, pyran, isobenzofuran, chromene, xanthene, phenoxathiin, pyrrole, imidazole, pyrazole, isothiazole, isoxazole, pyridine, pyrazine, pyrimidine, pyridazine, indolizine, isoindole, indole, indazole, purine, quinolizine, isoquinoline, quinoline, phthalazine, naphthyridine, quinoxaline, quinazoline, cinnoline, pteridine, carbazole, carboline, phenanthridine, acridine, pyrimidine, phenanthroline, phenazine, phenarsazine, phenothiazine, furazan, phenoxazine, pyrrolidine, oxolane, thiolane, oxazole, piperidine, piperazine, morpholine, lactones, lactams such as azetidinones and pyrrolidinones, sultams, sultones, and the like. The heterocyclic ring can be substituted at one or more positions with such substituents as described above, as for example, halogen, alkyl, aralkyl, alkenyl, alkynyl, cycloalkyl, hydroxyl, amino, nitro, sulfhydryl, imino, amido, phosphonate, phosphinate, carbonyl, carboxyl, silyl, ether, alkylthio, sulfonyl, ketone, aldehyde, ester, a heterocyclyl, an aromatic or heteroaromatic moiety, -CF₃, -CN, or the like.

As used herein, the definition of each expression, *e.g*., alkyl, m, n, *etc.,* when it occurs more than once in any structure, is intended to be independent of its definition elsewhere in the same structure.

It will be understood that "substitution" or "substituted with" includes the implicit proviso that such substitution is in accordance with permitted valence of the substituted atom and the substituent, and that the substitution results in a stable compound, *e.g*., which does not spontaneously undergo transformation such as by rearrangement, cyclization, elimination, *etc.*

As used herein, the term "substituted" is contemplated to include all permissible substituents of organic compounds. In a broad aspect, the permissible substituents include acyclic and cyclic, branched and unbranched, carbocyclic and heterocyclic, aromatic and nonaromatic substituents of organic compounds. Illustrative substituents include, for example, those described herein above. The permissible substituents can be one or more and the same or different for appropriate organic compounds. For purposes of this invention, the heteroatoms such as nitrogen may have hydrogen substituents and/or any permissible substituents of organic compounds described herein which satisfy the valences of the heteroatoms. This invention is not intended to be limited in any manner by the permissible substituents of organic compounds.

Certain compounds of the present invention may exist in particular geometric or stereoisomeric forms. The present invention contemplates all such compounds, including *cis-*and trans-isomers, R- and S-enantiomers, diastereomers, (D)-isomers, (L)-isomers, the racemic mixtures thereof, and other mixtures thereof, as falling within the scope of the invention. Additional asymmetric carbon atoms may be present in a substituent such as an alkyl group. All such isomers, as well as mixtures thereof, are intended to be included in this invention. In certain embodiments, the present invention relates to a compound represented by any of the structures outlined herein, wherein the compound is a single stereoisomer.

If, for instance, a particular enantiomer of a compound of the present invention is desired, it may be prepared by asymmetric synthesis, or by derivation with a chiral auxiliary, where the resulting diastereomeric mixture is separated and the auxiliary group cleaved to provide the pure desired enantiomers. Alternatively, where the molecule contains a basic functional group, such as amino, or an acidic functional group, such as carboxyl, diastereomeric salts are formed with an appropriate optically-active acid or base, followed by resolution of the diastereomers thus formed by fractional crystallization or chromatographic means well known in the art, and subsequent recovery of the pure enantiomers.

Contemplated equivalents of the compounds described above include compounds which otherwise correspond thereto, and which have the same general properties thereof (e.g., functioning as anti-synucleinopathy farnesyl transferase inhibitor compounds), wherein one or more simple variations of substituents are made which do not adversely affect the efficacy of the compound. In general, the compounds of the present invention may be prepared by the methods illustrated in the general reaction schemes as, for example, described below, or by modifications thereof, using readily available starting materials, reagents and conventional synthesis procedures. In these reactions, it is also possible to make use of variants, which are in themselves known, but are not mentioned here.

For purposes of this invention, the chemical elements are identified in accordance with the Periodic Table of the Elements, CAS version, Handbook of Chemistry and Physics, 67th Ed., 1986-87, inside cover.

In another aspect, the present invention provides "pharmaceutically acceptable" compositions, which comprise a therapeutically effective amount of one or more of the compounds described herein, formulated together with one or more pharmaceutically acceptable carriers (additives) and/or diluents. As described in detail, the pharmaceutical compositions of the present invention may be specially formulated for administration in solid or liquid form, including those adapted for the following: oral administration, for example, drenches (aqueous or non-aqueous solutions or suspensions), tablets, e.g., those targeted for buccal, sublingual, and systemic absorption, boluses, powders, granules, pastes for application to the tongue; parenteral administration, for example, by subcutaneous, intramuscular, intravenous or epidural injection as, for example, a sterile solution or suspension, or sustained-release formulation; topical application, for example, as a cream, ointment, or a controlled-release patch or spray applied to the skin, lungs, or oral cavity; intravaginally or intrarectally, for example, as a pessary, cream or foam; sublingually; ocularly; transdermally; or nasally, pulmonary and to other mucosal surfaces.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically-acceptable carrier" as used herein means a pharmaceutically-acceptable material, composition or vehicle, such as a liquid or solid filler, diluent, excipient, or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: sugars, such as lactose, glucose and sucrose; starches, such as com starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, com oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; pH buffered solutions; polyesters, polycarbonates and/or polyanhydrides; and other non-toxic compatible substances employed in pharmaceutical formulations.

As set out herein, certain embodiments of the present compounds may contain a basic functional group, such as amino or alkylamino, and are, thus, capable of forming pharmaceutically-acceptable salts with pharmaceutically-acceptable acids. The term "pharmaceutically-acceptable salts" in this respect refers to the relatively non-toxic, inorganic and organic acid addition salts of compounds of the present invention. These salts can be prepared *in situ* in the administration vehicle or the dosage form manufacturing process, or by separately reacting a purified compound ofthe invention in its free base form with a suitable organic or inorganic acid, and isolating the salt thus formed during subsequent purification. Representative salts include the hydrobromide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, napthylate, mesylate, glucoheptonate, lactobionate, and laurylsulphonate salts and the like. (See, for example, Berge et al. (1977) "Pharmaceutical Salts", J. Pharm. Sci. 66:1-19; incorporated herein by reference)

The pharmaceutically acceptable salts of the subject compounds include the conventional nontoxic salts or quaternary ammonium salts of the compounds, e.g., from non-toxic organic or inorganic acids. For example, such conventional nontoxic salts include those derived from inorganic acids such as hydrochloride, hydrobromic, sulfuric, sulfamic, phosphoric, nitric, and the like; and the salts prepared from organic acids such as acetic, propionic, succinic, glycolic, stearic, lactic, malic, tartaric, citric, ascorbic, palmitic, maleic, hydroxymaleic, phenylacetic, glutamic, benzoic, salicyclic, sulfanilic, 2-acetoxybenzoic, fumaric, toluenesulfonic, ethanesulfonic, ethane disulfonic, oxalic, isothionic, and the like.

In other cases, the compounds of the present invention may contain one or more acidic functional groups and, thus, are capable of forming pharmaceutically-acceptable salts with pharmaceutically-acceptable bases. The term "pharmaceutically-acceptable salts" in these instances refers to the relatively non-toxic, inorganic and organic base addition salts of compounds of the present invention. These salts can likewise be prepared *in situ* in the administration vehicle or the dosage form manufacturing process, or by separately reacting the purified compound in its free acid form with a suitable base, such as the hydroxide, carbonate or bicarbonate of a pharmaceutically-acceptable metal cation, with ammonia, or with a pharmaceutically-acceptable organic primary, secondary or tertiary amine. Representative alkali or alkaline earth salts include the lithium, sodium, potassium, calcium, magnesium, and aluminum salts and the like. Representative organic amines useful for the formation of base addition salts include ethylamine, diethylamine, ethylenediamine, ethanolamine, diethanolamine, piperazine and the like. (See, for example, Berge et al., *supra*).

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically-acceptable antioxidants include: water soluble antioxidants, such as ascorbic acid, cysteine hydrochloride, sodium bisulfate, sodium metabisulfite, sodium sulfite and the like; oil-soluble antioxidants, such as ascorbyl palmitate, butylated hydroxyanisole (BHA), butylated hydroxytoluene (BHT), lecithin, propyl gallate, alpha-tocopherol, and the like; and metal chelating agents, such as citric acid, ethylenediamine tetraacetic acid (EDTA), sorbitol, tartaric acid, phosphoric acid, and the like.

Formulations of the present invention include those suitable for oral, nasal, topical (including buccal and sublingual), rectal, vaginal and/or parenteral administration. The formulations may conveniently be presented in unit dosage form and may be prepared by any methods well known in the art of pharmacy. The amount of active ingredient which can be combined with a carrier material to produce a single dosage form will vary depending upon the host being treated, and the particular mode of administration. The amount of active ingredient that can be combined with a carrier material to produce a single dosage form will generally be that amount of the compound which produces a therapeutic effect. Generally, this amount will range from about 1% to about 99% of active ingredient, preferably from about 5% to about 70%, most preferably from about 10% to about 30%.

In certain embodiments, a formulation of the present invention comprises an excipient selected from the group consisting of cyclodextrins, liposomes, micelle forming agents, e.g., bile acids, and polymeric carriers, e.g., polyesters and polyanhydrides; and a compound of the present invention. In certain embodiments, an aforementioned formulation renders orally bioavailable a compound of the present invention.

Methods of preparing these formulations or compositions include the step of bringing into association a compound ofthe present invention with the carrier and, optionally, one or more accessory ingredients. In general, the formulations are prepared by uniformly and intimately bringing into association a compound of the present invention with liquid carriers, or finely divided solid carriers, or both, and then, if necessary, shaping the product.

Formulations of the invention suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active ingredient. A compound of the present invention may also be administered as a bolus, electuary or paste.

In solid dosage forms of the invention for oral administration (capsules, tablets, pills, dragees, powders, granules and the like), the active ingredient is mixed with one or more pharmaceutically-acceptable carriers, such as sodium citrate or dicalcium phosphate, and/or any of the following: fillers or extenders, such as starches, lactose, sucrose, glucose, mannitol, and/or silicic acid; binders, such as, for example, carboxymethylcellulose, alginates, gelatin, polyvinyl pyrrolidone, sucrose and/or acacia; humectants, such as glycerol; disintegrating agents, such as agar-agar, calcium carbonate, potato or tapioca starch, alginic acid, certain silicates, and sodium carbonate; solution retarding agents, such as paraffin; absorption accelerators, such as quaternary ammonium compounds; wetting agents, such as, for example, cetyl alcohol, glycerol monostearate, and non-ionic surfactants; absorbents, such as kaolin and bentonite clay; lubricants, such as talc, calcium stearate, magnesium stearate, solid polyethylene glycols, sodium lauryl sulfate, and mixtures thereof; and coloring agents. In the case of capsules, tablets and pills, the pharmaceutical compositions may also comprise buffering agents. Solid compositions of a similar type may also be employed as fillers in soft and hard-shelled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made in a suitable machine in which a mixture of the powdered compound is moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be formulated for rapid release, e.g., freeze-dried. They may be sterilized by, for example, filtration through a bacteria-retaining filter, or by incorporating sterilizing agents in the form of sterile solid compositions that can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions that can be used include polymeric substances and waxes. The active ingredient can also be in micro-encapsulated form, if appropriate, with one or more of the above-described excipients.

Liquid dosage forms for oral administration of the compounds of the invention include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, com, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations of the pharmaceutical compositions of the invention for rectal or vaginal administration may be presented as a suppository, which may be prepared by mixing one or more compounds of the invention with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active compound.

Formulations of the present invention which are suitable for vaginal administration also include pessaries, tampons, creams, gels, pastes, foams or spray formulations containing such carriers as are known in the art to be appropriate.

Dosage forms for the topical or transdermal administration of a compound of this invention include powders, sprays, ointments, pastes, creams, lotions, gels, solutions, patches and inhalants. The active compound may be mixed under sterile conditions with a pharmaceutically-acceptable carrier, and with any preservatives, buffers, or propellants which may be required.

The ointments, pastes, creams and gels may contain, in addition to an active compound of this invention, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a compound of this invention, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Transdermal patches have the added advantage of providing controlled delivery of a compound of the present invention to the body. Dissolving or dispersing the compound in the proper medium can make such dosage forms. Absorption enhancers can also be used to increase the flux of the compound across the skin. Either providing a rate controlling membrane or dispersing the compound in a polymer matrix or gel can control the rate of such flux.

Ophthalmic formulations, eye ointments, powders, solutions and the like, are also contemplated as being within the scope of this invention.

Pharmaceutical compositions of this invention suitable for parenteral administration comprise one or more compounds of the invention in combination with one or more pharmaceutically-acceptable sterile isotonic aqueous or nonaqueous solutions, dispersions, suspensions or emulsions, or sterile powders which may be reconstituted into sterile injectable solutions or dispersions just prior to use, which may contain sugars, alcohols, antioxidants, buffers, bacteriostats, solutes which render the formulation isotonic with the blood of the intended recipient or suspending or thickening agents.

Examples of suitable aqueous and nonaqueous carriers, which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms upon the subject compounds may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution, which in turn, may depend upon crystal size and crystalline form.

Alternatively, delayed absorption of a parenterally-administered drug form is accomplished by dissolving or suspending the drug in an oil vehicle.

Injectable depot forms are made by forming microencapsule matrices of the subject compounds in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions, which are compatible with body tissue.

In certain embodiments, a compound or pharmaceutical preparation is administered orally. In other embodiments, the compound or pharmaceutical preparation is administered intravenously. Alternative routs of administration include sublingual, intramuscular, and transdermal administrations.

When the compounds of the present invention are administered as pharmaceuticals, to humans and animals, they can be given per se or as a pharmaceutical composition containing, for example, 0.1% to 99.5% (more preferably, 0.5% to 90%) of active ingredient in combination with a pharmaceutically acceptable carrier.

The preparations of the present invention may be given orally, parenterally, topically, or rectally. They are of course given in forms suitable for each administration route. For example, they are administered in tablets or capsule form, by injection, inhalation, eye lotion, ointment, suppository, etc. administration by injection, infusion or inhalation; topical by lotion or ointment; and rectal by suppositories. Oral administrations are preferred.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticulare, subcapsular, subarachnoid, intraspinal and intrasternal injection and infusion.

The phrases "systemic administration," "administered systemically," "peripheral administration" and "administered peripherally" as used herein mean the administration of a compound, drug or other material other than directly into the central nervous system, such that it enters the patient's system and, thus, is subject to metabolism and other like processes, for example, subcutaneous administration.

These compounds may be administered to humans and other animals for therapy by any suitable route of administration, including orally, nasally, as by, for example, a spray, rectally, intravaginally, parenterally, intracisternally and topically, as by powders, ointments or drops, including buccally and sublingually.

Regardless of the route of administration selected, the compounds of the present invention, which may be used in a suitable hydrated form, and/or the pharmaceutical compositions of the present invention, are formulated into pharmaceutically-acceptable dosage forms by conventional methods known to those of skill in the art.

Actual dosage levels of the active ingredients in the pharmaceutical compositions of this invention may be varied so as to obtain an amount of the active ingredient that is effective to achieve the desired therapeutic response for a particular patient, composition, and mode of administration, without being toxic to the patient.

The selected dosage level will depend upon a variety of factors including the activity of the particular compound of the present invention employed, or the ester, salt or amide thereof, the route of administration, the time of administration, the rate of excretion or metabolism of the particular compound being employed, the duration of the treatment, other drugs, compounds and/or materials used in combination with the particular compound employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the compounds of the invention employed in the pharmaceutical composition at levels lower than that required to achieve the desired therapeutic effect and then gradually increasing the dosage until the desired effect is achieved.

In some embodiments, a compound or pharmaceutical composition of the invention is provided to a synucleinopathic subject chronically. Chronic treatments include any form of repeated administration for an extended period of time, such as repeated administrations for one or more months, between a month and a year, one or more years, or longer. In many embodiments, a chronic treatment involves administering a compound or pharmaceutical composition of the invention repeatedly over the life of the synucleinopathic subject. Preferred chronic treatments involve regular administrations, for example one or more times a day, one or more times a week, or one or more times a month. In general, a suitable dose such as a daily dose of a compound of the invention will be that amount of the compound that is the lowest dose effective to produce a therapeutic effect. Such an effective dose will generally depend upon the factors described above. Generally doses of the compounds of this invention for a patient, when used for the indicated effects, will range from about 0.0001 to about 100 mg per kg of body weight per day. Preferably the daily dosage will range from 0.001 to 50 mg of compound per kg of body weight, and even more preferably from 0.01 to 10 mg of compound per kg of body weight. However, lower or higher doses can be used. In some embodiments, the dose administered to a subject may be modified as the physiology of the subject changes due to age, disease progression, weight, or other factors.

If desired, the effective daily dose of the active compound may be administered as two, three, four, five, six or more sub-doses administered separately at appropriate intervals throughout the day, optionally, in unit dosage forms.

While it is possible for a compound of the present invention to be administered alone, it is preferable to administer the compound as a pharmaceutical formulation (composition) as described above.

The compounds according to the invention may be formulated for administration in any convenient way for use in human or veterinary medicine, by analogy with other pharmaceuticals.

According to the invention, compounds for treating neurological conditions or diseases can be formulated or administered using methods that help the compounds cross the blood-brain barrier (BBB). The vertebrate brain [and CNS] has a unique capillary system unlike that in any other organ in the body. The unique capillary system has morphologic characteristics which make up the blood-brain barrier (BBB). The blood-brain barrier acts as a system-wide cellular membrane that separates the brain interstitial space from the blood.

The unique morphologic characteristics ofthe brain capillaries that make up the BBB are: (a) epithelial-like high resistance tight junctions which literally cement all endothelia of brain capillaries together, and (b) scanty pinocytosis or transendothelial channels, which are abundant in endothelia of peripheral organs. Due to the unique characteristics of the blood-brain barrier, hydrophilic drugs and peptides that readily gain access to other tissues in the body are barred from entry into the brain or their rates of entry and/or accumulation in the brain are very low.

In one aspect of the invention, farnesyl transferase inhibitor compounds that cross the BBB are particularly useful for treating synucleinopathies. In one embodiment, it is expected that farnesyl transferase inhibitors that are non-charged (e.g., not positively charged) and/or non-lipophilic may cross the BBB with higher efficiency than charged (e.g., positively charged) and/or lipophilic compounds. Therefore it will be appreciated by a person of ordinary skill in the art that some of the compounds of the invention might readily cross the BBB. Alternatively, the compounds of the invention can be modified, for example, by the addition of various substitutuents that would make them less hydrophilic and allow them to more readily cross the BBB.

Various strategies have been developed for introducing those drugs into the brain which otherwise would not cross the blood-brain barrier. Widely used strategies involve invasive procedures where the drug is delivered directly into the brain. One such procedure is the implantation of a catheter into the ventricular system to bypass the blood-brain barrier and deliver the drug directly to the brain. These procedures have been used in the treatment of brain diseases which have a predilection for the meninges, e.g., leukemic involvement of the brain (US 4,902,505, incorporated herein in its entirety by reference).

Although invasive procedures for the direct delivery of drugs to the brain ventricles have experienced some success, they are limited in that they may only distribute the drug to superficial areas of the brain tissues, and not to the structures deep within the brain. Further, the invasive procedures are potentially harmful to the patient.

Other approaches to circumventing the blood-brain barrier utilize pharmacologic-based procedures involving drug latentiation or the conversion of hydrophilic drugs into lipid-soluble drugs. The majority of the latentiation approaches involve blocking the hydroxyl, carboxyl and primary amine groups on the drug to make it more lipid-soluble and therefore more easily able to cross the blood-brain barrier.

Another approach to increasing the permeability of the BBB to drugs involves the intra-arterial infusion of hypertonic substances which transiently open the blood-brain barrier to allow passage of hydrophilic drugs. However, hypertonic substances are potentially toxic and may damage the blood-brain barrier.

Peptide compositions of the invention may be administered using chimeric peptides wherein the hydrophilic peptide drug is conjugated to a transportable peptide, capable of crossing the blood-brain barrier by transcytosis at a much higher rate than the hydrophilic peptides alone. Suitable transportable peptides include, but are not limited to, histone, insulin, transferrin, insulin-like growth factor I (IGF-I), insulin-like growth factor II (IGF-II), basic albumin and prolactin.

Antibodies are another method for delivery of compositions of the invention. For example, an antibody that is reactive with a transferrin receptor present on a brain capillary endothelial cell, can be conjugated to a neuropharmaceutical agent to produce an antibody-neuropharmaceutical agent conjugate (US 5,004,697, incorporated herein in its entirety by reference). The method is conducted under conditions whereby the antibody binds to the transferrin receptor on the brain capillary endothelial cell and the neuropharmaceutical agent is transferred across the blood brain barrier in a pharmaceutically active form. The uptake or transport of antibodies into the brain can also be greatly increased by cationizing the antibodies to form cationized antibodies having an isoelectric point of between about 8.0 to 11.0 (US 5,527,527, incorporated herein in its entirety by reference).

A ligand-neuropharmaceutical agent fusion protein is another method useful for delivery of compositions to a host (US 5,977,307, incorporated herein in its entirety by reference). The ligand is reactive with a brain capillary endothelial cell receptor. The method is conducted under conditions whereby the ligand binds to the receptor on a brain capillary endothelial cell and the neuropharmaceutical agent is transferred across the blood brain barrier in a pharmaceutically active form. In some embodiments, a ligand-neuropharmaceutical agent fusion protein, which has both ligand binding and neuropharmaceutical characteristics, can be produced as a contiguous protein by using genetic engineering techniques. Gene constructs can be prepared comprising DNA encoding the ligand fused to DNA encoding the protein, polypeptide or peptide to be delivered across the blood brain barrier. The ligand coding sequence and the agent coding sequence are inserted in the expression vectors in a suitable manner for proper expression of the desired fusion protein. The gene fusion is expressed as a contiguous protein molecule containing both a ligand portion and a neuropharmaceutical agent portion.

The permeability of the blood brain barrier can be increased by administering a blood brain barrier agonist, for example bradykinin (US 5,112,596, incorporated herein in its entirety by reference), or polypeptides called receptor mediated permeabilizers (RMP) (US 5,268,164, incorporated herein in its entirety by reference). Exogenous molecules can be administered to the host's bloodstream parenterally by subcutaneous, intravenous or intramuscular injection or by absorption through a bodily tissue, such as the digestive tract, the respiratory system or the skin. The form in which the molecule is administered (e.g., capsule, tablet, solution, emulsion) depends, at least in part, on the route by which it is administered. The administration of the exogenous molecule to the host's bloodstream and the intravenous injection of the agonist of blood-brain barrier permeability can occur simultaneously or sequentially in time. For example, a therapeutic drug can be administered orally in tablet form while the intravenous administration of an agonist of blood-brain barrier permeability is given later (*e.g*., between 30 minutes later and several hours later). This allows time for the drug to be absorbed in the gastrointestinal tract and taken up by the bloodstream before the agonist is given to increase the permeability of the blood-brain barrier to the drug. On the other hand, an agonist of blood-brain barrier permeability (*e.g*., bradykinin) can be administered before or at the same time as an intravenous injection of a drug. Thus, the term "co-administration" is used herein to mean that the agonist of blood-brain barrier and the exogenous molecule will be administered at times that will achieve significant concentrations in the blood for producing the simultaneous effects of increasing the permeability of the blood-brain barrier and allowing the maximum passage of the exogenous molecule from the blood to the cells of the central nervous system.

In other embodiments, compounds of the invention can be formulated as a prodrug with a fatty acid carrier (and optionally with another neuroactive drug). The prodrug is stable in the environment of both the stomach and the bloodstream and may be delivered by ingestion. The prodrug passes readily through the blood brain barrier. The prodrug preferably has a brain penetration index of at least two times the brain penetration index of the drug alone. Once in the central nervous system, the prodrug, which preferably is inactive, is hydrolyzed into the fatty acid carrier and the farnesyl transferase inhibitor (and optionally another drug). The carrier preferably is a normal component of the central nervous system and is inactive and harmless. The compound and/or drug, once released from the fatty acid carrier, is active. Preferably, the fatty acid carrier is a partially-saturated straight chain molecule having between about 16 and 26 carbon atoms, and more preferably 20 and 24 carbon atoms. Examples of fatty acid carriers are provided in US Patents. 4,939,174; 4,933,324; 5,994,932; 6,107,499; 6,258,836; and 6,407,137, the disclosures of which are incorporated herein by reference in their entirety.

The administration of the agents of the present invention may be for either prophylactic or therapeutic purposes. When provided prophylactically, the agent is provided in advance of disease symptoms. The prophylactic administration of the agent serves to prevent or reduce the rate of onset of symptoms of a synucleinopathy. When provided therapeutically, the agent is provided at (or shortly after) the onset of the appearance of symptoms of actual disease. In some embodiments, the therapeutic administration of the agent serves to reduce the severity and duration of the disease.

The function and advantage of these and other embodiments of the present invention will be more fully understood from the examples described below. The following examples are intended to illustrate the benefits of the present invention, but do not exemplify the full scope of the invention.

### EXAMPLES

### Experimental Procedures

Tissue culture: All cell lines were obtained by ATCC. SH-SY5Y and Cos-7 were grown in 10% FBS DMEM (Sigma). Cells were split the day before experiments including transfection, metabolic labeling and drug treatment.

Proteins and antibodies: UCH-L1 variants were purified according to the published procedure. Synuclein antibody (SYN-1) was purchased from Signal Transduction Lab. Actin antibody and FLAG antibody (M2) were from Sigma. UCH-L1 antibody (anti-PGP 9.5) was from Chemicon.

Chemicals: FTI-277 and lactacystin was purchased from Calbiochem. Crosslinking reagent DE was from Pierce. DMEM and MEM were purchased from Gibco. All the other material was purchased from Sigma.

Plasmids: C220S cDNA was generated by PCR site-specific mutagenesis. For the PCR, the 5' primer is uchforw SEQ ID NO: 1 (CTAAAGCTTATGCAGCTCAAGCCGATGGAG), and 3' primer is uchc220s SEQ ID NO:2 (CTAAGA CTCGAGTTAGGCTGCCTTGCTGAGAGC). Wt UCH-L1 served as the template. The PCR fragment was inserted into pcDNA vector. For S 18YC220S mutant, S 18Y UCH-L1 served as the template in PCR. For the FLAG tagged UCH-L1, the 5' primer is FLAGuchforw SEQ ID NO: 3 (CTAAAGCTTATGGACTACAAGGATGACGACGACAAAGATGCAGCTCAAGC CGATGGAG), and the 3' primer is uchrev SEQ ID NO: 4 (ATCCTCGAGTTAGGCTGCCTTGACGAGAGC). Wt UCH-L1 or C220S served as the template. PCR fragment was purified and inserted into pcDNA vector. For the FLAG tagged UCH-L3, the 5' primer is L3HindIII SEQ ID NO: 5 (CTAAAGCTTATGGACTAC AAGGATGACGACGACAAAGATGGAGGGTCAACGCTGGCTG), the 3'primer is L3XhoISAA SEQ ID NO: 6 (ATCCTCGAGCTATGCTGCAGAAAGAGCAATCGCA). For the UCH-L3 CKAA variant, the 5' primer is L3 HindIII and the 3' primer is L3XhoICKAA SEQ ID NO: 7 (ATCCTCGAGCTATGCTGCCTTAGAAAGAGCAATCGCATTAAATC). α-synuclein degradation assay: Lipofectamine 2000 was used to transfect COS-7 cells according to the Invitrogen protocol. Transfected cells were cultured at 37 °C for 48 hours before being treated with 35 µM lactacystin or DMSO. After 24 hours of incubation, the cells were lysed with Tris buffer (50 mM Tris, 2% SDS, 0.1% NP-40), and subjected to SDS-PAGE, followed by quantitative Western blotting.

Salt and detergent treatment of SV fraction: SV fraction was prepared as describe elsewhere. SV was incubated with various salts at designed concentration for 30 minutes on ice, or 1% Triton X-100 or control without salts and detergent. Treated SV was pelleted at 100,000g for 30 minutes. Supernatants and pellets were subjected to SDS-PAGE and Western blotting.

Membrane fractionation: Cells were harvested by scraping and washed with PBS. Cell pellet was suspended in lysis buffer (50 mM Tris-HCl, 1 mM EDTA) supplemented with protease inhibitor cocktail (Sigma) and homogenized by passing through 26G needles 10 times. Suspension was clarified by spinning at 600g for 5 minutes. Clarified suspension was ultracentrifuged at 100,000g for 2 hours and separated into membrane and cytosol. Membrane fraction was washed with washing buffer (50 mM Tris-HCl, 1 mM EDTA 1 M NaCl), and pelleted each time with bench-top centrifuge.

2D electrophoresis: For the isolation of total cellular protein, cultured SH-SY5Y cells maintained as described above were rinsed with ice-cold PBS. Cells were lysed in 1ml dSDS buffer (50mM Tris-HCl, pH 8.0 0.1% SDS) supplemented with protease inhibitor cocktail. Lysates were boiled for 3 min, and were treated with Dnase and Rnase as described. Lysates were precipitated with ice-cold acetone for at least 2 hours, and pellets were resuspended in 2D sample buffer (8M urea, 0.5% CHAPS, 0.2% DTT, 0.5% IPG buffer, 0.002% bromophenol blue). 2D electrophoresis was carried out according to manufacture's protocol (Amersham Life Science). 7cm pH 4-7 strips were used. For SH-SY5Y membrane fraction, culture SH-SY5Y cells were rinsed with cold PBS and harvested with lysis buffer (50mM Tris-HCl, pH 8.0, 1mM ZnAc2, 250mM sucrose). Lysate was passed through 25G needles for several times and spun at 1000g for 5 min. Supernatant was centrifuged at 200,000g for 2 hours. Pellet was extensively washed with lysis buffer and extracted with cold acetone. Pellet was resuspended in 2D sample buffer.

Viral Infection: Viral infection and MTT assay in SH-SY5Y cells: The viruses were amplified and purified according to the published procedure. SH-SY5Y cells were grown on 100mm petri-dishes and induced with 100nM retinoic acid for 3-5 days before the virus infection with M.I.O at 75. Viruses were diluted with DPBS to desired M.I.O.. After four hours of incubation, 10ml growth medium was added. On the second day, cells were splitted into 96-well plates and treated with compounds for next 48 hours. The growth medium in each well was replaced with growth medium with 5ug/ml MTT. Medium was removed after three hours incubation, and 200ul isopropyl (0.04N HCl) was added into each well. The signal was read at 570nm.

Viable cell counting: At stated time poins, SH-SY5Y cells were trypsinized with 100ul trypsin-EDTA for 1 minute and neutralized with 400ul growth medium. Cell suspension was made up by mixing 0.2 ml of cells in growth medium, 0.3 ml of HBSS and 0.5 ml of 0.4% Trypan Blue solution. Viable cell numbers were counted by standard cell counting chamber.

Western Blotting: Following transfer of SDS gels onto NC membrane, all membranes were blocked with 5% non-fat milk in TBST (50mM Tris-HCl pH7.4, 150mM NaCl, 0.1% Tween 20), and incubated with primary antibody overnight with 1% BSA in TBST, washed three times with TBST, and incubated with horseradish peroxidase-conjugated secondary antibody for 1 hour (Promega). Bound antibodies were detected using enhanced chemiluminascence (NEM).

### Example 1: UCH-L1 is farnesylated in vivo and in cell culture

The UCH-L1 sequence contains the sequence CXXX, a consensus farnesylation site, at its C-terminus. This sequence is not present in UCH-L3. The possibility that this sequence was modified *in vivo* was investigated. First, the chemical nature of the previously reported association of UCH-L1 and synaptic vesicles from rat brain was probed.

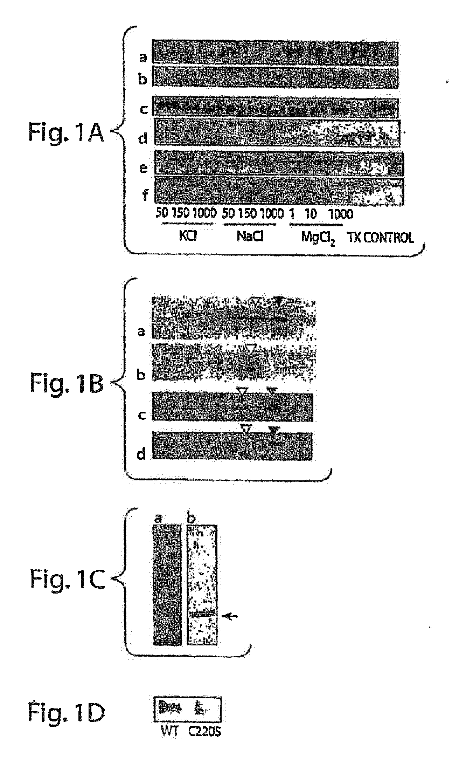

The results are shown in *Figure 1**,* panel A: Effects of various amount of salt and non-ionic detergent on the dissociations of synapsin I, synaphysin and UCH-L1 from SV was analyzed by treating aliquots of SV fraction with either KCl, NaCl, MgCl₂, or 1% Triton X-100. Membrane fraction and soluble fraction was separated by centrifugation and each fraction was subjected to SDS-PAGE followed by Western blots. a (synapsin I), c (synaphysin) and e (UCH-L1) are from pellet, and b (synapsin I), d (synaphysin) and f (UCH-L1) are supernatant fractions. Unlike synapsin (*Figure 1*, panel A, rows a and b), which is not an integral membrane protein, and like synaptophysin (rows c and d), UCH-L1 (rows e and f) could not be separated from the vesicular fraction by increasing salt concentration. Only treatment with detergent was sufficient to solubilize UCH-L1, consistent with its farnesylation.

Analysis of various fractions from SH-SY5Y neuroblastoma cells (similar results from rat brain, not shown) by two-dimensional SDS-PAGE gel electrophoresis showed two major and two minor species in the total homogenate and one species in the membrane-associated fraction (*Figure 1**,* panel B: More than 2 forms of UCH-L1 were present in SH-SY5Y cell (gel a) detected using 2D electrophoretic analysis followed by Western blotting. Only one of them (open arrow) is associated with membrane (gel b). Treatment of SH-SY5Y cells with FTI-277 (gel d) results in a significant decrease in the amount of membrane bound UCH-L1 (open arrow) without affecting the amount of cytosolic UCH-L1 (close arrow) when compared to cells treated with DMSO (gel c). This species was presumably the fully processed species: farnesylated, truncated and C-terminally methylated.

Consistent with this premise, treatment of the cells with the farnesyl transferase inhibitor FTI-277 decreased the amount of the membrane-associated species. In addition, a UCH-L1-containing species was immunoprecipitated from whole cell lysate by an anti-farnesyl antibody (Calbiochem). Finally, treatment of the cells with 14C-mevalonic acid or with 3H-farnesol resulted in incorporation of radiolabel into UCH-L1 (*Figure 1*, panel C). UCH-L1 was modified with [¹⁴C] mevalonate (gel a) and [³H] farnesol (gel b) *in vivo.* (b). Transfection of the C220S mutant into COS-7 cells prevented radioincorporation and eliminated the membrane-associated species (not shown). *Figure 1**,* panel D, shows that WT UCH-L1 but not the C220S variant was detected in the membrane fraction of COS-7 cells transfected with either of the UCH-L1 variants).

### Example 2: Removal of the farnesyltation site has no effect on the in vitro enzymatic activity or aggregation properties of UCH-L1

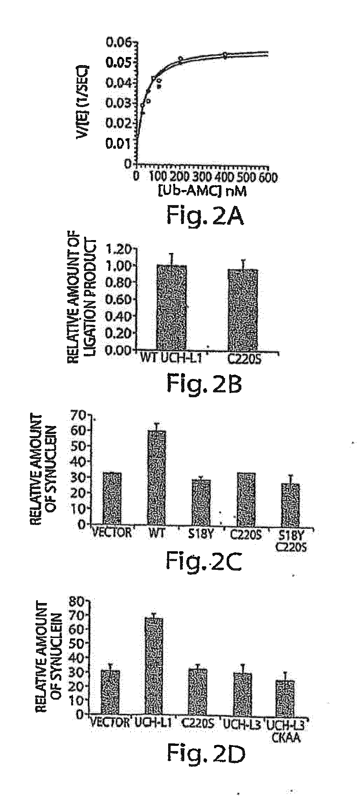

The C220S mutant as expressed in E. coli and purified using a published method. As expected from examination of structural models of UCH-L1, the point mutation had no effect on the *in vitro* hydrolase (*Figure 2*, panel A) or ligase (panel B) activities. (A) Michaelis-Menten plot of various amount Ub-AMC titrated against either UCH-L1 WT (close circle) or C220S (open circle) showed comparable hydrolytic activities. (B) The mutation does not affect UCH-L1 *in vitro* ligase activity. In addition, the C220S mutation did not eliminate the propensity of S 18 to oligomerize. This finding cleared the way to examine the effects of C220S in cell culture.

### Example 3: Farnesylation and membrane association of UCH-L1 is required to promote accumulation of α-synuclein in COS-7 cells.

The C220S mutation eliminated the ability of S 18 to promote α-synuclein accumulation in COS-7 cells but had no effect on the S18Y polymorph (*Figure 2*, panel C: the relative amount of 16kDa α-synuclein was quantified and normalized against the amount of actin in transfected COS-7 cells with the presence of UCH-L1 variants. 100% accumulation of α-synuclein was achieved in cells treated with the proteasome inhibitor lactacystin). This finding suggested that farnesylation and membrane attachment of UCH-L1 are both required. In order to isolate the latter possibility, a mutant form of UCH-L3 was constructed in which the UCH-L1 farnesylation sequence was added to the UCH-L3 C-terminus. This protein did not cause accumulation of α-synuclein (panel (D): The relative amount of α-synuclein in COS-7 cells transfected with UCH-L1 and UCH-L3 variants was compared), although it was farnesylated and incorporated into the membrane (not shown). Thus, membrane attachment of an active hydrolase was insufficient to cause accumulation of α-synuclein.

### Example 4: Inhibition of farnesylation rescues cell death caused by α-synuclein overexpression in SH-SY5Y cells.

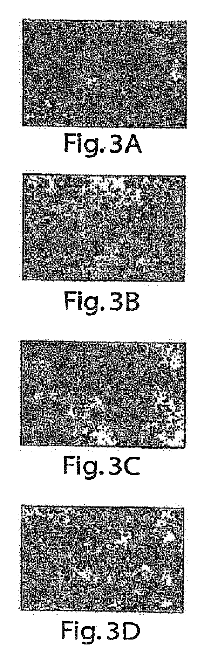

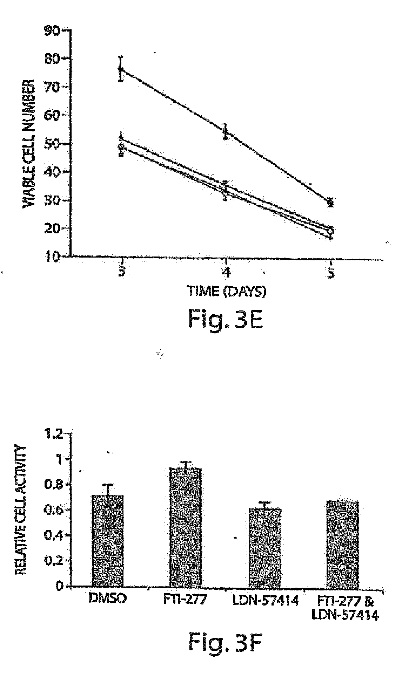

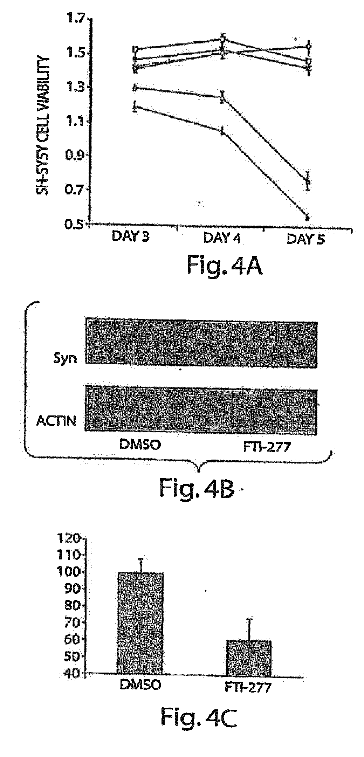

Since α-synuclein neurotoxicity is dose-dependent, it follows that accumulation of α-synuclein, caused by UCH-L1 farnesylation, should promote its toxicity. We demonstrated this to be true in mammalian neuroblastoma SH-SY5Y cells. This dopaminergic cell line has been used to demonstrate the rescue of α-synuclein toxicity by parkin, an effect that has also been demonstrated in primary dopaminergic cultures. These cells express high endogenous levels of UCH-L1. The α-synuclein gene was overexpressed (as compared to endogenous levels) via infection with an adenoviral vector and toxicity was demonstrated by the Trypan blue (*Figure 3*) and MTT assays (*Figure 4*). *Figure 3* shows SH-SY5Y cells infected by α -synuclein-expressing adenovirus treated with DMSO (A), FTI-277 (B), LDN57414 (C), FTI-277 and LDN57414 (D). (E) Viable cell numbers were quantified by counting the cells treated with either DMSO (lower dark circles), FTI-277 (upper dark circles), LDN57414 (light triangles) or LDN57414 and FTI-277 (dark triangles) that did not stain with trypan blue. The unit of y-axis is 10⁵/ml. (F) Cell viability was assessed by the amount of metabolic activity using MTT assay. *Figure 4* shows: (A) the viability of SH-SY5Y cells infected by α-synuclein-expressing adenovirus after treatment of DMSO (closed triangles) or FTI-277 (open triangles), and of cells infected with lacZ-expressing adenovirus after treatment of DMSO (closed circles) or FTI-277 (open circles), and of cells infected with empty adenovirus after treatment of DMSO (closed squares) or FTI-277 (open squares) were assessed using MTT assay. The effect of FTI-277 on the α-synuclein accumulation in the SH-SY5Y infected with α-synuclein-expressing adenovirus were analyzed by Western blotting (B) and the amount of α-synuclein (C) was quantified using NIH Image program and normalized against the amount of actin.

The commercially-available small molecule farnesyl transferase inhibitor FTI-277, which had previously been shown to reduce the amount of membrane-associated, farnesylated species (*Figure 1**,* panel B, row d), resulted in a significantly decreased loss of cells (compare *Figure 3**,* panel B to panel A). This neuroprotective effect was eliminated by co-adminstration of the small-molecule UCH-L1 inhibitor (not shown), suggesting that the FTI effect was primarily due to its effect on UCH-L1. Treatment with FTI-277 reduced the total amount of UCH-L1 in SH-SY5Y cells and increased its rate of turnover (pulse-chase experiment not shown), in addition to reducing the amount of membrane-associated protein. This treatment also reduced the amount of α-synuclein in these cells (*Figure 4*, panels B and C).

The following publications describe useful farnesyl transferase inhibitor compounds, their structural and functional analogs and compositions and related synthetic methods: US 6,258,824, US 6,388,092, US 6,710,209, US 6,479,513, US 6,740,757, US 6,734,308, US 6,645,982, US 6,579,887, US 6,545,020, US 6,458,800, US 6,451,812, US 6,420,387, US 6,294,552, US 6,187,786, US 6,177,432, US 6,169,096, US 6,150,377, US 6,037,350, US 5,968,952, WO 2002050058, W0 2002085364, W0 2002064142, W0 2002043733, W0 2001064252, US 2002019530, US 2002120145, US 2003212008, W0 2001064246, US 2003022918, W0 2001064226, US 2003027808, US 2003114487, US 2004192727, W0 2001064218, US 2003125326, W0 2001064217, US 2003078281, W0 2001064199, US 2003181473, W0 2001064198, US 2003050323, W0 2001064197, US 2003125268, W0 2001064196, US 2003060480, W0 2001064195, US 2003186925, W0 2001064194, US 2003100553, W0 2001062234, US 2003060450, W0 2001056552, US 2003027839, W0 2000001411, US 6545020, W0 2000001386, US 6451812, W0 9855124, US 6365600, US 2002091138, W0 9721701, US 6169096, US 6420387, W0 2002024687, US 2003199547, W0 2002024686, US 2003207887, W0 2002024683, W0 2002072574, US 6358961, WO 2003080058, WO 2003/021355, WO 2001/53289, WO 2000/47574, and WO 2000/12499; each of which is incorporated herein by reference. The disclosures of these and all patents, published patent applications, and scientific publications are incorporated herein by reference in their entirety.

### Example 5: Treatment with Zarnestra Decreases α-Synuclein Levels in the Brain

Farnesyl transferase inhibitors Zarnestra and OSI-754 were administered to mice of the α-synuclein transgenic line described in Masliah *et al.* (Masliah et al. "Dopaminergic loss and inclusion body formation in alpha-synuclein mice: implications for neurodegenerative disorders" Science 287(5456):1265-69, 2000; incorporated herein by reference). Animals from this line have α-synuclein neuronal inclusions in the cortex, hippocampus, and the olfactory bulb (Masliah et al. "Dopaminergic loss and inclusion body formation in alpha-synuclein mice: implications for neurodegenerative disorders" Science 287(5456):1265-69, 2000; incorporated herein by reference). Transgenic mice were orally administered either FTI in 20% cyclodextrin solution or the same volume of vehicle alone twice a day for 30 or 90 days. In some cases, non-transgenic mice also received vehicle twice a day for 30 to 90 days. At the end of treatment, mice were sacrificed, and the brains removed and hemisected. One hemisphere of each was fixed in 4% paraformaldehyde/PBS (pH 7.4), cryoperserved, then sectioned for histology. The other hemisphere was subdivided into four brain regions, including the cortex and hippocampus, that were homogenized and processed into cytoplasmic and membrane fractions.

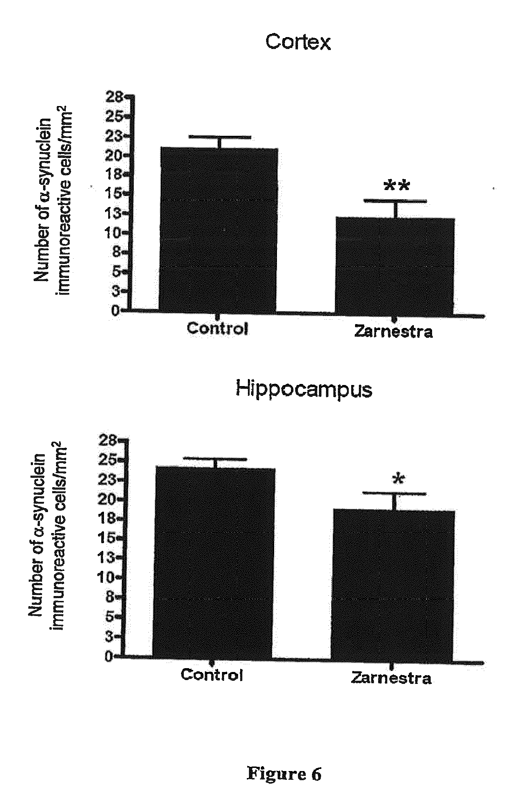

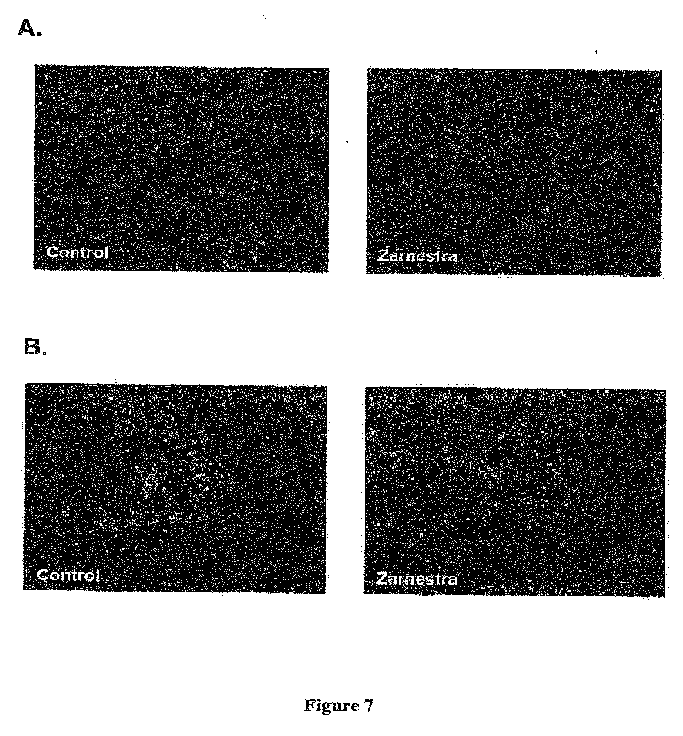

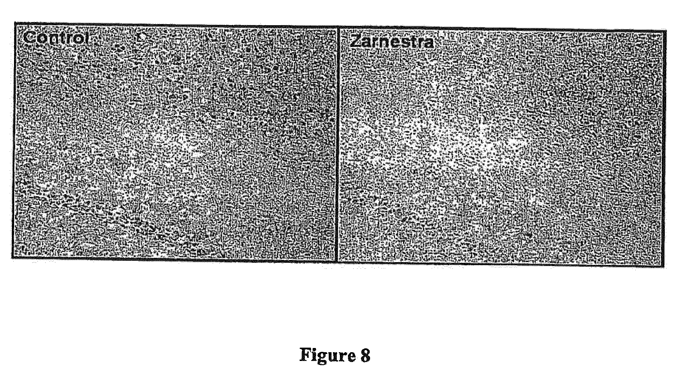

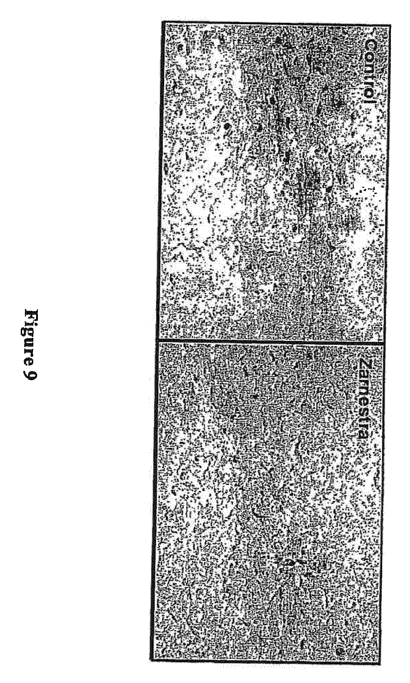

Transgenic animals treated with 35 mg/kg Zarnestra twice a day for 30 days exhibited fewer inclusions than transgenic animals administered vehicle alone. Formation of α -synuclein inclusions in the cortex and hippocampus was probed by immunostaining with an antibody for full-length human α-synuclein. Cells positive for human α-synuclein were quantified. In both regions, transgenic mice that received Zarnestra had significantly fewer α -synuclein-positive cells per mm² than those treated with vehicle (*Figure 6*). Representative images are shown in *Figure 7*. These regions were also analyzed for ubiquitin-immunoreactive inclusions and by the Campbell Switzer method of silver staining. Ubiquitin is known to be a constituent of Lewy Bodies and in the α-synculein inclusions found in the transgenic mouse line used in the study (Masliah et al. "Dopaminergic loss and inclusion body formation in alpha-synuclein mice: implications for neurodegenerative disorders" Science 287(5456):1265-69, 2000; incorporated herein by reference). Transgenic mice that received Zarnestra had fewer ubiquitin-immuoreactive inclusions than those treated with vehicle alone (*Figure 8*). Campbell-Switzer staining is a general marker of Lewy Body type inclusions (Uchihara et al. "Silver stainings distinguish Lewy bodies and glial cytoplasmic inclusions: comparison between Gallyas-Braak and Campbell-Switzer methods" Acta Neuropathol. (Berl.) 110(3):255-60, 2005; incorporated herein by reference). Transgenic mice treated with Zarnestra had fewer inclusions than those that received vehicle alone (*Figure 9*).

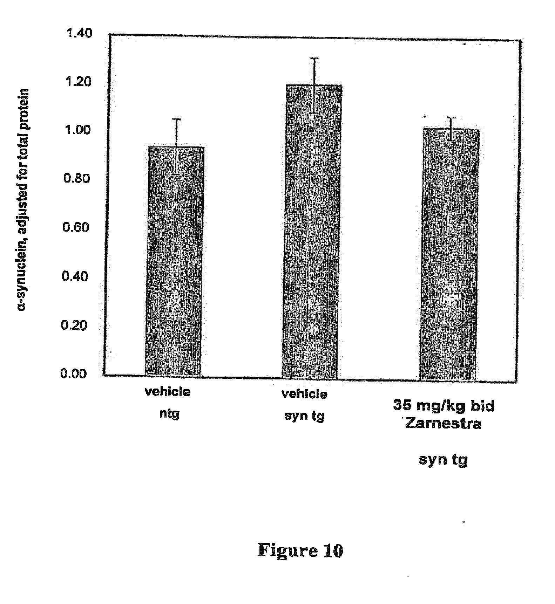

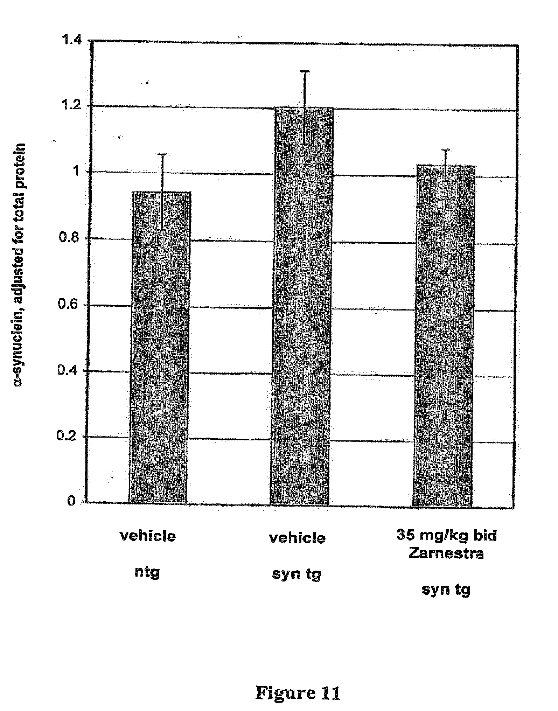

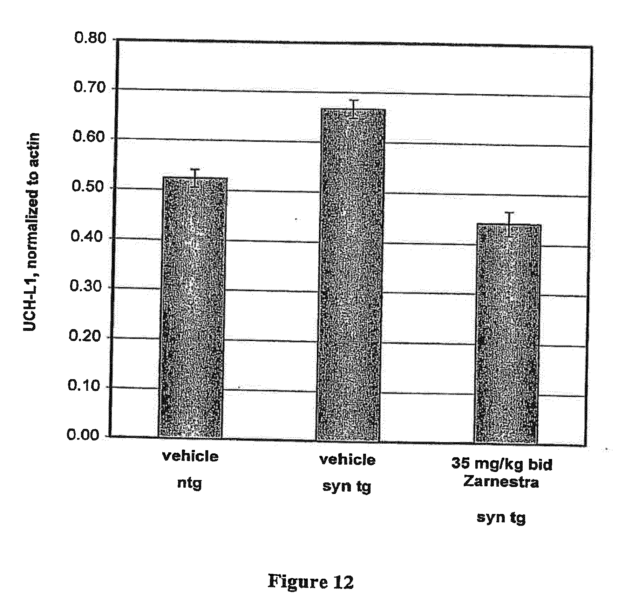

Treatment with 35 mg/kg Zarnestra twice a day for 30 days decreased levels of α-synuclein protein in the cortex and the amount of farnesylated UCH-L1 in the cortex of transgenic mice. Total α -synuclein levels were analyzed by a sandwich ELISA assay similar to one previously described (El-Agnaf et al. "Detection of oligomeric forms of alpha-synuclein protein in human plasma as a potential biomarker for Parkinson's disease" FASEB J. 20(3):419-25, 2006; incorporated herein by reference). In the cortex of vehicle treated animals, α -synuclein protein levels in the α -synuclein transgenic line are greater than in non-transgenic mice in both cytoplasmic (*Figure 10*) and membrane fractions (*Figure 11*). Transgenic mice that received Zarnestra had lower α -synuclein protein levels than vehicle-treated transgenic mice and nearly the same as that detected in the non-transgenic group in both the cytoplasmic (*Figure 10*) and membrane fractions (*Figure 11*), which represent soluble and insoluble α -synuclein, respectively. Farnesylated UCH-L1 in the cortex is contained in the membrane fraction. The amount of UCH-L1 was determined by quantitative Western Blot. Vehicle-treated α-synuclein transgenic mice had significantly more farnesylated UCH-L1 than non-transgenic mice. Treatment with Zarnestra decreased the amount of farnesylated UCH-L1 in transgenic mice to levels similar to non-transgenic mice that received vehicle alone (*Figure 12*).

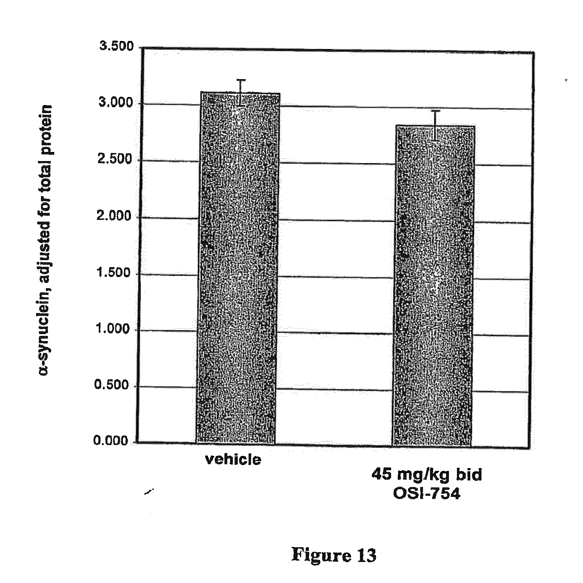

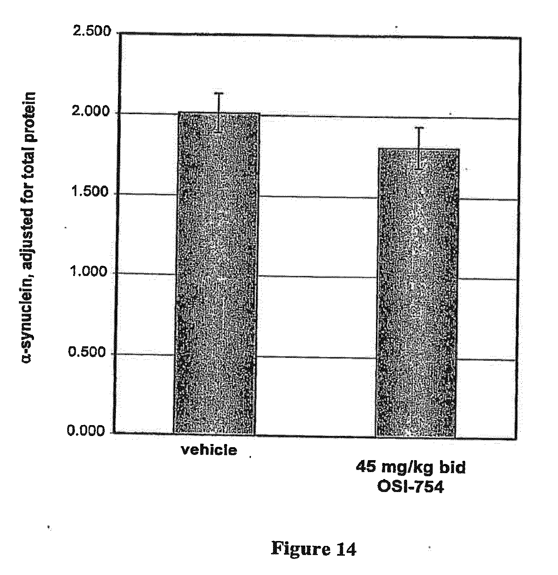

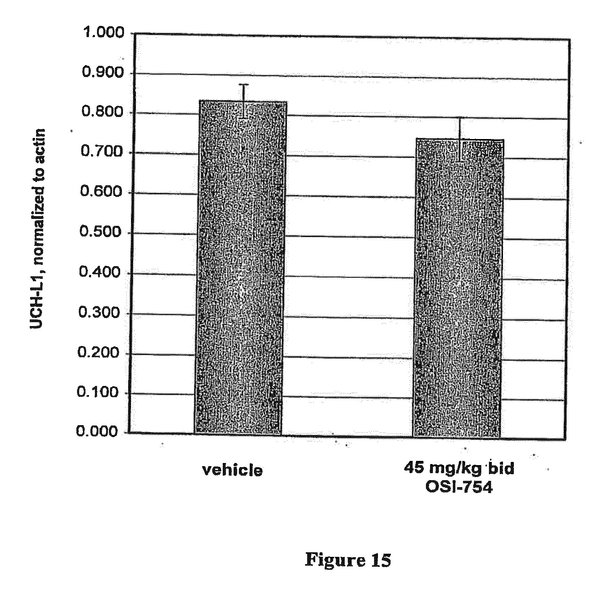

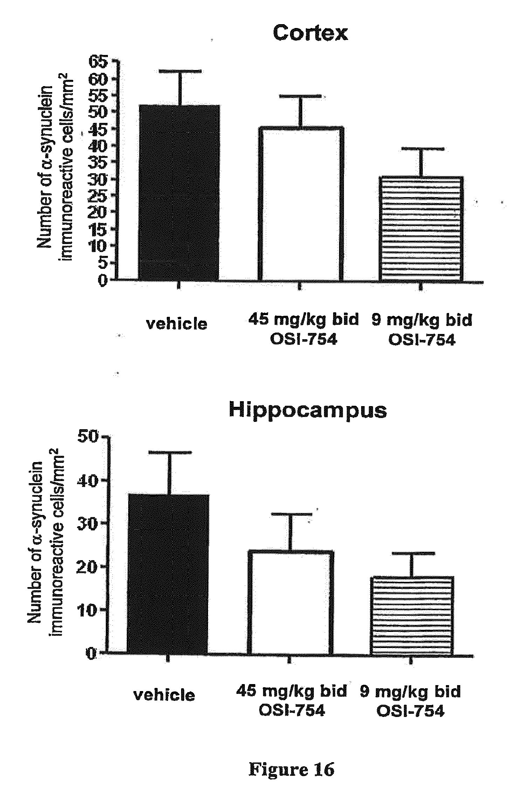

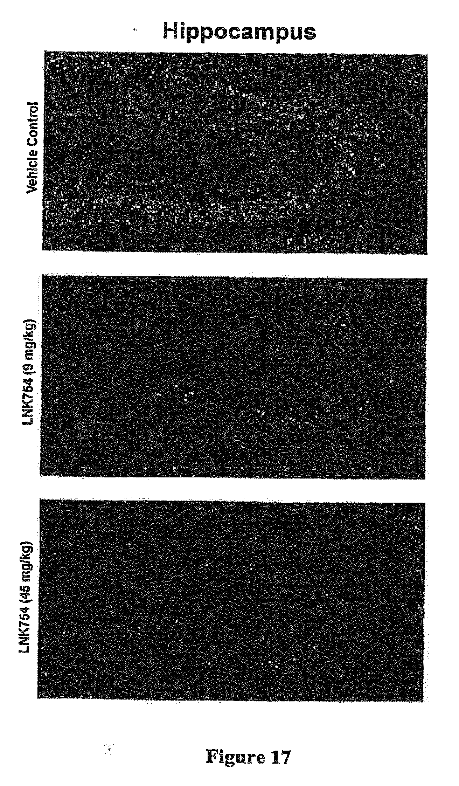

Treatment with 45 mg/kg OSI-754 twice a day for 30 days decreased levels of α-synuclein protein in the cortex and the amount of farnesylated UCH-L1 in the cortex of transgenic mice. Total α-synuclein levels were analyzed by a sandwich ELISA assay. Transgenic mice that received OSI-754 had lower α-synuclein protein levels than vehicle-treated transgenic mice in both cytoplasmic (*Figure 13*) and membrane fractions (*Figure 14*)*.* The amount of farnesylated UCH-L1 was determined by quantitative Western Blot, then normalized to actin. Treatment with OSI-754 decreased the amount of farnesylated UCH-L1 in transgenic mice (*Figure 15*).
Transgenic α-synuclein mice treated with either 45 mg/kg OSI-754 twice a day or with 9 mg/kg OSI-754 twice a day for 90 days exhibited fewer inclusions than transgenic animals administered vehicle alone. Formation of α -synuclein inclusions in the cortex and hippocampus was probed by immunostaining with an antibody for full-length human α - synuclein. Cells positive for human α -synuclein were quantified. In both regions, transgenic mice that received OSI-754 at either dose had fewer α-synuclein-positive cells per mm² than those treated with vehicle (*Figure 16*). Representative images are shown in *Figure 17**.*

Having now described some illustrative embodiments of the invention, it should be apparent to those skilled in the art that the foregoing is merely illustrative and not limiting, having been presented by way of example only. Numerous modifications and other illustrative embodiments are within the scope of one of ordinary skill in the art and are contemplated as falling within the scope of the invention. In particular, although many of the examples presented herein involve specific combinations of method acts or system elements, it should be understood that those acts and those elements may be combined in other ways to accomplish the same objectives. Acts, elements and features discussed only in connection with one embodiment are not intended to be excluded from a similar role in other embodiments. Further, for the one or more means-plus-function limitations recited in the following claims, the means are not intended to be limited to the means disclosed herein for performing the recited function, but are intended to cover in scope any means, known now or later developed, for performing the recited function. Use of ordinal terms such as "first", "second", "third", *etc.,* in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements. Similarly, use of a), b), *etc.,* or i), ii), *etc.* does not by itself connote any priority, precedence, or order of steps in the claims. Similarly, the use of these terms in the specification does not by itself connote any required priority, precedence, or order.

The foregoing written specification is considered to be sufficient to enable one skilled in the art to practice the invention. The present invention is not to be limited in scope by examples provided, since the examples are intended as a single illustration of one aspect of the invention and other functionally equivalent embodiments are within the scope of the invention. Various modifications of the invention in addition to those shown and described herein will become apparent to those skilled in the art from the foregoing description and fall within the scope of the appended claims. The advantages and objects of the invention are not necessarily encompassed by each embodiment of the invention.
1. A method of treating a synucleinopathic subject, the method comprising, administering to a synucleinopathic subject a therapeutically effective amount of a farnesyl transferase inhibitor of formula: 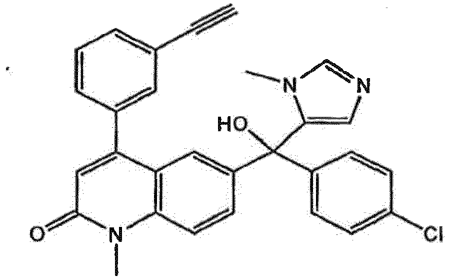 or a pharmaceutically acceptable derviative, analog, stereoisomer, isomer, solvate, or salt thereof.
2. A method of treating a synucleinopathic subject, the method comprising, administering to a synucleinopathic subject a therapeutically effective amount of a farnesyl transferase inhibitor of formula: 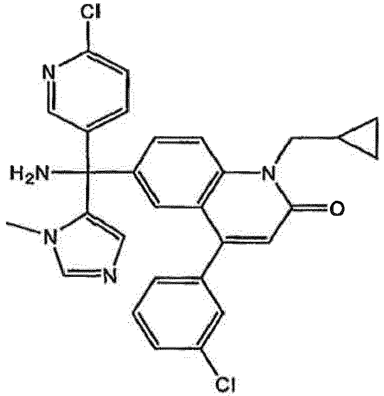 or a pharmaceutically acceptable derviative, analog, stereoisomer, isomer, solvate, or salt thereof.
3. The method of clause 1 or 2, wherein the farnesyl transferase inhibitor is in a tartrate salt form.
4. The method of clause 1 or 2, wherein the synucleinopathic subject has a synucleinopathy selected from the group consisting of: Parkinson's disease, diffuse Lewy body disease, and multiple system atrophy.
5. The method of clause 1 or 2, wherein the subject is a human.
6. The method of clause 1 or 2, wherein the therapeutically effective amount comprises about 10 mg/kg of body weight to about 1000 mg/kg of body weight at a frequency of administration from once a day to once a month.
7. The method of clause 1 or 2, further comprising administering to the subject an amount of one or more non-farnesyl transferase inhibitor compounds effective in treating a neurological disorder.
8. The method of clause 7, wherein each non-farnesyl transferase inhibitor compound is selected from the group consisting of: dopamine agonist, DOPA decarboxylase inhibitor, dopamine precursor, monoamine oxidase blocker, cathechol O-methyl transferase inhibitor, anticholinergic, and NMDA antagonist.
9. The method of clause 7, wherein each non-farnesyl trasferase inhibitor compound is selected from the group consisting of Memantine, Aricept, and other acetylcholinesterase inhibitors.
10. A pharmaceutical composition for treating a synucleinopathic subject comprising a farnesyl transferase inhibitor compound of clause 1 or 2 and a pharmaceutically acceptable excipient.
11. The pharmaceutical composition of clause 10, wherein the synucleinopathy is selected from the group consisting of: Parkinson's disease, diffuse Lewy body disease, and multiple system atrophy.
12. The pharmaceutical composition of clause 10, further comprising one or more non-farnesyl transferase inhibitor compounds effective to treat a neurological disorder.
13. The pharmaceutical composition of clause 12, wherein each non-farnesyl transferase inhibitor compound is selected from the group consisting of: dopamine agonist, DOPA decarboxylase inhibitor, dopamine precursor, monoamine oxidase blocker, cathechol O-methyl transferase inhibitor, anticholinergic, and NMDA antagonist.
14. An article of manufacture comprising packaging material and a farnesyl transferase inhibitor compound of clause 1 or 2, wherein the article of manufacture further comprises a label or package insert indicating that the farnesyl transferase inhibitor compound can be administered to a subject for treating a synucleinopathy.
15. The article of manufacture of clause 14, wherein the synucleinopathy is selected from the group consisting of: Parkinson's disease, diffuse Lewy body disease, and multiple system atrophy.
16. The article of manufacture of clause 14, further comprising one or more non-farnesyl transferase inhibitor compounds effective to treat a neurological disorder.
17. The article of manufacture of clause 14, wherein each non-farnesyl transferase inhibitor compound is selected from the group consisting of: dopamine agonist, DOPA decarboxylase inhibitor, dopamine precursor, monoamine oxidase blocker, cathechol O-methyl transferase inhibitor, anticholinergic, and NMDA antagonist.
18. A method of treating a synucleinopathic subject, the method comprising, administering to a synucleinopathic subject a therapeutically effective amount of a farnesyl transferase inhibitor of formula (I): 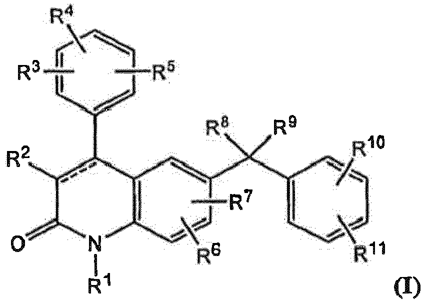 wherein
   the dashed line indicates that the bond between C-3 and C-4 of the quinolin-2-one ring is a single or double bond;
   R¹ is selected from H, C₁-C₁₀ alkyl, -(CR¹³R¹⁴)_{q}C(O)R¹², -(CR¹³R¹⁴)_{q}C(O)OR¹⁵, -(CR¹³R¹⁴)_{q}OR¹², -(CR¹³R¹⁴)_{q}SO₂R¹⁵, -(CR¹³R¹⁴)ₜ(C₃-C₁₀ cycloalkyl), -(CR¹³R¹⁴)ₜ(C₆-C₁₀ aryl), and -(CR¹³R¹⁴)ₜ(4-10 membered heterocyclic), wherein t is an integer from 0 to 5 and q is an integer from 1 to 5, said cycloalkyl, aryl and heterocyclic R¹ groups are optionally fused to a C₆-C₁₀ aryl group, a C₅-C₈ saturated cyclic group, or a 4-10 membered heterocyclic group; and the foregoing R¹ groups, except H but including any optional fused rings referred to above, are optionally substituted by 1 to 4 R⁶ groups;
   R² is halo, cyano, -C(O)OR¹⁵, or a group selected from the substituents provided in the definition of R¹²;
   each R³, R⁴, R⁵, R⁶, and R⁷ is independently selected from H, C₁-C₁₀ alkyl, C₂-C₁₀ alkenyl, halo, cyano, nitro, mercapto, trifluoromethyl, trifluoromethoxy, azido, -OR¹², - C(O)R¹², -C(O)OR¹², -NR¹³C(O)OR¹⁵, -OC(O)R¹², -NR¹³SO₂R¹⁵, -SO₂NR¹²R¹³, - NR¹³C(O)R¹², -C(O)NR¹²R¹³, -NR¹²R¹³, -CH=NOR¹², -S(O)ⱼR¹² wherein j is an integer from 0 to 2, -(CR¹³R¹⁴)ₜ(C₆-C₁₀ aryl), -(CR¹³R¹⁴)ₜ(4-10 membered heterocyclic), -(CR¹³R¹⁴)ₜ(C₃-C₁₀ cycloalkyl), and -(CR¹³R¹⁴)ₜC≡CR¹⁶, and wherein in the foregoing R³, R⁴, R⁵, R⁶, and R⁷ groups t is an integer from 0 to 5; the cycloalkyl, aryl and heterocyclic moieties of the foregoing groups are optionally fused to a C₆-C₁₀ aryl group, a C₅-C₈ saturated cyclic group, or a 4-10 membered heterocyclic group; and said alkyl, alkenyl, cycloalkyl, aryl and heterocyclic groups are optionally substituted by 1 to 3 substituents independently selected from halo, cyano, nitro, trifluoromethyl, trifluoromethoxy, azido, -NR¹³SO₂R¹⁵, - SO₂NR¹²R¹³, -C(O)R¹², -C(O)OR¹², -OC(O)R¹², -NR¹³C(O)OR¹⁵, -NR¹³C(O)R¹², - C(O)NR¹²R¹³, -NR¹²R¹³, -OR¹², C₁-C₁₀ alkyl, C₂-C₁₀ alkenyl, C₂-C₁₀ alkynyl, -(CR¹³R¹⁴)ₜ(C₆-C₁₀ aryl), and -(CR¹³R¹⁴)ₜ(4-10 membered heterocyclic), wherein t is an integer from 0 to 5;
   R⁸ is H, -OR¹², -NR¹²R¹³, -NR¹²C(O)R¹³, cyano, -C(O)OR¹³, -SR¹², -(CR¹³R¹⁴)ₜ(4-10 membered heterocyclic), wherein t is an integer from 0 to 5, or C₁-C₆ alkyl, wherein said heterocyclic and alkyl moieties are optionally substituted by 1 to 3 R⁶ substituents;
   R⁹ is -(CR¹³R¹⁴)ₜ(imidazolyl) wherein t is an integer from 0 to 5 and said imidazolyl moiety is optionally substituted by 1 or 2 R⁶ substituents;
   each R¹⁰ and R¹¹ is independently selected from the substituents provided in the definition of R6;
   each R¹² is independently selected from H₁ C₁-C₁₀ alkyl, -(CR¹³R¹⁴)ₜ(C₃-C₁₀ cycloalkyl), -(CR¹³R¹⁴)ₜ(C₆-C₁₀ aryl), and -(CR¹³R¹⁴)ₜ(4-10 membered heterocyclic), wherein t is an integer from 0 to 5; said cycloalkyl, aryl and heterocyclic R¹² groups are optionally fused to a C₆-C₁₀ aryl group, a C₅-C₈ saturated cyclic group, or a 4-10 membered heterocyclic group; and the foregoing R¹² substituents, except H, are optionally substituted by 1 to 3 substituents independently selected from halo, cyano, nitro, trifluoromethyl, trifluoromethoxy, azido, -C(O)R¹³, -C(O)OR¹³, -OC(O)R¹³, -NR¹³C(O)R¹⁴, -C(O)NR¹³R¹⁴, - NR¹³R¹⁴, hydroxy, C₁-C₆ alkyl, and C₁-C₆ alkoxy;
   each R¹³ and R¹⁴ is independently H or C₁-C₆ alkyl, and where R¹³ and R¹⁴ are as -(CR¹³R¹⁴)_{q} or (CR¹³R¹⁴)ₜ each is independently defined for each iteration of q or t in excess of 1;
   R¹⁵ is selected from the substituents provided in the definition of R¹² except R¹⁵ is not H;
   R¹⁶ is selected from the list of substituents provided in the definition of R¹² and -SiR¹⁷R¹⁸R¹⁹;
   R¹⁷, R¹⁸ and R¹⁹ are each independently selected from the substituents provided in the definition of R¹² except R¹⁷, R¹⁸ and R¹⁹ are not H; and
   provided that at least one of R³, R⁴ and R⁵ is -(CR¹³R¹⁴)ₜC≡CR¹⁶ wherein t is an integer from 0 to 5 and R¹³, R¹⁴, and R¹⁶ are as defined above;
   or a derviative, analog, stereoisomer, isomer, solvate, or salt thereof.
19. The method of clause, 8, wherein R¹ is methyl.
20. The method of clause 8, wherein the dotted line represents a bond.
21. The method of clause 8, wherein R¹ is hydrogen or C₁₋₆ alkyl.
22. The method clause 18, wherein R² is hydrogen.
23. The method of clause 18, wherein R² is hydrogen, a halogen, or C₁-C₆ alkyl.
24. The method of clause 18, wherein R³ is hydrogen; R⁴ ishydrogen; and R⁵ is ethynyl.
25. The method of clause 18, wherein at least one of R³, R⁴, and R⁵ is ethynyl.
26. The method of clause 18, wherein at least one of R³, R⁴, and R⁵ is -(CR¹³R¹⁴)ₜ≡CR¹⁶.
27. The method of clause 18, wherein R⁶ is hydrogen.
28. The method of clause 18, wherein R⁷ is hydrogen.
29. The method of clause 18, wherein R⁸ is hydrogen, -OR¹², or -NR¹²R¹³.
30. The method of clause 18, wherein R⁸ is -OH.
31. The method of clause 18, wherein R⁸ is -NH₂
32. The method of clause 18, wherein R⁹ is
33. The method of clause 18, wherein R⁹ is 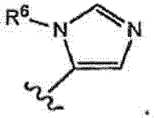 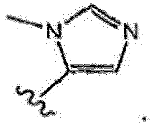
34. The method of clause 18, wherein R¹⁰ is hydrogen.
35. The method of clause 18, wherein R¹⁰ is halogen.
36. The method of clause 18, wherein R¹⁰ is chlorine
37. The method of clause 18, wherein at least one of R¹⁰ and R¹¹ is hydrogen.
38. The method of clause 18, wherein the compound as the stereochemistry as shown in formula: 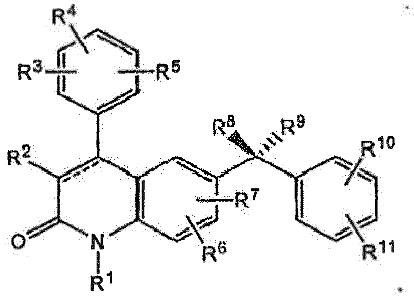
39. The method of clause 18, wherein the compound as the stereochemistry as shown in formula: 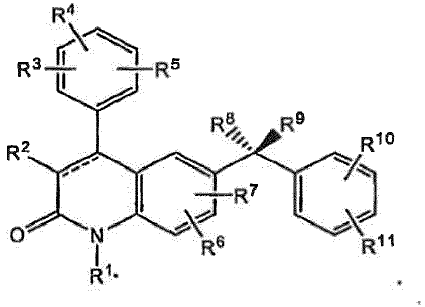
40. The method of clause 18, wherein the compound is of the formula: 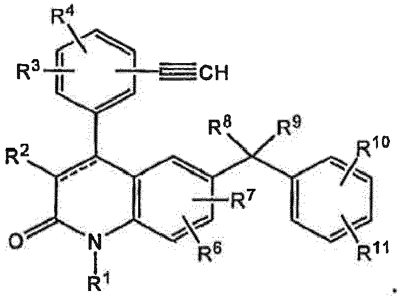
41. The method of clause 18, wherein the compound is of the formula: 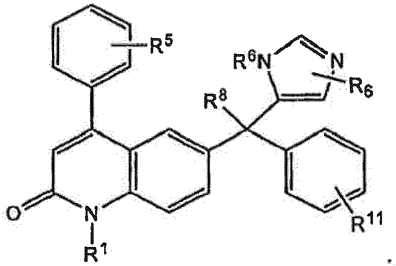
42. The method of clause 18, wherein the compound is of one of the formulae: 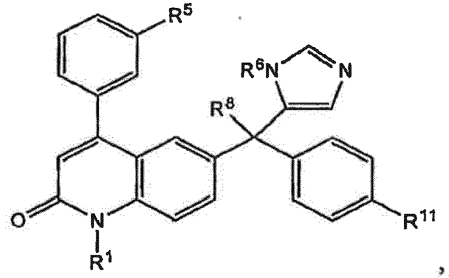 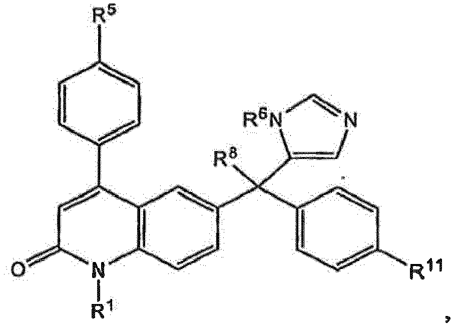 or 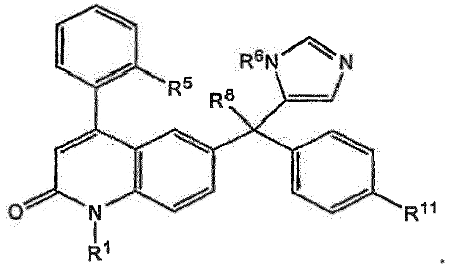
43. The method of clause 18, wherein the compound is of the formula: 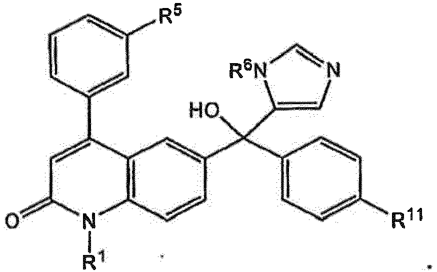
44. The method of clause 18, wherein the synucleinopathic subject has a synucleinopathy selected from the group consisting of: Parkinson's disease, diffuse Lewy body disease, and multiple system atrophy.
45. The method of clause 18, wherein the subject is a human.
46. The method of clause 18, wherein the effective amount comprises about 10 ng/kg of body weight to about 1000 mg/kg of body weight at a frequency of administration from once a day to once a month.
47. The method of clause 18, further comprising administering to the subject an amount of one or more non-farnesyl transferase inhibitor compounds effective in treating a neurological disorder.
48. The method of clause 47, wherein each non-farnesyl transferase inhibitor compound is selected from the group consisting of: dopamine agonist, DOPA decarboxylase inhibitor, dopamine precursor, monoamine oxidase blocker, cathechol O-methyl transferase inhibitor, anticholinergic, and NMDA antagonist.
49. The method of clause 47, wherein each non-farnesyl trasferase inhibitor compound is selected from the group consisting of Memantine, Aricept, and other acetylcholinesterase inhibitors.
50. A pharmaceutical composition for treating a synucleinopathy comprising a farnesyl transferase inhibitor compound of clause 18 and a pharmaceutically acceptable excipient.
51. The pharmaceutical composition of clause 50, wherein the synucleinopathy is selected from the group consisting of: Parkinson's disease, diffuse Lewy body disease, and multiple system atrophy.
52. The pharmaceutical composition of clause 50, further comprising one or more non-farnesyl transferase inhibitor compounds effective to treat a neurological disorder.
53. An article of manufacture comprising packaging material and a farnesyl transferase inhibitor compound of clause 18 wherein the article of manufacture further comprises a label or package insert indicating that the farnesyl transferase inhibitor compound can be administered to a subject for treating a synucleinopathy.
54. The article of manufacture of clause 53, wherein the synucleinopathy is selected from the group consisting of: Parkinson's disease, diffuse Lewy body disease, and multiple system atrophy.
55. The article of manufacture of clause 53, further comprising one or more non-farnesyl transferase inhibitor compounds effective to treat a neurological disorder.
56. The article of manufacture of clause 55, wherein each non-farnesyl transferase inhibitor compound is selected from the group consisting of: dopamine agonist, DOPA decarboxylase inhibitor, dopamine precursor, monoamine oxidase blocker, cathechol O-methyl transferase inhibitor, anticholinergic, and NMDA antagonist.
57. A method of treating a synucleinopathic subject, the method comprising, administering to a synucleinopathic subject a therapeutically effective amount of 6-[(4-chloro-phenyl)-hydroxy(3-methyl-3H-imidazol-4-yl)-methyl]-4-(3-ethynyl-phenyl)-1-methyl-1H-quinolin-2-one, 2,3-dihydroxy butanedioate.
58. A method of treating a synucleinopathic subject, the method comprising, administering to a synucleinopathic subject a therapeutically effective amount of 6-[amino-(6-chloro-pyridin-3-yl)-(3-methyl-3H-imidazol-4-yl)-methyl]-4-(3-chloro-phenyl)-1- cyclopropylmethyl-1H-quinolin-2-one.
59. The method of clause 57 or 58, wherein the synucleinopathic subject has a synucleinopathy selected from the group consisting of Parkinson's disease, diffuse Lewy body disease, and multiple system atrophy.
60. The method of clause 57 or 58, wherein the subject is a human.
61. The method of clause 57 or 58, wherein the effective amount comprises about 10 mg/kg of body weight to about 1000 mg/kg of body weight at a frequency of administration from once a day to once a month.
62. The method of clause 57 or 58, further comprising administering to the subject an amount of one or more non-farnesyl transferase inhibitor compounds effective in treating a neurological disorder.
63. The method of clause 62, wherein each non-famesyl transferase inhibitor compound is selected from the group consisting of: dopamine agonist, DOPA decarboxylase inhibitor, dopamine precursor, monoamine oxidase blocker, cathechol 0-methyl transferase inhibitor, anticholinergic, and NMDA antagonist.
64. The method of clause 62, wherein each non-famesyl trasferase inhibitor compound is selected from the group consisting of Memantine, Aricept, and other acetylcholinesterase inhibitors.
65. A pharmaceutical composition for treating a synucleinopathy comprising 64[(4-chloro-phenyl)-hydroxy(3-methyl-3H-imidazol-4-yl)-methyl]-4-(3-ethynyl-phenyl)-1-methyl-1H-quinolin-2-one, 2,3-dihydroxy butanedioate and a pharmaceutically acceptable excipient.
66. A pharmaceutical composition for treating a synucleinopathy comprising 6-[amino-(6-chloro-pyridin-3-yl)-(3-methyl-3H-imidazol-4-yl)-methyl]-4-(3-chloro-phenyl)-1-cyclopropylmethyl-1H-quinolin-2-one and a pharmaceutically acceptable excipient.
67. The pharmaceutical composition of clause 65 or 66, wherein the synucleinopathy is selected from the group consisting of: Parkinson's disease, diffuse Lewy body disease, and multiple system atrophy.
68. The pharmaceutical composition of clause 65 or 66, further comprising one or more non-farnesyl transferase inhibitor compounds effective to treat a neurological disorder.
69. An article of manufacture comprising packaging material and 6-[(4-chloro-phenyl)-hydroxy(3-methyl-3H-imidazol-4-yl)-methyl] -4-(3-ethynyl-phenyl)-1-methyl-1H-quinolin-2-one, 2,3-dihydroxy butanedioate wherein the article of manufacture further comprises a label or package insert indicating that the farnesyl transferase inhibitor compound can be administered to a subject for treating a synucleinopathy.
70. An article of manufacture comprising packaging material and 6-[amino-(6-chloro-pyridin-3-yl)-(3-methyl-3H-imidazol-4-yl)-methyll-4-(3-chloro-phenyl)-1- cyclopropylmethyl-1H-quinolin-2-one wherein the article of manufacture further comprises a label or package insert indicating that the farnesyl transferase inhibitor compound can be administered to a subject for treating a synucleinopathy.
71. The article of manufacture of clause 69 or 70, wherein the synucleinopathy is selected from the group consisting of: Parkinson's disease, diffuse Lewy body disease, and multiple system atrophy.
72. The article of manufacture of clause 69 or 70, further comprising one or more non-farnesyl transferase inhibitor compounds effective to treat a neurological disorder.
73. The article of manufacture of clause 72, wherein each non-farnesyl transferase inhibitor compound is selected from the group consisting of: dopamine agonist, DOPA decarboxylase inhibitor, dopamine precursor, monoamine oxidase blocker, cathechol O-methyl transferase inhibitor, anticholinergic, and NMDA antagonist.
74. A method of reducing a-synuclein toxicity in a cell, the method comprising, administering to a cell a therapeutically effective amount of a farnesyl transferase inhibitor of formula: 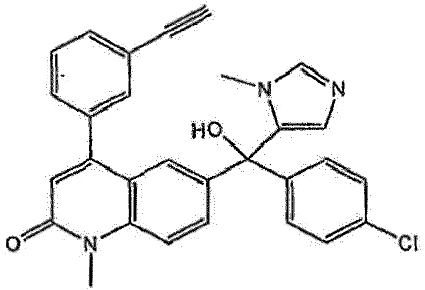 or a pharmaceutically acceptable derviative, analog, stereoisomer, isomer, solvate, or salt thereof.
75. A method of reducing α-synuclein toxicity in a cell, the method comprising, administering to a cell a therapeutically effective amount of a farnesyl transferase inhibitor of formula: 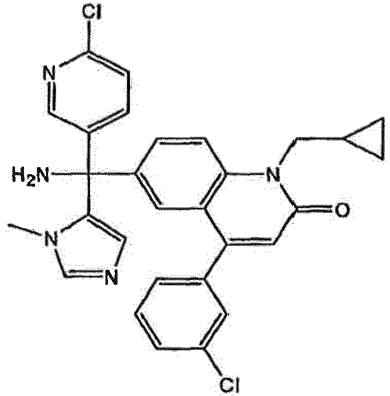 or a pharmaceutically acceptable derviative, analog, stereoisomer, isomer, solvate, or salt thereof.
76. The method of clause 74 or 75, wherein the cell is a neuronal cell.
77. The method of clause 74 or 75, wherein the cell expresses α-synuclein.

## Claims

1. A farnesyl transferase inhibitor of formula: 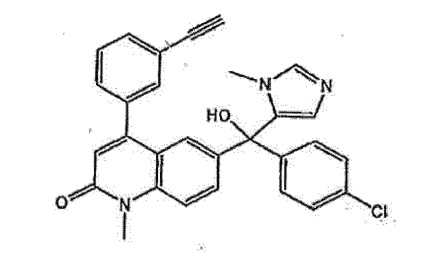 or a farnesyl transferase inhibitor of formula: 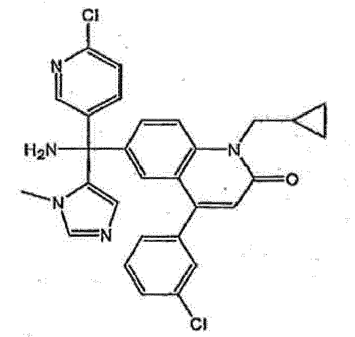 or a pharmaceutically acceptable derivative, analog, stereoisomer, isomer, solvate, or salt thereof for use in treating a synucleinopathic subject, wherein said inhibitor is administered in a therapeutically effective amount.

2. The farnesyl transferase inhibitor of claim 1, wherein the farnesyl transferase inhibitor is in a tartrate salt form.

3. A farnesyl transferase inhibitor of formula (**I**): 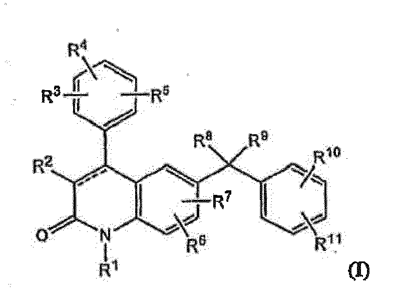 wherein
the dashed line indicates that the bond between C-3 and C-4 of the quinolin-2-one ring is a single or double bond;
R¹ is selected from H, C₁-C₁₀ alkyl, -(CR¹³R¹⁴)_{q}C(O)R¹², -(CR¹³R¹⁴)_{q}C(O)OR¹⁵, -(CR¹³R¹⁴)_{q}OR¹⁵, -(CR¹³R¹⁴)_{q}SO₂R¹⁵, -(CR¹³R¹⁴)ₜ(C₃-C₁₀ cycloalkyl), -(CR¹³R¹⁴)ₜ(C₆-C₁₀ aryl), and -(CR¹³R¹⁴)ₜ(4-10 membered heterocyclic), wherein t is an integer from 0 to 5 and q is an integer from 1 to 5, said eycloalkyl, aryl and heterocyclic R¹ groups are optionally fused to a C₆-C₁₀ aryl group, a C₅-C₈ saturated cyclic group, or a 4-10 membered heterocyclic group; and the foregoing R¹ groups, except H but including any optional fused rings referred to above, are optionally substituted by 1 to 4 R⁶ groups;
R² is halo, cyano, -C(O)OR¹⁵, or a group selected from the substituents provided in the definition of R¹²;
each R³, R⁴, R⁵, R⁶, and R⁷ is independently selected from H, C₁-C₁₀ alkyl, C₂-C₁₀ alkenyl, halo, cyano, nitro, mercapto, trifluoromethyl, trifluoromethoxy, azido, -OR¹²,-C(O)R¹², -C(O)OR¹², -NR¹³C(O)OR¹⁵, -OC(O)R¹², -NR¹³SO₂R¹⁵, -SO₂NR¹²R¹³,-MR¹³C(O)R¹², -C(O)NR¹²R¹³, -NR¹²R¹³, -CH=NOR¹², -S(O)ⱼR¹² wherein j is an integer from 0 to 2, **-**(CR¹³R¹⁴)ₜ(C₆-C₁₀ aryl), -(CR¹³R¹⁴)ₜ(4-10 membered heterocyclic), -(CR¹³R¹⁴)ₜ(C₃-C₁₀ cycloalkyl), and -(CR¹³R¹⁴)ₜC≡CR¹⁶, and wherein in the foregoing R³, R⁴, R⁵, R⁶, and R⁷ groups t is an integer from 0 to 5; the cycloalkyl, aryl and heterocyclic moieties of the foregoing groups are optionally fused to a C₆-C₁₀ aryl group, a C₅-C₈ saturated cyclic group, or a 4-10 membered heterocyclic group; and said alkyl, akenyl, cycloalkyl, aryl and heterocyclic groups are optionally substituted by 1 to 3 substituents independently selected from halo, cyano, nitro, trifluoromethyl, trifluoromethoxy, azido, -NR¹³SO₂R¹⁵, - SO₂NR¹²R¹³, -C(O)R¹², -C(O)OR¹², -OC(O)R¹², -NR¹³C(O)OR¹⁵, -NR¹³C(O)R¹², - C(O)NR¹²R¹³, -NR¹²R¹³, -OR¹², C₁-C₁₀ alkyl, C₂-C₁₀ alkenyl, C₂-C₁₀ alkynyl, -(CR¹³R¹⁴)ₜ(C₆-C₁₀ aryl), and -(CR¹³R¹⁴)ₜ(4-10 membered heterocyclic), wherein t is an integer from 0 to 5;
R⁸ is H, -OR¹², -NR¹²R¹³, -NR¹²C(O)R¹³, cyano, -C(O)OR¹³, -SR¹², -(CR¹³R¹⁴)ₜ(4-10 membered heterocyclic), wherein t is an integer from 0 to 5, or C₁-C₆ alkyl, wherein said heterocyclic and alkyl moieties are optionally substituted by 1 to 3 R⁶ substituents;
R⁹ is -(CR¹³R¹⁴)ₜ(imidazolyl) wherein t is an integer from 0 to 5 and said imidazolyl moiety is optionally substituted by 1 or 2 R⁶ substituents;
each R¹⁰ and R¹¹ is independently selected from the substituents provided in the definition of R6;
each R¹² is independently selected from H₁ C₁-C₁₀ alkyl, -(CR¹³R¹⁴)ₜ(C₃-C₁₀ cycloalkyl), -(CR¹³R¹⁴)ₜ(C₆-C₁₀ aryl), and -(CR¹³R¹⁴)ₜ(4-10 membered heterocyclic), wherein t is an integer from 0 to 5; said cycloalkyl, aryl and heterocyclic R¹² groups are optionally, fused to a C₆-C₁₀ aryl group, a C₅-C₈ saturated cyclic group, or a 4-10 membered heterocyclic group; and the foregoing R¹² substituents, except H, are optionally substituted by 1 to 3 substituents independently selected from halo, cyano, nitro, trifluoromethyl, trifluoromethoxy, azido, -C(O)R¹³,-C(O)OR¹³, -OC(O)R¹³, -NR¹³C(O)R¹⁴, -C(O)NR¹³R¹⁴, - NR¹³R¹⁴, hydroxy, C₁-C₆ alkyl, and C₁-C₆ alkoxy;
each R¹³ and R¹⁴ is independently H or C₁-C₆ alkyl, and where R¹³ and R¹⁴ are as -(CR¹³R¹⁴)_{q} or (CR¹³R¹⁴)ₜ each is dependently defined for each iteration of q or t in excess of 1;
R¹³ is selected from the substituents provided in the definition of R¹² except R¹⁵ is not H;
R¹⁶ is selected from the list of substituents provided in the definition of R¹² and -SiR¹⁷R¹⁸R¹⁹;
R¹⁷, R¹⁸ and R¹⁹ are each independently selected from the substituents provided in the definition of R¹² except R¹⁷, R¹⁸ and R¹⁹ are not H; and
provided that at least one of R³, R⁴ and R⁵ is -(CR¹³R¹⁴)ₜC≡CR¹⁶ wherein t is an integer from 0 to 5 and R¹³, R¹⁴, and R¹⁶ are as defined above;
or a derivative, analog, stereoisomer, isomer, solvate, or salt thereof for use in treating a synucleinopathic subject, wherein said inhibitor is administered in a therapeutically effective amount.

4. The fanesyl transferase inhibitor of claim 3 wherein R³ is hydrogen; R⁴ is hydrogen; and R⁵ is ethynyl or wherein at least one of R³, R⁴ and R⁵ is ₋(CR¹³R¹⁴)ₜC≡CR¹⁶_{.}

5. The farnesyl transferase inhibitor of claim 3 wherein the compound is of the formula: 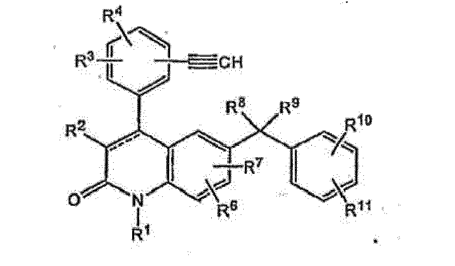 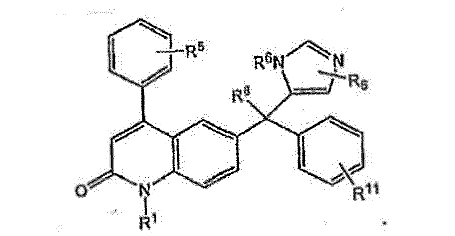 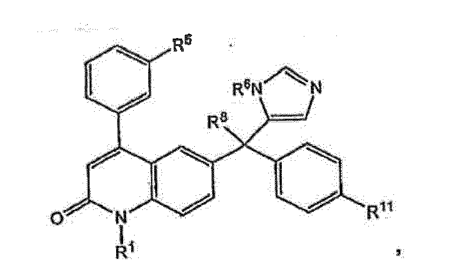 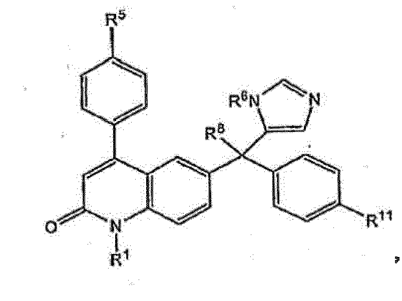 or 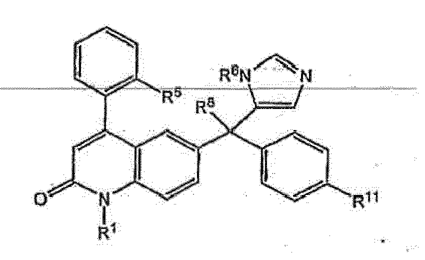 or

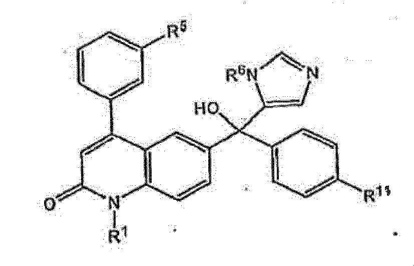

6. 6-[(4-Chloro-phenyl)-hydroxy(3-methyl-3H-imidazol-4-yl)-methyl]-4(3-ethynyl-phenyl)-1-methyl-1H-quinolin-2-one,2,3-dihydroxy butanedioate or 6-[amino-(6-chloro-pyridin-3-yl)-(3-methyl-3H-imidazol-4-yl)-methyl]-(3-chlorophenyl)-1-cyclopropylmethyl-1H-quinolin-2-one for use in treating a synucleinopathic subject, wherein said inhibitor is administered in a therapentically effective amount.

7. The inhibitor of claim 1, 2, 3 or 6, wherein the synucleinopathic subject has a synucleinopathy selected from the group consisting of: Parkinson's disease, diffuse Lewy body disease, and multiple system atrophy.

8. The inhibitor of claim 1, 2, 3 or 6, wherein the subject is a human.

9. The inhibitor of claim 1, 2, 3 or 6, wherein the effective amount comprises about 10 ng/kg of body weight to about 1000 mg/kg of body weight at a frequency of administration from once a day to once a month.

10. The inhibitor of claim 1, 2, 3 or 6, further comprising administering to the subject an amount of one or more non-farnesyl transferase inhibitor compounds effective in treating a neurological disorder preferably wherein each non-farnesyl transferase inhibitor compound is selected from the group consisting of; dopamine agonist, DOPA decarboxylase inhibitor, dopamine precursor, monoamine oxidase blocker, cathechol 0-methyl transferase inhibitor, anticholinergic, and NMDA antagonist or wherein each non-farnesyl transferase inhibitor compound is selected from the group consisting of Memantine, Aricept, and other acetylcholinesterase inhibitors.

11. A pharmaceutical composition for treating a synucleinopathy comprising a farnesyl transferase inhibitor of claim 1, 2 or 3 or 6-[(4-chloro-phenyl)-hydroxy(3-methyl-3H-imidazol-4-yl)-methyl]-4-(3-ethynyl-phenyl)-1-methyl-1H-quinolin-2-one,2,3-dihydroxy butanedioate or 6-[amino-(6-chloro-pyridin-3-yl)-(3-methyl-3H-imidazol-4-yl)-methyl]-4-(3-chloro-phenyl)-1-cyclopropylmethyl-1H-quinolin-2-one.

12. An article of manufacture comprising packaging material a farnesyl transferase inhibitor of claim 1 or 2, or 3 or 6-[(4-chloro-phenyl)-hydroxy(3-methyl-3H-imidazol-4-yl)-methyl]-4-(3-ethynyl-phenyl)-1-methyl-1H-quinolin-2-one,2,3-dihydroxy butanedioate or 6-[amino-(6-chloro-pyridin-3-yl)-(3-methyl-3H-imidazol-4-yl)-methyl]4-(3-chloro-phenyl)-1-cyclopropylmethyl-1H-quinolin-2-one wherein the article of manufacture further comprises a label or package insert indicating that the farnesyl transferase inhibitor compound can be administered to a subject for treating a synucleinopathy.

13. The pharmaceutical composition of claim 11 or the article of manufacture of claim 12, wherein the synucleinopathy is selected from the group consisting of: Parkinson's disease, diffuse Lewy body disease, and multiple system atrophy.

14. The pharmaceutical composition of claim 11 or the article of manufacture of claim 12, further comprising one or more non-farnesyl transferase inhibitor compounds effective to treat a neurological disorder preferably wherein each non-farnesyl transferase inhibitor compound is selected from the group consisting of: dopamine agonist, DOPA decarboxylase inhibitor, dopamine precursor, monoamine oxidase blocker, cathechol O-methyl transferase inhibitor, anticholinergic, and NMDA antagonist.

15. A method of reducing α-synuclein toxicity in a cell, the method comprising, administrating to a cell a therapeutically effective amount of a farnesyl transferase inhibitor of formula: 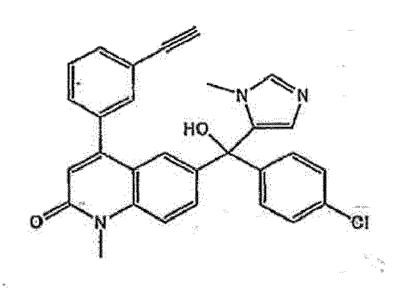 or a therapeutically effective amount of a farnesyl transferase inhibitor of formula: 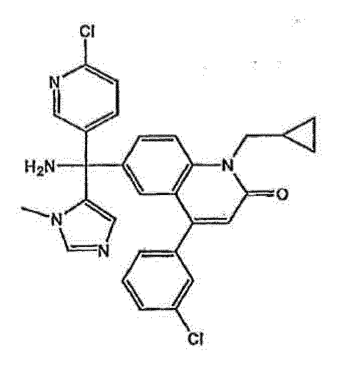 or a pharmaceutically acceptable derivative, analog, stereoisomer, isomer, solvate, or salt thereof, preferably wherein the cell is a neuronal cell or wherein the cell expresses α-synuclein.
